(19) Europäisches Patentamt
European Patent Office
Office européen des brevets

(11) EP 3 597 637 A1

(12) EUROPEAN PATENT APPLICATION

(43) Date of publication:
**22.01.2020 Bulletin 2020/04**

(21) Application number: **18184456.4**

(22) Date of filing: **19.07.2018**

(51) Int Cl.:
*C07D 213/74* (2006.01) *A61K 31/337* (2006.01)
*A61K 31/4025* (2006.01) *A61K 31/44* (2006.01)
*C07D 213/76* (2006.01) *C07C 311/44* (2006.01)
*C07D 207/14* (2006.01) *C07D 211/58* (2006.01)
*C07D 211/96* (2006.01) *C07D 305/08* (2006.01)
*A61P 31/20* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **IRBM S.P.A.**
  **00071 Pomezia (RM) (IT)**
- **Promidis S.r.l.**
  **20132 Milano MI (IT)**
- **Ospedale San Raffaele S.r.l.**
  **20132 Milano (IT)**
- **Istituto Nazionale Di Genetica Molecolare-INGM**
  **20122 Milano (IT)**

(72) Inventors:
- **De Francesco, Raffaele**
  **20146 Milano (IT)**
- **Prandi, Adolfo**
  **20132 Milano (IT)**
- **Randazzo, Pietro**
  **20132 Milano (IT)**
- **Donnici, Lorena**
  **20122 Milano (IT)**
- **Guidotti, Luca**
  **20132 Milano (IT)**
- **Iannacone, Matteo**
  **20132 Milano (IT)**
- **Di Fabio, Romano**
  **00071 Pomezia (IT)**
- **Summa, Vincenzo**
  **00071 Pomezia (IT)**
- **Bencheva, Leda Ivanova**
  **20132 Milano (IT)**
- **De Matteo, Marilenia**
  **20132 Milano (IT)**
- **Ferrante, Luca**
  **20132 Milano (IT)**

(74) Representative: **Capasso, Olga et al**
**De Simone & Partners SpA**
**Via Vincenzo Bellini, 20**
**00198 Roma (IT)**

# (54) INHIBITORS OF HEPATITIS B VIRUS

(57) FIELD OF THE INVENTION

The present invention relates to compounds that are inhibitors of hepatitis B virus (HBV). Compounds of this invention are useful alone or in combination with other agents for treating, ameliorating, preventing or curing HBV infection and related conditions. The present invention also relates to pharmaceutical compositions containing said compounds.

EP 3 597 637 A1

SEQUENCE LISTING

<110> PROMIDIS S.R.L.
IRBM SCIENCE PARK S.P.A.
OSPEDALE SAN RAFFAELE S.R.L.
ISTITUTO NAZIONALE DI GENETICA MOLECOLARE - INGM

<120> INHIBITORS OF HEPATITIS B VIRUS

<130> BE138506

<160> 2

<170> PatentIn version 3.5

<210> 1
<211> 22
<212> DNA
<213> Artificial Sequence

<220>
<223> synthetic primer

<400> 1
attttgttcag tggttcgtag gg 22

<210> 2
<211> 22
<212> DNA
<213> Artificial Sequence

<220>
<223> synthetic primer

<400> 2
cggtaaaaag ggactcaaga tg 22

## Description

FIELD OF THE INVENTION

The present invention relates to compounds that are inhibitors of hepatitis B virus (HBV). Compounds of this invention are useful alone or in combination with other agents for treating, ameliorating, preventing or curing HBV infection and related conditions. The present invention also relates to pharmaceutical compositions containing said compounds.

BACKGROUND OF THE INVENTION

The Hepatitis B virus (HBV) is an enveloped, partially double-stranded DNA (dsDNA) virus of the hepadnaviridae family that is spread by contact with infected blood and body fluids and causes acute and chronic necroinflammatory liver diseases of varying severity (Guidotti LG, Chisari FV. Annu Rev Pathol. 2006; 1:23-61.). The HBV lipid envelope contains 3 in-frame viral envelope proteins (large, middle and small), each of which possesses the hepatitis B virus surface antigen (HBsAg) determinant (Seeger C, Mason WS.Virology. 2015 May; 479-480:672-86). This envelope encloses a protein shell, or capsid, that is composed of 240 monomers of the core protein and each monomer possesses the hepatitis B virus core antigen (HBcAg or Cp) determinant. The capsid in turn encloses a partially double-stranded, relaxed circular DNA (rcDNA) form of the viral genome as well as a molecule of the viral polymerase. Upon entry onto susceptible cells (i.e. the hepatocytes) via the interaction of the large envelope protein with specific receptors on the hepatocellular membrane, the capsid is released into the cytoplasm and transported at the nuclear membrane. The rcDNA is then released into the nucleus and repaired by cellular polymerases into an episomal "minichromosome", termed covalently closed circular DNA (cccDNA), which represents the viral transcriptional template. The minus strand of the viral DNA encodes 3.5, 2.4, 2.1 and 0.7 kb mRNA species that are translated into structural (envelope and core) and nonstructural (polymerase, precure and X) proteins of the virus. Following transport into the cytoplasm, one of the 3.5 kb RNAs (termed pregenomic RNA) is selectively packaged into a nascent capsid by interacting with the core and polymerase proteins that have been translated from their respective mRNAs. Within these capsids, the viral polymerase reverse transcribes the pregenomic RNA into a single minus strand DNA molecule that serves as template for the viral polymerase-mediated DNA plus strand synthesis and the cohesive structure of the linear DNA intermediates converts them into a relaxed circular double stranded molecule. A fraction of these HBV DNA-containing "mature" capsids are transported back to the nucleus where second strand synthesis is completed and the ends of both strands are ligated, leading to amplification of the pool of cccDNA. Another fraction of the capsids binds to viral envelope proteins that have been independently translated and translocated to membranes of endoplasmic reticulum (ER)-like structures. Following binding, the enveloped capsids bud into the lumen of the ER and exit the cell as infectious virions to initiate new cycles of infection.

Thus, the HBV core protein and the related capsids are essential components and regulators of the HBV life cycle. The full-length core protein Cp183, or its N-terminal domain Cp149, predominantly assembles into a T = 4 icosahedral capsids. Due to its critical roles in capsid assembly, pregenomic RNA packaging, and cccDNA maintenance, it is not surprising that the HBV core protein and the related capsids have been widely recognized as attractive antiviral targets (Durantel D, Zoulim F; J Hepatol. 2016 Apr;64(1 Suppl):S117-S131).

According to World Health Organization (WHO) statistics, HBV infection is one of the major medical scourges of our time. As a sexually transmitted disease that is also transferred by intravenous drug abuse and from mother to infant at birth, over one third of the world's population has been infected by HBV at some point in their lives (Burns GS, Thompson AJ; Cold Spring Harb Perspect Med. 2014 Oct 30;4(12)). While most of these people have successfully cleared the virus, more than 240 million people remain persistently infected and almost 800,000 of these individuals die annually from the complications of chronic infection (i.e. cirrhosis and/or hepatocellular carcinoma). HBV infection is highly endemic in sub-Saharan Africa, the Pacific, and particularly Asia. Regions with high rates of chronic HBV infection also include the Middle East, the Indian subcontinent, areas of South and Central America, and the southern parts of Eastern and Central Europe. In recent years the number of chronic carriers has increased steadily in the western world as well, mostly because of the influx of immigrants from endemic areas. Additionally, HBV acts as a helper virus to hepatitis delta virus (HDV) and it should be noted that the more than 15 million people co-infected with HBV and HDV have an increased risk of rapid progression to cirrhosis and hepatic decompensation (Hughes, S.A. et al. Lancet 2011, 378, 73-85).

Well-tolerated vaccines that elicit neutralizing antibodies to HBsAg efficiently prevent de novo HBV infection, but have no therapeutic potential for the millions of people that are already persistently infected (Zoulim, Durantel D; Cold Spring Harb Perspect Med. 2015 Apr 1;5(4)). Therapy for these individuals mainly relies on direct acting antiviral (DAA) drugs (e.g. tenofovir, lamivudine, adefovir, entecavir or telbivudine) that suppress virus production but do not eradicate HBV from the liver, requiring lifelong treatment. Cohorts of patients still receive a therapy based on pegylated interferon-$\alpha$ (PEG-IFN-$\alpha$), which has the advantages of limited treatment duration and higher rates of HBsAg seroconversion but the relevant disadvantage of greater adverse effects. As such, the number of patients receiving PEG-IFN-α is progressively decreasing.

Different chemical classes of inhibitors targeting the encapsidation process of HBV (also termed capsid assembly modulators or CAMs) are under development, and they include heteroaryldihydropyrimidines (HAPs) and sulfamoylbenzamides (SBAs). For instance, Novira Therapeutics recently utilized a humanized mouse model of HBV infection to show that a combination of CAM and PEG-IFN-α has higher antiviral activity than that previously observed with DAAs. This class of CAM is now in Phase 2 clinical development, with compound NVR3-778 that is administered in patients in combination with DAAs or PEG-IFN-α (Gastroenterology 2018; 154: 652-662).

WO2013/006394, published on January 10, 2013, relates to a subclass of sulfamoyl-arylamides having general formula A, useful for the treatment of HBV infection:

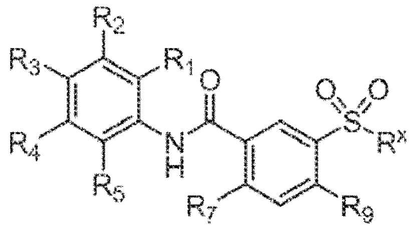

(A)

WO2013/096744, published on June 26, 2013, relates to sulfamoyl-arylamides of formula B active against HBV:

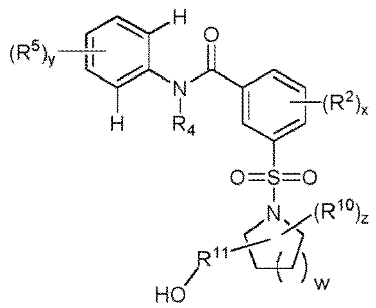

(B)

WO2014/106019, published on July 3, 2014, relates to compounds of formula C, useful as nucleocapsid assembly inhibitors for the treatment of viruses, especially but not exclusively, including pregenomic RNA encapsidation inhibitors of HBV for the treatment of Hepatitis B virus (HBV) infection and related conditions:

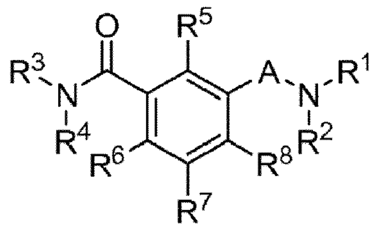

(C)

WO2014/165128, published on October 9, 2014, WO2015/109130 published on July 23, 2015, US2015274652, published on October 1, 2015, all relate to sulfamoyl-arylamides compounds active against HBV.

WO2015/120178, published on August 13, 2015, relates to sulfamoyl-arylamides compounds used in combinantion therapy with peginterferon alfa-2a, or another interferon analog for the treatment of HBV infection.

WO2016/089990, published on June 9, 2016, relates to sulfide alkyl and pyridyl reverse sulphonamide compounds for HBV treatment.

US2016185748, published on June 30, 2016, relates to pyridyl reverse sulfonamides for HBV treatment.

US2016151375, published on June 2, 2016 relates to sulfide alkyl compounds for HBV treatment. WO2017/001655A1 published on January 5, 2017, relates to cyclized sulfamoylarylamide derivatives.

Amongst the problems which HBV direct antivirals may encounter are toxicity, mutagenicity, lack of selectivity, poor efficacy, poor bioavailability, low solubility, off-target activity and to date no compounds from the above chemical classes has been approved as a drug for the treatment of HBV patients.

There is a need for additional HBV inhibitors that may overcome at least one of these disadvantages or that have additional advantages such as increased potency or an increased safety window.

The present invention provides small molecule drugs obtained through chemical modification of the known sulfamoyl arylamides derivatives. From the structural point of view, the distinguishing feature characterizing the sulfamoyl amides of the invention is the presence of an amino group ortho or para to the sulfamoyl group. This substitution pattern results in potent HBV inhibitors with improved pharmacokinetic properties, good kinetic solubility, stability in mouse and human hepatocytes, low in vivo clearance and positive liver-to-plasma concentration. Given the liver's key role in metabolic regulation and the fact that it is the principal tissue affected by hepatitis B disease, designing HBV inhibitors with hepatoselective distribution profiles is an important strategy in developing safe drug candidates (Tu M. et al., Current Topics in Medicinal Chemistry, 2013, 13, 857-866).

DESCRIPTION OF THE INVENTION

The compounds of this invention are inhibitors of hepatitis B virus (HBV).

It is therefore an object of the present invention a compound of general formula (I):

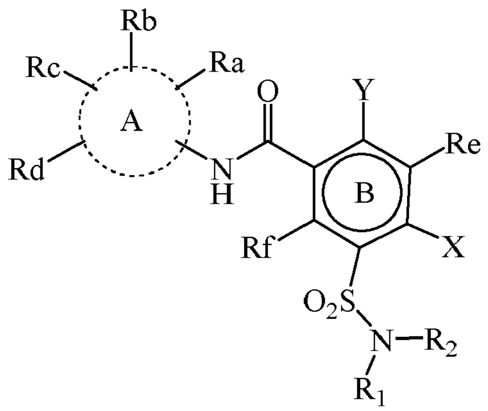

(I)

wherein:

A is a 6-membered aromatic or heteroaromatic ring;

B is a 6-membered aryl optionally containing one or more N atoms;

X is H or $NR_3R_4$;

Y is selected from the group consisting of hydrogen, halogen, $C_{1-6}$alkyl, $NH_2$, $NH(C_{1-6}alkyl)$, $N(CH_3)_2$, $NHC(O)CH_3$, OH, saturated or partially unsaturated $C_{3-7}$cycloalkyl, 5- or 6-membered heteroaryl and CN or is absent;

with the proviso that, when X is H, Y is selected form the group consisting of $NH_2$, $NH(C_{1-6}alkyl)$, $N(CH_3)_2$, $NHC(O)CH_3$;

$R_1$ and $R_2$ are each independently selected from H, linear or branched $C_{1-6}$alkyl, saturated or partially unsaturated $C_{3-7}$cycloalkyl, $C_{3-7}$heterocycloalkyl and heteroaryl, each of said linear or branched $C_{1-6}$alkyl, saturated or partially unsaturated $C_{3-7}$cycloalkyl, $C_{3-7}$heterocycloalkyl or heteroaryl group being optionally substituted with one or more substituents selected from OH, halogen, $NH_2$, $NH(C{=}O)OC_{1-6}alkyl$, $NH(C_{1-6}alkyl)$, $C_{1-6}$alkyl, $C_{3-7}$cycloalkyl, $C_{3-7}$heterocycloalkyl, $C_{1-6}$hydroxyalkyl, 5- or 6-membered heteroaryl, $C(=O)C_{1-6}alkyl$, $C(=O)OC_{1-6}alkyl$, $OC_{1-6}alkyl$, $O(CH_2)_nC_{3-10}$cycloalkyl and $O(CH_2)_nC_{3-10}$heterocycloalkyl;

or $R_1$ and $R_2$ taken together form with the N atom to which they are attached a saturated or partially unsaturated 3-10 membered heterocyclic ring optionally containing another heteroatom selected from N, O and S, said saturated or partially unsaturated 3-10 membered heterocyclic ring being optionally substituted with one or more substituents selected from OH, halogen, $C_{1-6}$alkyl, $C_{1-6}$haloalkyl and $(CH_2)_nR_5$;

each occurrence of n is independently 0, 1, 2, 3 or 4;

$R_3$ and $R_4$ are each independently H, or linear or branched $C_{1-3}$alkyl optionally substituted with one or more groups

selected from halogen, $NH_2$, $NHC_{1-6}$alkyl, $N(C_{1-6}alkyl)_2$, $NH(C=O)C_{1-6}$alkyl, $NH(C=O)OC_{1-6}$alkyl, $OC_{1-6}$alkyl, $O(CH_2)_nC_{3-10}$cycloalkyl and $O(CH_2)_nC_{3-10}$heterocycloalkyl, with the proviso that $NR_3R_4$ does not form a saturated, partially saturated or unsaturated heterocyclic ring;
$R_5$ is selected from the group consisting of OH, $NH_2$, $NH(CH_3)$, $N(CH_3)_2$, $NHC(O)CH_3$, CN, halo$C_{1-3}$alkyl, $C_{1-3}$alkoxy, halo$C_{1-3}$alkoxy, heterocyclic ring, aryl and heteroaryl;
Ra is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, NHC(O) $CH_3$, OH and CN; or is absent;
Rb is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rc is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rd is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Re is selected from the group consisting of hydrogen, halogen, $C_{1-3}$alkyl; or is absent;
Rf is hydrogen, halogen, $C_{1-3}$alkyl; or is absent;
provided that the compound is not 2-amino-N-(4-chloro-2-methylphenyl)-5-sulfamoylbenzamide or N-(2-methoxyphenyl)-2-(methylamino)-5-(piperidin-1-ylsulfonyl)benzamide;
and pharmaceutically acceptable salts, tautomers, isomers, stereoisomers thereof.

Preferably, A is phenyl. Preferably, B is phenyl. Preferably, A and B are both phenyl.

Preferably, X is $NR_3R_4$, wherein more preferably $R_3$ and $R_4$ are both H.

Preferably, Y is selected from the group consisting of: hydrogen, halogen (in particular Cl or Br), $C_{1-6}$alkyl (in particular methyl) and $NH_2$. In another preferred embodiment, X is hydrogen and Y is $NH_2$.

Preferably, $R_1$ and $R_2$ are each independently selected from: hydrogen, linear or branched $C_{1-6}$alkyl optionally substituted with halogen, saturated $C_{3-6}$cycloalkyl optionally substituted with OH or with $NH(C=O)OC_{1-6}$alkyl and $C_{3-6}$heterocycloalkyl optionally substituted with $NH(C=O)OC_{1-6}$alkyl. In a preferred embodiment, $R_1$ and $R_2$ taken together form with the N atom to which they are attached a saturated 4-6 membered heterocyclic ring optionally substituted with OH or with $CH_2OH$. More preferably, $R_1$ is hydrogen, methyl, or is selected from the group consisting of:

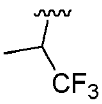, 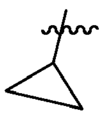, 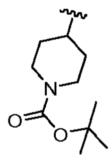, 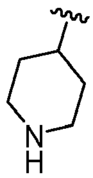, 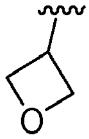, 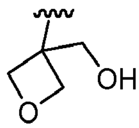, 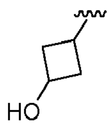, 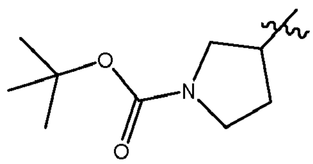,

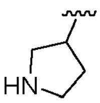, 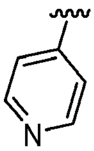, 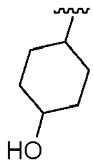, 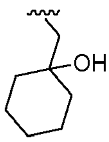, 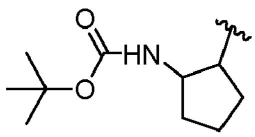, 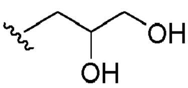, 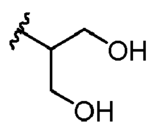, 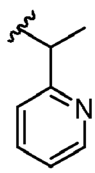,

and

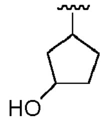.

Also preferably, $R_2$ is H or methyl.

Preferably, $R_3$ and $R_4$ are both H. Preferably, $R_5$ is OH. Preferably, Ra is H. Preferably Rb and Rd are each independently selected from the group consisting of: hydrogen, F, $CF_3$, CN, $CHF_2$, Cl and methyl. Preferably, Rc is F. Preferably, Re is hydrogen or $C_{1-3}$alkyl, in particular methyl. Preferably, Rf is hydrogen.

In a preferred embodiment, the compound of the invention has general formula (Ia):

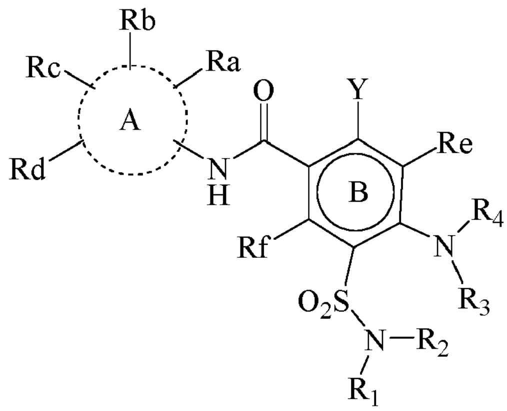

(Ia)

wherein:

A is a 6-membered aromatic or heteroaromatic ring;
B is a 6-membered aryl optionally containing one or more N atoms;
Y is selected from the group consisting of hydrogen, halogen, $C_{1-3}$alkyl, $NH_2$, NH($C_{1-6}$alkyl), $N(CH_3)_2$, $NHC(O)CH_3$, OH, saturated or partially unsaturated $C_{3-7}$cycloalkyl, 5- or 6-membered heteroaryl and CN or is absent;
$R_1$ and $R_2$ are each independently selected from H, linear or branched $C_{1-6}$alkyl, saturated or partially unsaturated $C_{3-7}$cycloalkyl, $C_{3-7}$heterocycloalkyl and heteroaryl, each of said linear or branched $C_{1-6}$alkyl, saturated or partially unsaturated $C_{3-7}$cycloalkyl, $C_{3-7}$heterocycloalkyl or heteroaryl group being optionally substituted with one or more substituents selected from OH, halogen, $NH_2$, NH(C=O)O$C_{1-6}$alkyl, NH($C_{1-6}$alkyl), $C_{1-6}$alkyl, $C_{3-7}$cycloalkyl, $C_{3-7}$heterocycloalkyl, $C_{1-6}$hydroxyalkyl, 5- or 6-membered heteroaryl, C(=O)$C_{1-6}$alkyl, C(=O)O$C_{1-6}$alkyl, O$C_{1-6}$alkyl, $O(CH_2)_nC_{3-10}$cycloalkyl and $O(CH_2)_nC_{3-10}$heterocycloalkyl;
or $R_1$ and $R_2$ taken together form with the N atom to which they are attached a saturated or partially unsaturated 3-10 membered heterocyclic ring optionally containing another heteroatom selected from N, O and S, said saturated or partially unsaturated 3-10 membered heterocyclic ring being optionally substituted with one or more substituents selected from OH, halogen, $C_{1-6}$alkyl, $C_{1-6}$haloalkyl, $(CH_2)_nR_5$;
each occurrence of n is independently 0, 1, 2, 3 or 4;
$R_3$ and $R_4$ are each independently H or linear or branched $C_{1-3}$alkyl optionally substituted with one or more groups selected from halogen, $NH_2$, NH$C_{1-6}$alkyl, N($C_{1-6}$alkyl)$_2$, NH(C=O)$C_{1-6}$alkyl, NH(C=O)O$C_{1-6}$alkyl, O$C_{1-6}$alkyl, $O(CH_2)_nC_{3-10}$cycloalkyl and $O(CH_2)_nC_{3-10}$heterocycloalkyl, with the proviso that $NR_3R_4$does not form a saturated, partially saturated or unsaturated heterocyclic ring;
$R_5$ is selected from the group consisting of OH, $NH_2$, $NH(CH_3)$, $N(CH_3)_2$, $NHC(O)CH_3$, CN, halo$C_{1-3}$alkyl, $C_{1-3}$alkoxy, halo$C_{1-3}$alkoxy, heterocyclic ring, aryl and heteroaryl;
Ra is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rb is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rc is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rd is selected from the group consisting hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Re is selected from the group consisting of hydrogen, halogen and $C_{1-3}$alkyl; or is absent;
Rf is hydrogen, halogen and $C_{1-3}$alkyl; or is absent;
and pharmaceutically acceptable salts, tautomers, isomers, stereoisomers thereof.

In a further preferred embodiment, the invention relates to a compound of formula (Ia) wherein:

A is a 6-membered aromatic or heteroaromatic ring;
B is a 6-membered aryl optionally containing one or more N atoms;
Y is selected from the group consisting of hydrogen, halogen, $C_{1-3}$alkyl, $NH_2$, NH($C_{1-6}$alkyl), $N(CH_3)_2$, $NHC(O)CH_3$, OH and CN or is absent;
$R_1$ is H, linear or branched $C_{1-6}$alkyl, cyclopropyl, cyclobutyl, cyclopentyl, cyclohexyl, piperidinyl, pirrolidinyl, oxetanyl, tetrahydrofuranyl, pyridinyl, said $C_{1-6}$alkyl, cyclopropyl, cyclobutyl, cyclopentyl, cyclohexyl, piperidinyl, pirrolidinyl,

oxetanyl, tetrahydrofuranyl or pyridinyl being optionally substituted with one or more substituents selected from OH, halogen, $NH_2$, $NH(C{=}O)OC_{1\text{-}6}alkyl$, $NH(C_{1\text{-}6}alkyl)$, $C_{1\text{-}6}hydroxyalkyl$, $C({=}O)C_{1\text{-}6}alkyl$, $C({=}O)OC_{1\text{-}6}alkyl$, $OC_{1\text{-}6}alkyl$;
$R_2$ is H or methyl;
or $R_1$ and $R_2$ taken together form with the N atom to which they are attached a heterocyclic ring selected from piperidine, pirrolidine, morpholine, thiomorpholine and piperazine, said ring being optionally substituted with one or more substituents selected from halogen, $C_{1\text{-}3}alkyl$, OH and $CH_2R_5$;
$R_3$ and $R_4$ are each independently H or $C_{1\text{-}3}alkyl$; in particular hydrogen or methyl;
$R_5$ is selected from the group consisting of OH, $NH_2$, $NH(CH_3)$, $N(CH_3)_2$, $NHC(O)CH_3$, CN, halo$C_{1\text{-}3}alkyl$, $C_{1\text{-}3}alkoxy$, halo$C_{1\text{-}3}alkoxy$, heterocyclic ring, aryl and heteroaryl;
Ra is selected from the group consisting of hydrogen, halogen, $C_{1\text{-}3}alkyl$, halo$C_{1\text{-}3}alkyl$ and CN; or is absent;
Rb is selected from the group consisting of hydrogen, halogen, $C_{1\text{-}3}alkyl$, halo$C_{1\text{-}3}alkyl$ and CN; or is absent;
Rc is selected from the group consisting of hydrogen, halogen, $C_{1\text{-}3}alkyl$, halo$C_{1\text{-}3}alkyl$ and CN; or is absent;
Rd is selected from the group consisting of hydrogen, halogen, $C_{1\text{-}3}alkyl$, halo$C_{1\text{-}3}alkyl$ and CN; or is absent;
Re is selected from the group consisting of hydrogen, halogen, $C_{1\text{-}3}alkyl$, or is absent;
Rf is hydrogen or is absent;
and pharmaceutically acceptable salts, tautomers, isomers, stereoisomers thereof.

In a preferred embodiment, the compounds of the invention have formula (Ia), wherein:

A is a 6-membered aromatic or heteroaromatic ring;
B is a 6-membered aryl optionally containing one or more N atoms;
Y is selected from the group consisting of hydrogen, halogen, $C_{1\text{-}3}alkyl$, or is absent;
$R_1$ is hydrogen, methyl, or is selected from the group consisting of:

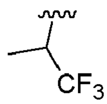, 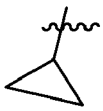, 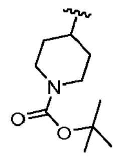,

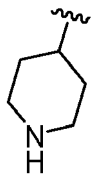, 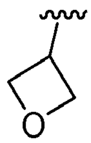, 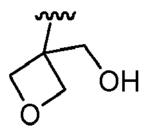, 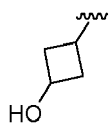, 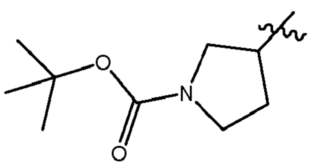, 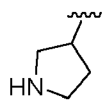, 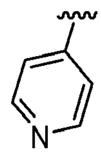, 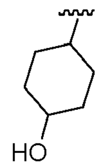,

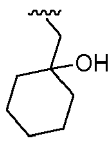, 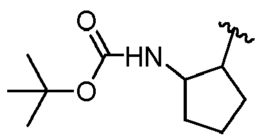, 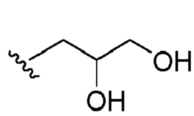, 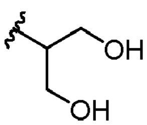, 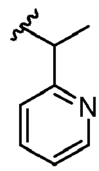,

and

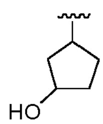;

$R_2$ is H or methyl;
or $R_1$ and $R_2$ taken together form with the N atom to which they are attached a heterocyclic ring selected from the group consisting of:

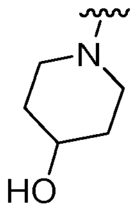,
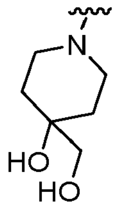,
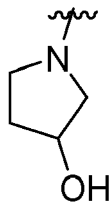

and

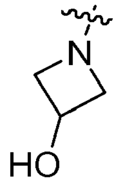;

$R_3$ and $R_4$ are each independently H or $C_{1-3}$alkyl; in particular hydrogen or methyl;
Ra is selected from the group consisting of hydrogen, halogen, $C_{1-3}$alkyl, halo$C_{1-3}$alkyl and CN; or is absent;
Rb is selected from the group consisting of hydrogen, halogen, $C_{1-3}$alkyl, halo$C_{1-3}$alkyl and CN; or is absent;
Rc is selected from the group consisting of hydrogen, halogen, $C_{1-3}$alkyl, halo$C_{1-3}$alkyl and CN; or is absent;
Rd is selected from the group consisting of hydrogen, halogen, $C_{1-3}$alkyl, halo$C_{1-3}$alkyl and CN; or is absent;
Re is selected from the group consisting of hydrogen, halogen, $C_{1-3}$alkyl, or is absent;
Rf is hydrogen; or is absent;
and pharmaceutically acceptable salts, tautomers, isomers, stereoisomers thereof.

Preferably, A is phenyl or pyridyl. Preferably, B is phenyl or pyridyl. Preferably, A is phenyl and B is phenyl.

Preferably, at least one of Ra, Rb, Rc and Rd is F and the other(s) is/are hydrogen.

All the definitions of substituents, such as for example "alkyl", "alkoxy", "aryl", "heteroaryl" and so on, are reported herein below and apply to formula (I) and formula (Ia).

In a further embodiment, the invention relates to a compound of formula (Ia) wherein $R_1$, $R_2$, $R_3$, $R_4$, $R_5$, Re, Rf and Y are as defined above and A is phenyl or pyridyl, B is phenyl or pyridyl; and Ra is selected from the group consisting of hydrogen, halogen, $C_{1-3}$alkyl, halo$C_{1-3}$alkyl and CN; Rb is selected from the group consisting of hydrogen, halogen, $C_{1-3}$alkyl, halo$C_{1-3}$alkyl and CN; Rc is selected from the group consisting of hydrogen, halogen, $C_{1-3}$alkyl, halo$C_{1-3}$alkyl and CN; Rd is selected from the group consisting of hydrogen, halogen, $C_{1-3}$alkyl, halo$C_{1-3}$alkyl and CN; and pharmaceutically acceptable salts, tautomers, isomers, stereoisomers thereof.

In a further embodiment, the invention relates to a compound of formula (Ia) wherein $R_1$, $R_2$, $R_3$, $R_4$, $R_5$, Re, Rf and Y are as defined above, and A is phenyl, B is phenyl; and
Ra is selected from the group consisting of hydrogen, halogen, $C_{1-3}$alkyl, halo$C_{1-3}$alkyl and CN; Rb is selected from the group consisting of hydrogen, halogen, $C_{1-3}$alkyl, halo$C_{1-3}$alkyl and CN; Rc is selected from the group consisting of hydrogen, halogen, $C_{1-3}$alkyl, halo$C_{1-3}$alkyl and CN; Rd is selected from the group consisting of hydrogen, halogen, $C_{1-3}$alkyl, halo$C_{1-3}$alkyl and CN; and pharmaceutically acceptable salts, tautomers, isomers, stereoisomers thereof.

In a further embodiment the invention relates to a compound of formula (Ia) wherein $R_1$, $R_2$, $R_3$, $R_4$, $R_5$, Re, Rf and Y are as defined above and A is phenyl; B is phenyl; at least one of Ra, Rb, Rc and Rd are F and the other(s) is/are hydrogen; and pharmaceutically acceptable salts, tautomers, isomers, stereoisomers thereof.

In a further embodiment the invention relates to a compound of formula (Ia) wherein $R_1$, $R_2$, $R_3$, $R_4$, $R_5$, Re, Rf and Y are as defined above and A is phenyl; B is phenyl; at least two of Ra, Rb, Rc and Rd are F and the other(s) is/are hydrogen; and pharmaceutically acceptable salts, tautomers, isomers, stereoisomers thereof.

In a further embodiment, the invention relates to a compound having formula (Ia) wherein $R_1$, $R_2$, $R_3$, $R_4$, $R_5$, Re, Rf and Y are as defined above and A is phenyl; B is phenyl; at least three of Ra, Rb, Rc and Rd are F and the other(s) is/are hydrogen; and pharmaceutically acceptable salts, tautomers, isomers, stereoisomers thereof.

In a further embodiment, the invention relates to a compound of formula (Ia) wherein $R_1$, $R_2$, $R_3$, $R_4$, $R_5$, Re, Rf and Y are as defined above and A is phenyl, B is phenyl; Ra, Rc and Rd are each independently hydrogen or F; Rb is selected from the group consisting of methyl, Cl, $CF_3$, $CHF_2$ and CN; and pharmaceutically acceptable salts, tautomers, isomers, stereoisomers thereof.

In a further embodiment, the invention relates to a compound wherein $R_1$, $R_2$, $R_4$, $R_5$, Re, Rf and Y are as

defined above and A is phenyl, B is phenyl; $R_3$ are each hydrogen; and Ra is selected from the group consisting of hydrogen, halogen, $C_{1-3}$alkyl, halo$C_{1-3}$alkyl and CN; Rb is selected from the group consisting of hydrogen, halogen, $C_{1-3}$alkyl, halo$C_{1-3}$alkyl and CN; Rc is selected from the group consisting of hydrogen, halogen, $C_{1-3}$alkyl, halo$C_{1-3}$alkyl and CN; Rd is selected from the group consisting of hydrogen, halogen, $C_{1-3}$alkyl, halo$C_{1-3}$alkyl and CN; and pharmaceutically acceptable salts, tautomers, isomers, stereoisomers thereof.

Preferred compounds exhibit an HBV inhibition and/or an $EC_{50}$, as defined hereinbelow, greater than 50%. In particular, preferred compounds are selected from the following list:

- 4-amino-3-(N-methylsulfamoyl)-N-(3,4,5-trifluorophenyl)benzamide;
- 4-amino-3-sulfamoyl-*N*-(3,4,5-trifluorophenyl)benzamide;
- 4-amino-N-(3,4-difluorophenyl)-3-sulfamoylbenzamide;
- 4-amino-2-chloro-5-sulfamoyl-N-(3,4,5-trifluorophenyl)benzamide;
- 4-amino-2-bromo-5-sulfamoyl-N-(3,4,5-trifluorophenyl)benzamide;
- 4-amino-N-(4-fluoro-3-(trifluoromethyl)phenyl)-3-sulfamoylbenzamide;
- 4-amino-N-(3-cyano-4-fluorophenyl)-3-sulfamoylbenzamide;
- 4-amino-N-(3-(difluoromethyl)-4-fluorophenyl)-3-sulfamoylbenzamide;
- 4-amino-N-(3-chloro-4-fluorophenyl)-3-sulfamoylbenzamide;
- 4-amino-N-(4-fluoro-3-methylphenyl)-3-sulfamoylbenzamide;
- 4-amino-2-methyl-5-sulfamoyl-N-(3,4,5-trifluorophenyl)benzamide;
- (R)-4-amino-N-(3,4,5-trifluorophenyl)-3-(N-(1,1,1-trifluoropropan-2-yl)sulfamoyl)benzamide;
- (S)-4-amino-N-(3,4,5-trifluorophenyl)-3-(N-(1,1,1-trifluoropropan-2-yl)sulfamoyl)benzamide;
- 4-amino-3-(N-cyclopropylsulfamoyl)-N-(3,4,5-trifluorophenyl)benzamide;
- *trans*-4-amino-3-(N-(4-hydroxycyclohexyl)sulfamoyl)-N-(3,4,5-trifluorophenyl)benzamide;
- *cis*-4-amino-3-(N-(4-hydroxycyclohexyl)sulfamoyl)-N-(3,4,5-trifluorophenyl)benzamide;
- *trans*-4-amino-5-(N-(4-hydroxycyclohexyl)sulfamoyl)-2-methyl-N-(3,4,5-trifluorophenyl) benzamide;
- *cis*-4-amino-3-(N-3-hydroxycyclobutyl)sulfamoyl)-N-(3,4,5-trifluorophenyl)benzamide;
- *trans*-4-amino-3-(N-3-hydroxycyclobutyl)sulfamoyl)-N-(3,4,5-trifluorophenyl)benzamide;
- 4-amino-3-(N-((1R,3R)-3-hydroxycyclopentyl)sulfamoyl)-N-(3,4,5-trifluorophenyl)benzamide;
- 4-amino-3-((4-hydroxypiperidin-1-yl)sulfonyl)-N-(3,4,5-trifluorophenyl)benzamide;
- 4-amino-3-(N-(oxetan-3-yl)sulfamoyl)-N-(3,4,5-trifluorophenyl)benzamide;
- *tert*-butyl(S)-3-((2-amino-5-((3,4,5-trifluorophenyl)carbamoyl)phenyl)sulfonamido) pyrrolidine-1-carboxylate;
- 4-amino-3-methyl-5-sulfamoyl-*N*-(3,4,5-trifluorophenyl)benzamide;
- 4-amino-3-(N-(3-(hydroxymethyl)oxetan-3-yl)sulfamoyl)-N-(3,4,5-trifluorophenyl)benzamide;
- 4-amino-3-(N-((1-hydroxycyclohexyl)methyl)sulfamoyl)-N-(3,4,5-trifluorophenyl)benzamide;
- 4-amino-N-(4-fluoro-3-methylphenyl)-2-methyl-5-sulfamoylbenzamide;
- 4-amino-5-(N-((1R,4R)-4-hydroxycyclohexyl)sulfamoyl)-2-methyl-N-(3,4,5-trifluorophenyl) benzamide;
- trans-4-amino-N-(3-chloro-4-fluorophenyl)-3-(N-(4-hydroxycyclohexyl)sulfamoyl)benzamide;
- 4-amino-N-(3-(difluoromethyl)-4-fluorophenyl)-3-(N-((1r1R,4r4R)-4-hydroxycyclohexyl) sulfamoyl)benzamide;
- trans-4-amino-N-(3-(difluoromethyl)-4-fluorophenyl)-3-(N-(4-hydroxycyclohexyl)sulfamoyl) benzamide;
- 4-amino-3-(N-((1S,3R)-3-hydroxycyclopentyl)sulfamoyl)-N-(3,4,5-trifluorophenyl)benzamide;
- 4-amino-3-(N-((1R,3S)-3-hydroxycyclopentyl)sulfamoyl)-N-(3,4,5-trifluorophenyl)benzamide;
- 4-amino-3-((4-hydroxy-4-(hydroxymethyl)piperidin-1-yl)sulfonyl)-N-(3,4,5-trifluorophenyl)benzamide;
- tert-butyl((1R,2S)-2-((2-amino-5-((3,4,5-trifluorophenyl)carbamoyl)phenyl)sulfonamido) cyclopentyl)carbamate;
- tert-butyl((1S,2R)-2-((2-amino-5-((3,4,5-trifluorophenyl)carbamoyl)phenyl)sulfonamido) cyclopentyl)carbamate;
- 4-amino-3-((3-hydroxypyrrolidin-1-yl)sulfonyl)-N-(3,4,5-trifluorophenyl)benzamide;
- 4-amino-N-(3-chloro-4-fluorophenyl)-3-((4-hydroxypiperidin-1-yl)sulfonyl)benzamide;
- 4-amino-N-(3-chloro-4-fluorophenyl)-3-((3-hydroxyazetidin-1-yl)sulfonyl)benzamide;
- 4-amino-3-(N-(2,3-dihydroxypropyl)sulfamoyl)-N-(3,4,5-trifluorophenyl)benzamide;
- trans-4-amino-3-(N-(3-hydroxycyclopentyl)sulfamoyl)-N-(3,4,5-trifluorophenyl)benzamide;
- trans-2-amino-5-(N-(4-hydroxycyclohexyl)sulfamoyl)-N-(3,4,5-trifluorophenyl)benzamide;
- 2-amino-5-((4-hydroxypiperidin-1-yl)sulfonyl)-N-(3,4,5-trifluorophenyl)benzamide;
- (R)-4-amino-2-methyl-N-(3,4,5-trifluorophenyl)-5-(N-(1,1,1-trifluoropropan-2-yl)sulfamoyl) benzamide;
- (S)-4-amino-2-methyl-N-(3,4,5-trifluorophenyl)-5-(N-(1,1,1-trifluoropropan-2-yl)sulfamoyl) benzamide;
- 4-amino-N-(3-chloro-4,5-difluorophenyl)-2-methyl-5-sulfamoylbenzamide;
- 4-amino-N-(4-fluoro-3-(trifluoromethyl)phenyl)-2-methyl-5-sulfamoylbenzamide;
- 4-amino-N-(3-(difluoromethyl)-4-fluorophenyl)-2-methyl-5-sulfamoylbenzamide;
- 4-amino-N-(3-cyano-4-fluorophenyl)-2-methyl-5-sulfamoylbenzamide;
- 4-amino-N-(3-chloro-4-fluorophenyl)-2-methyl-5-sulfamoylbenzamide;

and pharmaceutically acceptable salts, tautomers, isomers, stereoisomers thereof.

In particular, compound 4-amino-3-sulfamoyl-*N*-(3,4,5-trifluorophenyl)benzamide is preferred.

It is an object of the invention a compound as defined above for medical use. Preferably, the compound as defined above is for use in the treatment and/or prevention of a HBV infection.

In a preferred embodiment, the compound for use in the treatment and/or prevention of a HBV infection has general formula (I):

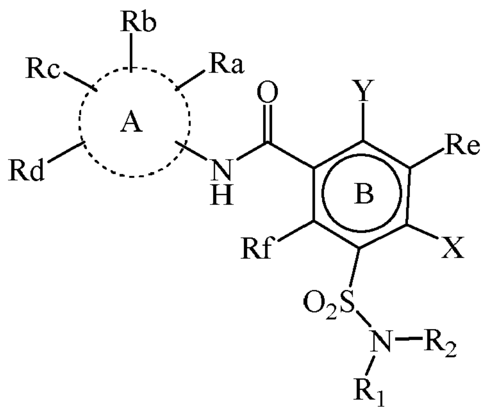

(I)

wherein:

A is a 6-membered aromatic or heteroaromatic ring;
B is a 6-membered aryl optionally containing one or more N atoms;
X is H or $NR_3R_4$;
Y is selected from the group consisting of hydrogen, halogen, $C_{1-6}$alkyl, $NH_2$, $NH(C_{1-6}alkyl)$, $N(CH_3)_2$, $NHC(O)CH_3$, OH, saturated or partially unsaturated $C_{3-7}$cycloalkyl, 5- or 6-membered heteroaryl and CN or is absent;
with the proviso that, when X is H, Y is selected form the group consisting of $NH_2$, $NH(C_{1-6}alkyl)$, $N(CH_3)_2$, $NHC(O)CH_3$;
$R_1$ and $R_2$ are each independently selected from H, linear or branched $C_{1-6}$alkyl, saturated or partially unsaturated $C_{3-7}$cycloalkyl, $C_{3-7}$heterocycloalkyl and heteroaryl, each of said linear or branched $C_{1-6}$alkyl, saturated or partially unsaturated $C_{3-7}$cycloalkyl, $C_{3-7}$heterocycloalkyl or heteroaryl group being optionally substituted with one or more substituents selected from OH, halogen, $NH_2$, $NH(C{=}O)OC_{1-6}$alkyl, $NH(C_{1-6}alkyl)$, $C_{1-6}$alkyl, $C_{3-7}$cycloalkyl, $C_{3-7}$heterocycloalkyl, $C_{1-6}$hydroxyalkyl, 5- or 6-membered heteroaryl, $C(=O)C_{1-6}$alkyl, $C(=O)OC_{1-6}$alkyl, $OC_{1-6}$alkyl, $O(CH_2)_nC_{3-10}$cycloalkyl and $O(CH_2)_nC_{3-10}$heterocycloalkyl;
or $R_1$ and $R_2$ taken together form with the N atom to which they are attached a saturated or partially unsaturated 3-10 membered heterocyclic ring optionally containing another heteroatom selected from N, O and S, said saturated or partially unsaturated 3-10 membered heterocyclic ring being optionally substituted with one or more substituents selected from OH, halogen, $C_{1-6}$alkyl, $C_{1-6}$haloalkyl and $(CH_2)_nR_5$;
each occurrence of n is independently 0, 1, 2, 3 or 4;
$R_3$ and $R_4$ are each independently H, or linear or branched $C_{1-3}$alkyl optionally substituted with one or more groups selected from halogen, $NH_2$, $NHC_{1-6}$alkyl, $N(C_{1-6}alkyl)_2$, $NH(C{=}O)C_{1-6}$alkyl, $NH(C{=}O)OC_{1-6}$alkyl, $OC_{1-6}$alkyl, $O(CH_2)_nC_{3-10}$cycloalkyl and $O(CH_2)_nC_{3-10}$heterocycloalkyl, with the proviso that $NR_3R_4$ does not form a saturated, partially saturated or unsaturated heterocyclic ring;
$R_5$ is selected from the group consisting of OH, $NH_2$, $NH(CH_3)$, $N(CH_3)_2$, $NHC(O)CH_3$, CN, halo$C_{1-3}$alkyl, $C_{1-3}$alkoxy, halo$C_{1-3}$alkoxy, heterocyclic ring, aryl and heteroaryl;
Ra is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, NHC(O) $CH_3$, OH and CN; or is absent;
Rb is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rc is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rd is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Re is selected from the group consisting of hydrogen, halogen, $C_{1-3}$alkyl; or is absent;
Rf is hydrogen, halogen, $C_{1-3}$alkyl; or is absent;

and pharmaceutically acceptable salts, tautomers, isomers, stereoisomers thereof.

It is a further object of the invention a compound as defined above for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection. Preferably, the compound as defined above is for use in combination with at least one further therapeutic agent. Preferably, said use in combination comprises the administration of at least one therapeutic agent.

It is an object of the invention a pharmaceutical composition comprising the compound as defined above, alone or in combination with at least one further therapeutic agent, and at least one pharmaceutically acceptable excipient.

Preferably, the at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof.

Preferably, the therapeutic vaccine is selected from: HBsAG-HBIG, HB-Vac, ABX203, NASVAC, GS-4774, GX- 110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell.

Preferably, the RNA interference therapeutic is a siRNA, a ddRNA or a shRNA. Preferably, the RNA interference therapeutic is selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404.

Preferably, the immunomodulator is a TLR agonist. Preferably the TLR agonist is a TLR7, TLR8 or TLR9 agonist. Preferably, the TLR7, TLR8 or TLR9 agonist is selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598.

Preferably, the RIG-I modulator is SB-9200. Preferably, the IL agonist or other immune acting protein is INO-9112 or recombinant IL12. Preferably, the immune checkpoint modulator/inhibitor is BMS-936558 (Opdivo (nivolumab)) or pembrolizumab. Preferably, the HBV entry inhibitor is Myrcludex B, IVIG-Tonrol or GC-1102.

Preferably, the cccDNA modulator is selected from: a direct cccDNA inhibitor, an inhibitor of cccDNA formation or maintenance, a cccDNA epigenetic modifier and an inhibitor of cccDNA transcription.

Preferably, the capsid assembly inhibitor/modulator, core or X protein targeting agent, direct cccDNA inhibitor, inhibitor of cccDNA formation or maintenance, or cccDNA epigenetic modifier is selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389.

Preferably, the interferon or modified interferon is selected from: interferon alpha (IFN-$\alpha$), pegylated interferon alpha (PEG-IFN-$\alpha$), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-$\lambda$), peginterferon lambda-1, interferon omega, interferon tau, interferon gamma (IFN-$\gamma$), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter. Particularly preferred are: peginterferon alpha-2a, peginterferon alpha-2b, glycosylated interferon alpha-2b, peginterferon beta-la, and peginterferon lambda-1. More particularly preferred is peginterferon alpha-2a. Preferably, the HBV antiviral of distinct or unknown mechanism is selected from: AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259. Preferably, the cyclophilin inhibitor is selected from: OCB-030 (NVP-018), SCY-635, SCY-575 and CPI-431-32.

Preferably, said HBV polymerase inhibitor is selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir. Preferably, tenofovir is in a salt form. Preferably, tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157.

Preferably, the dinucleotide is SB9200. Preferably, the SMAC inhibitor is Birinapant. Preferably, the HDV targeting agent is Lonafamib.

Preferably, the HBV RNA destabilizer or other small-molecule inhibitor of HBV protein expression is RG7834 or AB-452.

Preferably, the at least one further therapeutic agent is an agent useful in the treatment and prevetion of hepatitis B. Preferably, the at least one further therapeutic agent is an anti-HDV agent, an anti-HCV agent and/or an anti-HIV agent.

Preferably the at least one further therapeutic agent is selected from the group consisting of: HBV polymerase inhibitor, interferon, viral entry inhibitor, BAY 41-4109, reverse transcriptase inhibitor, a TLR-agonist, AT-61 ((E)-N-(1-chloro-3-oxo-1-phenyl-3-(piperidin-1-yl)prop-1-en-2-yl)benzamide), AT-130 ((E)-N-(1-bromo-1-(2-methoxyphenyl)-3-oxo-3-(piperidin-1-yl)prop-1-en-2-yl)-4-nitrobenzamide), and a combination thereof, wherein the HBV polymerase inhibitor is preferably at least one of Lamivudine, Entecavir, Tenofovir, Adefovir, Telbivudine, Clevudine; and wherein the TLR agonist is preferably selected from the group consisting of SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-purin-9-yl)propyl][3-(4-morpholinyl)propyl]amino}methyl)phenyl]acetate) and a combination thereof.

Preferably, the compound of the invention is for use in combination with one, two or more further therapeutic agent(s) as defined above.

Preferably, the pharmaceutical composition of the invention comprises one, two or more further therapeutic agent(s) as defined above.

The pharmaceutical composition defined above is preferably intended for use in the treatment and/or prevention of a HBV infection. Preferably, the pharmaceutical composition of the invention is for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

In an embodiment, the invention provides a kit comprising at least one pharmaceutically acceptable vial or container containing one or more doses of a compound of the invention or of a pharmaceutical composition of the invention and optionally a) instructions for use thereof in mammals and/or b) an infusion bag or container containing a pharmaceutically acceptable diluent. It is a further object of the invention a process for the synthesis of a compound of general formula (I) or (Ia).

It is a further object of the invention a pharmaceutical composition comprising an effective amount of one or more compounds as defined above or a pharmaceutically acceptable prodrug thereof, alone or in combination with other active compounds, and at least one pharmaceutically acceptable excipient.

The present invention includes within its scope prodrugs of the compounds of formula (I) or (Ia) above. In general, such prodrugs will be functional derivatives of the compounds of the invention which are readily convertible *in vivo* into the required compound of formula (I) or (Ia). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The disclosure also includes all suitable isotopic variations of a compound of the disclosure. Examples of isotopes that can be incorporated into compounds of the disclosure include isotopes such as $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$, respectively. Certain isotopic variations of the disclosure, for example, those in which a radioactive isotope such as $^{3}H$ or $^{14}C$ is incorporated, are useful in drug and/or substrate tissue distribution studies. Further, substitution with isotopes such as deuterium $^{2}H$, may afford certain therapeutic advantages resulting from greater metabolic stability. Isotopic variations of the compounds of the disclosure can generally be prepared by conventional procedures such as by the illustrative methods or by the preparations described in the examples hereafter using appropriate isotopic variations of suitable reagents. The present invention includes within its scope solvates of the compounds of formula (I) or (Ia) and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and both tautomeric forms are intended to be encompassed by the scope of the invention, even though only one tautomeric structure is depicted. The compounds may exist in different isomeric forms, all of which are encompassed by the present invention.

When any variable (e.g. $R_3$ and $R_4$, etc.) occurs more than one time in any constituent, its definition on each

occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

It is also understood that the expression "is absent" (referring to a given substituent) means that the atom to which the substituent would be bound is a heteroatom, preferably nitrogen, which is comprised in a heteroaryl ring, such as a pyridine or a pyrimidine ring.

The expressions "one or more substituents" refer in particular to 1, 2, 3, 4 or more substituents, in particular to 1, 2, 3 or 4 substituents, more in particular to 1, 2 or 3 substituents.

As used herein, "alkyl" is intended to include both branched and straight-chain saturated aliphatic hydrocarbon groups having the specified number of carbon atoms. For example, "$C_{1-6}$alkyl" is defined to include groups having 1, 2, 3, 4, 5 or 6 carbons in a linear or branched arrangement. For example, "$C_{1-6}$ alkyl" specifically includes methyl, ethyl, *n*-propyl, *i*-propyl, *n*-butyl, *t*-butyl, *i*-butyl, pentyl, hexyl, and so on. "$C_{1-4}$alkyl" is defined to include groups having 1, 2, 3 or 4 carbons in a linear or branched arrangement. "$C_{1-3}$alkyl" is defined to include groups having 1, 2, or 3 carbons in a linear or branched arrangement. Preferred alkyl groups are methyl, ethyl, *i*-propyl or *t*-butyl.

As used herein, "alkoxy" represents an alkyl group of indicated number of carbon atoms attached through an oxygen bridge. "Alkoxy" therefore encompasses the definitions of alkyl above. $C_{1-6}$ alkoxy group is preferably a linear or branched $C_{1-4}$ alkoxy group, more preferably a $C_{1-3}$ alkoxy group, still more preferably a $C_{1-2}$ alkoxy group. Examples of suitable alkoxy groups include, but are not limited to methoxy, ethoxy, *n*-propoxy, *i*-propoxy, *n*-butoxy, *s*-butoxy or *t*-butoxy. The preferred alkoxy group is methoxy.

As used herein, the terms "halo$C_{1-6}$alkyl" and "halo$C_{1-6}$alkoxy" mean a $C_{1-6}$alkyl or $C_{1-6}$alkoxy group in which one or more (in particular, 1 to 3) hydrogen atoms have been replaced by halogen atoms, especially fluorine or chlorine atoms. Halo$C_{1-6}$alkoxy group is preferably a linear or branched halo$C_{1-4}$alkoxy group, more preferably a halo$C_{1-3}$alkoxy, even more prefarably a halo$C_{1-2}$alkoxy group. Halo$C_{1-6}$alkyl group is preferably a linear or branched halo$C_{1-3}$alkyl group, more preferably a halo$C_{1-2}$alkyl group for example, $CF_3$, $CHF_2$, $CH_2F$, $CH_2CH_2F$, $CH_2CHF_2$, $CH_2CF_3$, $CH(CH_3)CF_3$, $OCF_3$, $OCHF_2$, $OCH_2F$, $OCH_2CH_2F$, $OCH_2CHF_2$ or $OCH_2CF_3$, and most especially $CF_3$, $CHF_2$, $CH(CH_3)CF_3$, $OCF_3$ or $OCHF_2$.

As used herein, the term "$C_{1-6}$hydroxyalkyl" means a $C_{1-6}$alkyl group in which one or more (in particular, 1 to 3) hydrogen atoms have been replaced by hydroxy groups. Illustrative examples include, but are not limited to $CH_2OH$, $CH_2CH_2OH$, $CH(CH)_3OH$ and $CHOHCH_2OH$.

As used herein, the term "$C_{2-6}$alkenyl" refers to a non-aromatic hydrocarbon radical, straight or branched, containing from 2 to 6 carbon atoms and at least one carbon to carbon double bond. Preferably one carbon to carbon double bond is present, and up to four non-aromatic carbon-carbon double bonds may be present. Alkenyl groups include, but are not limited to ethenyl, propenyl, butenyl and 2-methylbutenyl. The straight or branched portion of the alkenyl group may contain double bonds and may be substituted if a substituted alkenyl group is indicated. Preferred alkenyl groups are ethenyl and propenyl.

As used herein, the term "aryl" means a monocyclic or polycyclic aromatic ring comprising carbon atoms and hydrogen atoms. If indicated, such aromatic ring may include one or more heteroatoms, then also referred to as "heteroaryl" or "heteroaromatic ring", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Illustrative examples of aryl groups are optionally substituted phenyl. Illustrative examples of heteroaryl groups according to the invention include optionally substituted thiophene, oxazole, thiazole, thiadiazole, imidazole, pyrazole, pyrimidine, pyrazine and pyridine. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl,

benzoisoxazolyl, benzoisothiazolyl, indolyl, indolizinyl, isoindolinyl, indazolyl, isobenzofuranyl, isoindolyl, isoquinolyl, quinazolinyl, quinolyl, quinoxalinyl, quinolizinyl, naphtyl, naphthyridinyl and phthalazinyl. A preferred aromatic ring according to the present invention is phenyl. A preferred heteroaromatic ring according to the present invention is pyridyl.

Heterocycle, heterocyclic compound or ring structure is a cyclic compound that has atoms of at least two different elements as members of its ring(s).

As used herein, the term "heterocyclic ring" is a saturated or partially saturated non aromatic ring system, of 3 to 10 members which contains one or more heteroatoms selected from N, O or S. Examples include, but are not limited to azetidinyl, piperazinyl, piperidinyl, morpholinyl, thiomorpholinyl, thiazolidinyl, pyrrolidinyl, azepanyl, diazepanyl, oxazepanyl, thiazepanyl, azocanyl and oxazocanyl.

A substituent on a saturated, partially saturated or unsaturated heterocycle can be attached at any substitutable position.

As used herein, the term "$C_{1-6}$ alkanediyl" as group or part of a group defines bivalent straight or branched chained saturated hydrocarbon radicals having from 1 to 4 carbon atoms. $C_{1-6}$ alkanediyl group, is preferably $C_{1-3}$ alkanediyl, more preferably $C_{1-2}$ alkanediyl. Examples include, but are not limited to methanediyl, ethanediyl, propanediyl, butanenediyl, pentanediyl and hexanediyl. Preferred are methanediyl, ethanediyl and propanediyl.

As used herein, the term "$C_{2-4}$ alkenediyl" as group or part of a group defines bivalent straight or branched chained saturated hydrocarbon radicals having from 2 to 4 carbon atoms and having at least one double bond, preferably one double bond, such as, but not limited to ethenediyl, propenediyl and butenediyl.

As used herein, the terms "$C_{3-7}$cycloalkyl", "$C_{3-10}$cylcloalkyl respectively" mean saturated cyclic hydrocarbon (cycloalkyl) with 3, 4, 5, 6 or 7 or with 3, 4, 5, 6, 7, 8, 9 or 10 carbon atoms and are respectively generic to cyclopropyl, cyclobutyl, cyclopentyl, cyclohexyl, cycloheptyl and to cyclopropyl, cyclobutyl, cyclopentyl, cyclohexyl, cycloheptyl, cyclooctyl, cyclononyl or cyclodecyl. Said saturated ring optionally contains one or more heteroatoms (also referred to as heterocyclyl or $C_{3-10}$heterocycloalkyl), such that at least one carbon atom is replaced by a heteroatom selected from N, O and S, in particular from N and O. Examples include, but are not limited to oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl or oxazocanyl. Preferred are saturated cyclic hydrocarbons with 3 or 4 carbon atoms and 1 oxygen or 1 nitrogen atom. Examples include oxetanyl, tetrahydrofuranyl, tetrahydro-2H-pyranyl, piperidinyl or pyrrolidinyl.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

As used herein, the term "halogen" includes fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

Included in the instant invention is the free base of compounds of formula (I) or (Ia), as well as the pharmaceutically acceptable salts and stereoisomers thereof. Some of the specific compounds exemplified herein are the protonated salts of amine compounds. Compounds of formula (I) or (Ia) containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of formula (I) or (Ia).The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reacting a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic,

glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Preferably, a pharmaceutically acceptable salt of this invention contains one equivalent of a compound of formula (I) or (Ia) and 1, 2 or 3 equivalent of an inorganic or organic acid. More particularly, pharmaceutically acceptable salts of this invention are the tartrate, trifluoroacetate or the chloride salts.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, $N,N^1$-dibenzylethylenediamine, diethylamin, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the present invention find use in a variety of applications for human and animal health. The compounds of the present invention are inhibitors of hepatitis B virus (HBV). The compounds of the present invention are inhibitors of hepatitis B virus (HBV) useful for the treatment and/or prevention of a HBV infection. In particular the compounds of the present invention are inhibitors of hepatitis B virus (HBV) core (HBc) protein useful for the treatment and/or prevention of a HBV infection.

The compounds, compositions and methods provided herein are particularly deemed useful for treating, ameliorating or preventing HBV infection and related conditions, including chronic hepatitis B, HBV/HDV co-infection, HBV/HCV co-infection, HBV/HIV co-infection.

In the present invention, the expression "HBV infection" comprises any and all conditions deriving from infection with HBV, including but not limited to hepatitis B, preferably chronic hepatitis B, HBV/HDV co-infection, HBV/HCV coinfection, HBV/HIV coinfection.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of

aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulstion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of formula (I) or (Ia) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of formula (I) or (Ia) are employed. (For purposes of this application, topical application shall include mouth washes and gargles.)

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of

polyethylene glycol.

When a compound according to this invention is administered into a human subject, the daily dosage will normally be determined by the prescribing physician with the dosage generally varying according to the age, weight, sex and response of the individual patient, as well as the severity of the patient's symptoms.

In one exemplary application, a suitable amount of compound is administered to a mammal undergoing anti HBV treatment. Administration generally occurs in an amount between about 0.01 mg/kg of body weight to about 60 mg/kg of body weight per day, preferably of between 0,5 mg/kg of body weight to about 40 mg/kg of body weight per day.

The instant compounds are also useful in combination with known therapeutic agents for simultaneous, separate or sequential administration.

In an embodiment, the compounds of the present invention may be used in combination with at least one or more additional therapeutic agents, in particular anti-HBV agents.

The term "anti-HBV agent", or more simply "HBV antiviral(s)" also includes compounds that are therapeutic nucleic acids, antibodies or proteins either in their natural form or chemically modified and/or stabilized. The term therapeutic nucleic acid includes but is not limited to nucleotides and nucleosides, oligonucleotides, polynucleotides, of which non limiting examples are antisense oligonucleotides, miRNA, siRNA, shRNA, therapeutic vectors and DNA/RNA editing components.

The term anti-HBV agent also includes compounds capable of treating HBV infection via immunomodulation, i.e. immunomodulators or immunomodulating compounds. Examples of immunomodulators are interferon-α (IFN-α), pegylated interferon-a or stimulants of the innate immune system such as Toll-like receptor 7 and/or 8 agonists and therapeutic or prophylactic vaccines. One embodiment of the present invention relates to combinations of a compound of formula (I) or (Ia) or any subgroup thereof, as specified herein, with an immunomodulating compound, more specifically a Toll-like receptor 7 and/or 8 agonist.

The additional HBV antiviral(s) can be selected for example, from therapeutic vaccines; RNA interference therapeutic/antisense oligonucleotides (e.g. siRNA, ddRNA, shRNA); immunomodulators (such as TLR agonists (e.g. TLR7, TLR8 or TLR9 agonists); STING agonists; RIG-I modulators; NKT modulators; IL agonists; Interleukin or other immune active proteins, therapeutic and prophylactic vaccines and immune checkpoint modulators; HBV entry inhibitors; cccDNA modulators (such as for example direct cccDNA inhibitors, inhibtors of cccDNA formation or maintenance, cccDNA epigenetic modifiers, inhibitors of cccDNA transcription); inhibitors of HBV protein espression; agents targeting HBV RNA; capsid assembly inhibitors/modulators; core or X protein targeting agents; nucleotide analogues; nucleoside analogues; interferons or modified interferons; HBV antivirals of distinct or unknown mechanism; cyclophilin inhibitors; sAg release inhibitors; HBV polymerase inhibitors; dinucleotides; SMAC inhibitors; HDV targeting agents; viral maturation inhibitors; reverse transcriptase inhibitors and HBV RNA destabilizers and other small-molecule inhibitors of HBV protein expression.

In particular, the combination of previously known anti-HBV agents, such as interferon-α (IFN-α), pegylated interferon-α, 3TC, tenofovir, lamivudine, entecavir, telbivudine, and adefovir or a combination thereof, and a compound of formula (I) or (Ia) or any subgroup thereof can be used as a medicine in a combination therapy. Additional examples of further therapeutic agents that may be combined with the compounds of the present invention include: Zidovudine, Didanosine, Zalcitabine, Stavudine, Abacavir, ddA, Emtricitabine, Apricitabine, Atevirapine, ribavirin, acyclovir, valacyclovir, famciclovir, ganciclovir, valganciclovir, cidofovir, Efavirenz, Nevirapine, Delavirdine and Etravirine.

Particular examples of such HBV antiviral(s) include, but are not limited to:

- RNA interference (RNAi) therapeutics: TKM-HBV (also known as ARB-1467), ARB-1740, ARC-520, ARC-521, BB-HB-331, REP-2139, ALN-HBV, ALN-PDL, LUNAR-HBV, GS3228836, and GS3389404;
- HBV entry inhibitors: Myrcludex B, IVIG-Tonrol, GC-1102;
- HBV capsid inhibitor/modulators, core or X protein targeting agents, direct cccDNA inhibitors, inhibitors of cccDNA formation or maintenance, or cccDNA epigenetic modifiers: BAY 41-4109, NVR 3-778, GLS-4, NZ-4 (also known as W28F), Y101, ARB-423, ARB-199, ARB-596, AB-506, JNJ-56136379, ASMB-101 (also known as AB-V102), ASMB-103, CHR-101, CC-31326, AT-130, RO7049389.
- HBV polymerase inhibitors: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir (in particular tenofovir disoproxil fumarate (Viread), tenofovir alafenamide fumarate (TAF)), tenofovir disoproxil orotate (also known as DA-2802), tenofovir disopropxil aspartate (also known as CKD-390), AGX-1009, and CMX157);
- HBV RNA destabilizers and other small-molecule inhibitors of HBV protein expression: RG7834, AB-452;
- cyclophilin inhibitors: OCB-030 (also known as NVP-018), SCY-635, SCY-575, and CPI-431-32;
- dinucleotides: SB9200;
- compounds of distinct or unknown mechanism, such as but not limited to AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), and similar analogs; REP-9AC (also known as REP-2055), REP-9AC' (also

known as REP-2139), REP-2165 and HBV-0259;

- TLR agonists (TLR7, 8 and/or 9): RG7795 (also known as RO-6864018), GS-9620, SM360320 (9-benzyl-8-hydroxy-2-(2-methoxy-ethoxy)adenine) and AZD 8848 (methyl[3-({[3-(6-amino-2-butoxy-8-oxo-7,8-dihydro-9H-pyrin-9-yl)propyl][3-(4-morpholinyl)propyl]amino}methyl)phenyl]acetate); ARB- 1598;
- RIG-I modulators: SB-9200;
- SMAC inhibitor: Birinapant;
- Immune Check Point inhibitors: BMS-936558 (Opdivo (nivolumab)), KEYTRUDA® (pembrolizumab);
- therapeutic vaccines: HBsAG-HBIG, HB-Vac, ABX203, NASVAC, GS-4774, GX- 110 (also known as HB-110E), CVI-HBV-002, RG7944 (also known as INO-1800), TG-1050, FP-02 (Hepsyn-B), AIC649, VGX-6200, KW-2, TomegaVax-HBV, ISA-204, NU-500, INX-102-00557 HBV MVA, PepTcell;
- IL agonists and immune acting proteins: INO-9112; recombinant IL12;
- interferons: interferon alpha (IFN-$\alpha$), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta- la, peginterferon beta-la, interferon delta, interferon lambda (IFN-$\lambda$), peginterferon lambda-1, interferon omega, interferon tau, interferon gamma (IFN-$\gamma$), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA, interferon alpha biobetter; in particular, peginterferon alpha-2a, peginterferon alpha-2b, glycosylated interferon alpha-2b, peginterferon beta-la, and peginterferon lambda-1; more in particular, peginterferon alpha-2a; - HDV targeting agent: Lonafamib.

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

In some embodiments, pulsed administration is more effective than continuous treatment because total pulsed doses are often lower than would be expected from continuous administration of the same composition. Each pulse dose can be reduced and the total amount of drug administered over the course of treatment is minimized. Individual pulses can be delivered to the patient continuously over a period of several hours, such as about 2, 4, 6, 8, 10, 12, 14 or 16 hours, or several days, such as 2, 3, 4, 5, 6 or 7 days.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. The present invention will be described by means of the following non-limiting examples and biological data are presented.

MATERIALS AND METHODS

***Chemistry***

*General*

Unless otherwise indicated, commercially available reagents and solvents (HPLC grade) were used without further purification.

Specifically, the following abbreviations may have been used in the descriptions of the experimental methods:

NMR: Nuclear Magnetic Resonance; $^1$H: proton; MHz: Megahertz; Hz: Hertz; HPLC: High Performance Liquid Chromatography; LC-MS: Liquid Chromatography Mass Chromatography Spectrum; s: second(s); min: minute(s); h: hour(s); mg: milligram(s); g: gram(s); Ml: microliter(s);

mL: millilitre(s); mmol: millimole(s); nm: nanometer(s) $\mu$M: micromolar; M: molarity or molar concentration; Rt: retention time in minutes; MW: microwave; Boc: *tert*-butyloxycarbonyl protecting group; DMF: dimethylformamide; DMSO: dimethylsulfoxide; MeOH: methanol; EtOH: ethanol; EtOAc: ethyl acetate; DCM: dichloromethane; PE: Petroleum Ether; TFA: trifluoroacetic acid; (g): gas; eq.: equivalent(s);; RT: room temperature; DIPEA: *N,N*-diisopropylethylamine; DIAD: Diisopropyl azodicarboxylate; sat.aq.: saturated aqueous solution; TEA: triethylamine;

THF: tetrahydrofuran; IPA: isopropylamine.; *p*TSA: para toluene sulfonic acid; TBDMS: *tert*-butyldimethylsilyl; LiHMDS: Lithium bis(trimehtylsilyl)amide; TBTU: 2-(1H-Benzotriazole-1-yl)-1,1,3,3-tetramethylaminium tetrafluoroborate;

Except where indicated otherwise, all temperatures are expressed in °C (degrees centigrade) or K (Kelvin).

The $^1$H-NMR spectra were acquired with an Avance II 300 MHz Bruker spectrometer. The chemical shifts are expressed in parts per million (ppm, $\delta$ units). The coupling constants are expressed in Hertz (Hz) and the splitting patterns are described as s (singlet), bs (broad signal), d (doublet), t (triplet), q (quartet), quint (quintet), m (multiplet).

The LC-MS analyses were performed by means of an UPLC Acquity Waters System equipped with the SQD spectrometer, single quadrupole mass detector, and a TUV detector, using column 1: ACQUITY UPLC BEH SHIELD, $RP_{18}$ (2.1x50mm, id=1.7 $\mu$m); column2: ACQUITY UPLC HSS T3, $RP_{18}$ (2.1x50mm, id=1.8 $\mu$m) and column3: ACQUITY UPLC BEH SHIELD, $RP_{18}$ (2.1x100mm, id=1.7 $\mu$m). Column temperature 40°C. Sample temperature 25°C. Phase A was composed by water (HiPerSolv Chromanorm Water VWR for HPLC-MS) + 0,05% Trifluoroacetic Acid; Phase B by $CH_3CN$ (HiPerSolv Chromanorm Acetonitrile SuperGradient VWR, suitable for UPLC/UHPLC instruments) + 0,05% Trifluoroacetic Acid; flow rate: 0,5 mL/min; UV detection (DIODE array) 200 nm; ESI+ and ESI- detection in the 100-1000 m/z range.

Method 1: column 1, run time: 3 minutes, run gradient: 5%B to 100%B in 2.80 min + 100%B for 0.2 min, equilibration time: 0,8 min, ionization mode: $ESI^+$.

Method 2: column 2, run time: 4 minutes, run gradient: 0%B to 45%B in 3.5 min + 45%B to 100%B in 0.05 min +100%B for 0.45 min, equilibration time: 0,8 min, ionization mode: $ESI^+$.

Method 3: column 3, run time: 6 minutes, run gradient: 5%B to 100%B in 5 min + 100%B for 1 min, equilibration time: 2 min.

Method 4: column 3, run time: 6 minutes, run gradient: 5%B to 50%B in 5 min + 50%B to 100%B in 0.2 min 100%B for 0.8 min, equilibration time: 2 min, ionization mode: $ESI^+$.

Method 5: column 1, run time: 3 minutes, run gradient: 5%B to 100%B in 2.80 min + 100%B for 0.2 min, equilibration time: 0,8 min, ionization mode: $ESI^+$.

Method 6: column 2, run time: 4 minutes. run gradient: 0%B to 45%B in 3.5 min + 45%B to 100%B in 0.05 min +100%B for 0.45 min. Equilibration time: 0,8 min, ionization mode: $ESI^+$.

Method 7: column 3, run time: 6 minutes, run gradient: 5%B to 100%B in 5 min + 100%B for 1 min, equilibration time: 2 min, ionization mode: $ESI^+$.

Method 8: column 3, run time: 6 minutes, run gradient: 5%B to 50%B in 5 min + 50%B to 100%B in 0.2 min 100%B for 0.8 min, Equilibration time: 2 min, ionization mode: $ESI^+$.

Method 9: column 1. run time: 4 minutes, column 1, run time: 4 minutes, run gradient:5%B to 100%B in 3.00 min + 100%B for 1 min, equilibration time: 0,8 min, ionization mode: $ESI^+$.

Method 10: column 1. run time: 4 minutes, run gradient: 5%B to 100%B in 3.00 min + 100%B for 1 min, equilibration time: 0,8 min, Ionization Mode: $ESI^-$.

Method 11: column 1, run time: 3 minutes, run gradient: 40%B to 100%B in 2.80 min + 100%B for 0.2 min, equilibration time: 0,8 min. Ionization Mode: $ESI^+$.

Method 12: column 3, run time: 6 minutes, run gradient: 25%B to 70%B in 5 min + 100%B for 1 min, equilibration time: 2 min, Flow: 0,5 mL/min, ionization mode: $ESI^+$.

*Synthesis*

According to a further aspect of the invention there is provided a process for the preparation of compounds of formula (I), (Ia) or salts thereof. The following schemes are examples of synthetic schemes that may be used to synthesise the compounds of the invention. In the following schemes reactive groups can be protected with protecting groups and deprotected according to well established techniques.

In the following schemes unless otherwise indicated A, B, Y, $R_1$, $R_2$, $R_3$, $R_4$, $R_5$, Ra, Rb, Rc, Rd, Re, Rf are as defined herein above in formula (I) or (Ia).

It will be understood by those skilled in the art that certain compounds of the invention can be converted into other compounds of the invention according to standard chemical methods. Unless otherwise indicated, compounds of the invention can be prepared following general procedures according to Scheme 1:

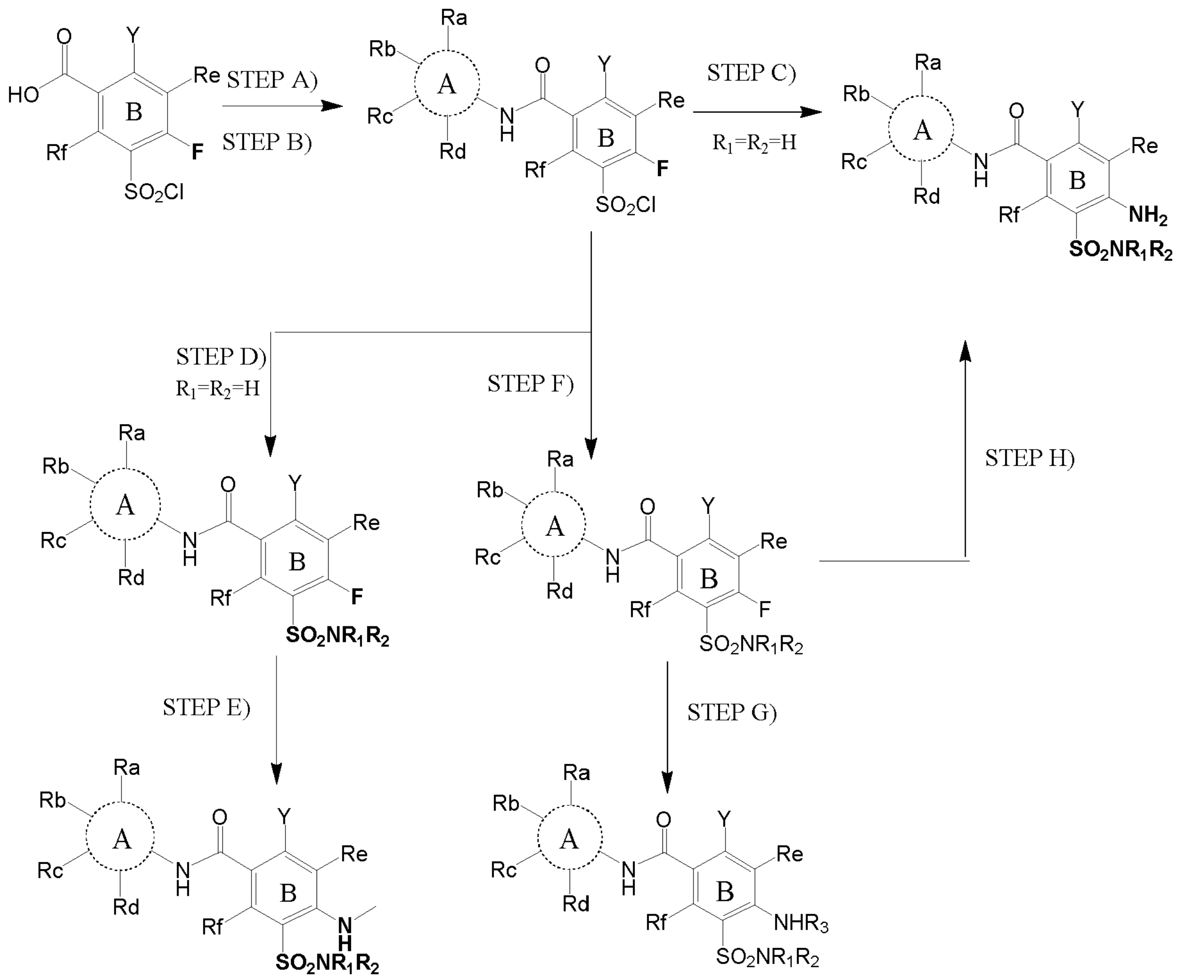


**Scheme 1**

STEP A) $SOCl_2$, reflux; STEP B) $ArNH_2$, toluene, reflux or $ArNH_2$, base, toluene, reflux; STEP C) $R_1$ = H; $NH_4OH$, 1,4-dioxane, 90-100°C; STEP D) $NH_4OH$, 1,4-dioxane, 0°C; STEP E) $NH_2CH_3$, base, DMSO, MeCN; STEP F) $NHR_1R_2$, MeCN, base, 0°C to room temperature; STEP G) $NH_2R_3$. 37% aq $H_2CO$; 10% HCl, 90°C; STEP H) $NH_4OH$, 90-100°C;

Example **E36** can be prepared according to Scheme 2:

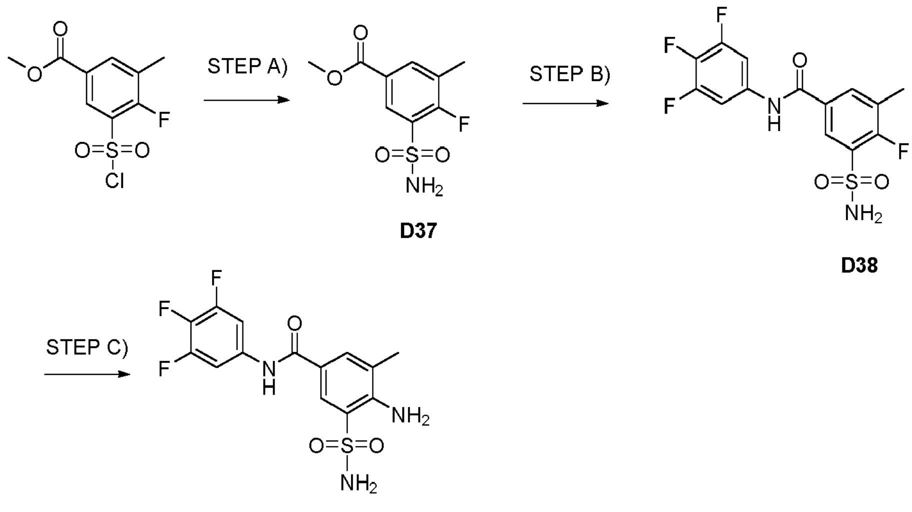


**D37**

**D38**

**E36**

**Scheme 2**

STEP A) $NH_4OH$, 1,4-dioxane, 0°C; STEP B) $ArNH_2$, (1M) LiHMDS in THF, room temperature; STEP C) $NH_4OH$, 90°C-120°C, 1,4-dioxane

Examples **E37** and **E58** can be prepared according to the following Scheme 3:

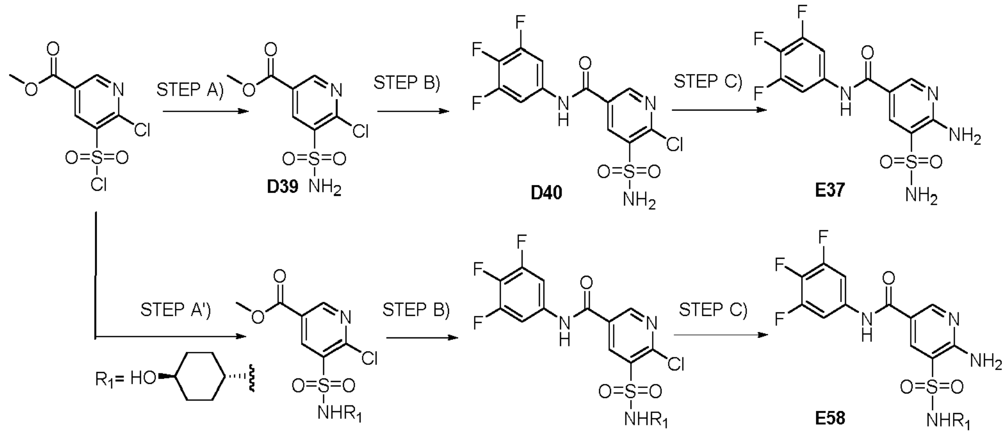


**Scheme 3**

STEP A) $NH_4OH$, 1,4-dioxane, room temperature or STEP A') $NH_2R_1$, MeCN, base, 0°C to room temperature; STEP B) $ArNH_2$ (1M) LiHMDS in THF, room temperature; STEP C) $NH_4OH$, 95°C, 1,4-dioxane

Examples **E59, E61** and **E62** can be prepared according to Scheme 4:

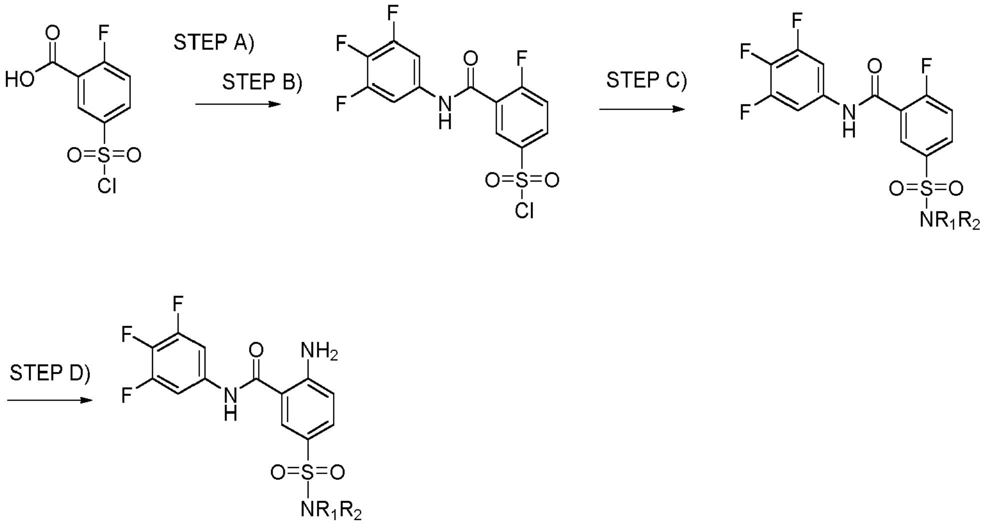


**Scheme 4**

STEP A) $SOCl_2$, reflux; STEP B) $ArNH_2$, toluene, reflux STEP C) $NHR_1R_2$, MeCN, base, 0°C to room temperature; STEP D) $NH_4OH$, 1,4-dioxane, 0°C.

The following Examples illustrate the preparation of certain compounds of Formula (I), (Ia) or salts thereof. The Descriptions 1 to 40 illustrate the preparation of intermediates used to make compounds of the invention or salts thereof. Examples 1 to 71 illustrate the present invention. In the procedures that follow, for the starting material reference is typically provided to a procedure in the Descriptions or in the Examples. This is provided merely for assistance to the skilled chemist. The starting material may not necessarily have been prepared from the batch of the Description or the Example referred to.

*Examples*

**Description 1: 3-(chlorosulfonyl)-4-fluorobenzoyl chloride (D1)**

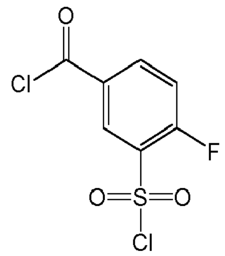

3-Chlorosulfonyl-4-fluoro-benzoic acid (Fluorochem, cat n° 037319) (14.2g, 59.51mmol) was added to thionyl chloride (80.39mL, 1106.9mmol) in a single portion. The resulting yellowish solution was heated to reflux for 4hrs, giving a slurry. Solvent was removed *in vacuo* by coevaporation with toluene, giving the title compound **D1** (15.4g, 59.91mmol) as a brown oil.

**Description 2: 2-chloro-5-(chlorosulfonyl)-4-fluorobenzoyl chloride (D2)**

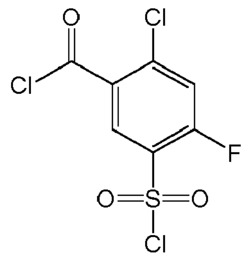

Similarly prepared according to procedure described for the preparation of compound **D1**, starting from 2-Chloro-5-chlorosulfonyl-4-fluoro-benzoic acid (Enamine, cat. n° EN300-01843).

**Description 3: 2-bromo-5-(chlorosulfonyl)-4-fluorobenzoyl chloride (D3)**

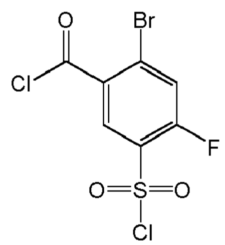

Similarly prepared according to procedure described for the preparation of **D1**, starting from 2-Bromo-5-(chlorosulfonyl)-4-fluorobenzoic acid (Enamine, cat. n° EN300-52736).

**Description 4: 5-(chlorosulfonyl)-2,4-difluorobenzoyl chloride (D4)**

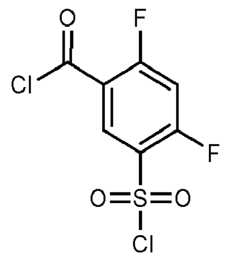

Similarly prepared according to procedure described for the preparation of **D1**, starting from 5-(Chlorosulfonyl)-2,4-difluorobenzoic acid (Enamine, cat. n° EN300-59276).

**Description 5: 3-(chlorosulfonyl)-4,5-difluorobenzoyl chloride (D5)**

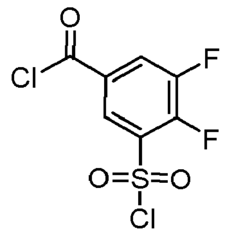

Similarly prepared according to the procedure described for the preparation of **D1**, starting from 3-(Chlorosulfonyl)-4,5-difluorobenzoic acid (Enamine, cat. n° EN300-107773).

**Description 6: 5-(chlorosulfonyl)-4-fluoro-2-methylbenzoyl chloride (D6)**

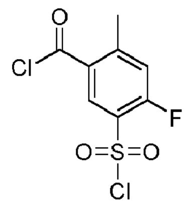

Similarly prepared according to procedure described for the preparation of **D1**, starting from 5-(chlorosulfonyl)-4-fluoro-2-methylbenzoic acid (Enamine, cat. n° EN300-114063).

**Description 7: 2-fluoro-5-((3,4,5-trifluorophenyl)carbamoyl)benzenesulfonyl chloride (D7)**

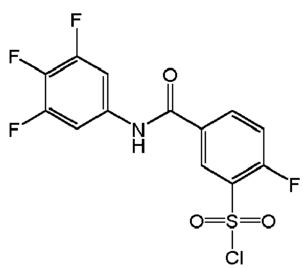

**D1** (15.4g, 59.91mmol) was dissolved in toluene (140mL), heated to reflux and then a solution of 3,4,5-trifluoroaniline (8.81g, 59.91mmol) in toluene (50 mL) was added dropwise over 10 min. A suspension was formed and refluxed for 1h. The reaction was cooled to room tempearture, filtered and the cake obtained washed with a small amount of toluene, giving the title compound **D7** (14.5g, 39.43mmol) as off-white solid. $^1$H NMR (300 MHz, DMSO-*d*$_6$) δ ppm 7.32 (t, J 9.03 Hz, 1 H) 7.68 - 7.81 (m, 2 H) 7.90 - 8.03 (m, 1 H) 8.30 (dd, J=6.83, 2.43 Hz, 1 H) 10.71 (s, 1 H). Method 1: Rt=2.43min, m/z=368.32 $(M+H)^+$.

**Description 8: 5-((3,4-difluorophenyl)carbamoyl)-2-fluorobenzenesulfonyl chloride (D8)**

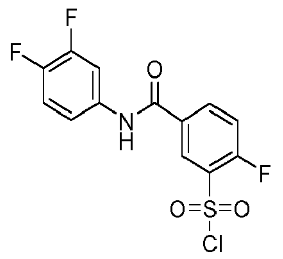

To a solution of **D1** (1.8 g, 7.0 mmol) in dry toluene (13.5 mL) at 90°C, triethylamine (1 mL, 7.175 mmol) and a solution of 3,4-difluoroaniline (0.904 g, 7.0 mmol) in dry toluene (3.5 mL) were added dropwise. Reaction mixture was stirred at reflux for 30 min, then was allowed to cool at room temperature and diluted with dichloromethane. Organic layer was washed with brine, dried over $Na_2SO_4$, filtered and evaporated under reduced pressure to give a beige solid (2.39 g) as crude product was suspended in DCM (5 mL) and sonicated. The resulting solid was filtered, washed with DCM and dried at vacuum pump to afford the title compound **D8** as white powder (1.73 g). $^1$H NMR (300 MHz, DMSO-*d*$_6$) δ ppm 7.26 - 7.48 (m, 3 H) 7.52 - 7.61 (m, 1 H) 7.91 - 7.99 (m, 2 H) 8.30 (dd, *J*=6.88, 2.38 Hz, 1 H) 10,59 (s, 1 H). Method 1: Rt=2.43min, m/z=350.02 $(M+H)^+$.

**Description 9: 5-[(3-chloro-4-fluorophenyl)carbamoyl]-2-fluorobenzene-1-sulfonyl chloride (D9)**

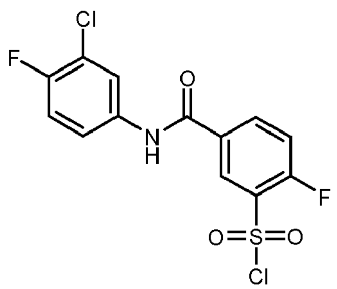

Similarly prepared according to procedure described for the preparation of **D8** starting from 3-(chlorosulfonyl)-4-fluorobenzoyl chloride **D1** and using 3-Chloro-4-fluoroaniline instead of 3,4-Difluoroaniline, to give the title compound **D9.** Method 1: Rt=2.43min, m/z=366.01 $(M+H)^+$.

**Description 10: 2-fluoro-5-((4-fluoro-3-(trifluoromethyl)phenyl)carbamoyl)benzenesulfonyl chloride (D10)**

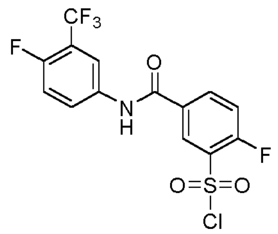

Similarly prepared according to procedure described for the preparation of **D7**, using as starting material **D1** (500mg, 1.94mmol) and 4-fluoro-3-(trifluoromethyl)aniline (0.25mL, 1.94mmol) instead of 3,4,5-trifluoroaniline. The reaction mixture was concentrated in vacuo to give to give the title compound **D10** (805mg) as beige solid, that was used in the next synthetic step directly.

**Description 11: 5-((3-cyano-4-fluorophenyl)carbamoyl)-2-fluorobenzenesulfonyl chloride (D11)**

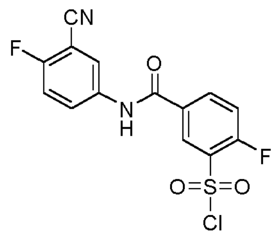

Similarly prepared according to procedure described for the preparation of **D7**, using as starting materials **D1** and 5-Amino-2-fluorobenzonitrile instead of 3,4,5-trifluoroaniline.

**Description 12: 5-((3-(difluoromethyl)-4-fluorophenyl)carbamoyl)-2-fluorobenzenesulfonyl chloride (D12)**

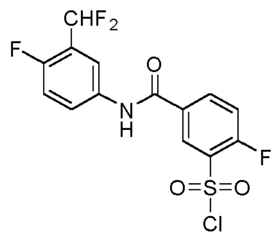

Similarly prepared according to procedure described for the preparation of **D7**, using as starting materials **D1** and 3-(Difluoromethyl)-4-fluoroaniline instead of 3,4,5-trifluoroaniline.

**Description 13: 5-((6-chloropyridin-3-yl)carbamoyl)-2-fluorobenzenesulfonyl chloride (D13)**

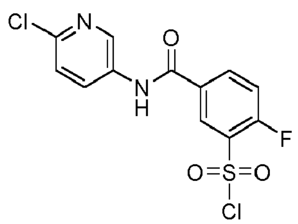

**D1** (307mg, 1.19mmol) was dissolved in Toluene (2.7mL) and heated to 90°C. A suspension of 6-chloropyridin-3-amine (153.5mg, 1.19mmol) in Toluene (1.2mL) was slowly added and the reaction mixture was refluxed at 110°C for 20min. DIPEA (0.31mL, 1.79mmol) was then added, and the reaction was refluxed at 110°C for another 1h50min. The mixture was concentrated in vacuo to give the crude title compound **D13** (702mg) as brown oil, that was used in the next synthetic step directly. Method 1; Rt=2.17min, m/z=349.00 $(M+H)^+$.

**Description 14: 2-fluoro-4-methyl-5-((3,4,5-trifluorophenyl)carbamoyl)benzenesulfonyl chloride (D14)**

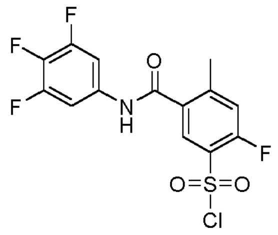

Similarly prepared according to procedure described for the preparation of **D7**, starting from 5-(chlorosulfonyl)-4-fluoro-2-methylbenzoyl chloride **D6** instead of **D1**. Method 1; Rt=2.49min, m/z=382.11 $(M+H)^+$.

**Description 15: 2-fluoro-5-((4-fluoro-3-methylphenyl)carbamoyl)benzenesulfonyl chloride (D15)**

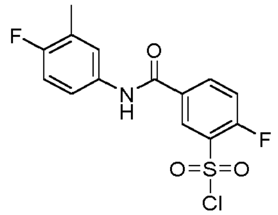

Similarly prepared according to procedure described for the preparation of **D7**, using as starting materials **D1** and 4-Fluoro-3-methylaniline instead of 3,4,5-trifluoroaniline. Method 1; Rt=2.38min, m/z=346.17 $(M+H)^+$.

**Description 16: 5-((3,5-difluoro-4-methylphenyl)carbamoyl)-2-fluorobenzenesulfonyl chloride (D16)**

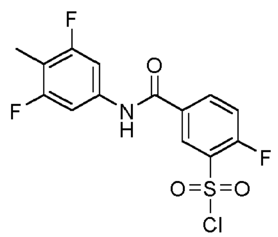

Similarly prepared according to procedure described for the preparation of **D7**, using as starting materials **D1** and 3,5-Difluoro-4-methylaniline instead of 3,4,5-trifluoroaniline. Method 1; Rt=2.54min, m/z=364.20 $(M+H)^+$.

**Description 17: 2-fluoro-5-((2,3,4-trifluorophenyl)carbamoyl)benzenesulfonyl chloride (D17)**

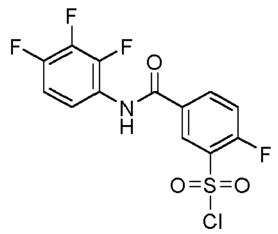

Similarly prepared according to procedure described for the preparation of **D7**, using as starting materials **D1** and 2,3,4-trifluoroaniline instead of 3,4,5-trifluoroaniline. Method 1; Rt=2.29min, m/z=368.19 $(M+H)^+$.

**Description 18: 2-fluoro-5-((2,4,5-trifluorophenyl)carbamoyl)benzenesulfonyl chloride (D18)**

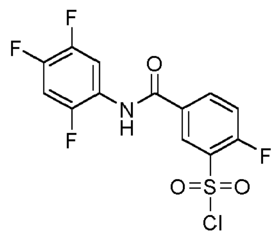

Similarly prepared according to procedure described for the preparation of **D7**, using as starting materials **D1** and 2,4,5-trifluoroaniline instead of 3,4,5-trifluoroaniline. Method 1; Rt=2.32min, m/z=368.39 $(M+H)^+$.

**Description 19: 2-fluoro-5-((2,4,5-trifluorophenyl)carbamoyl)benzenesulfonyl chloride (D19)**

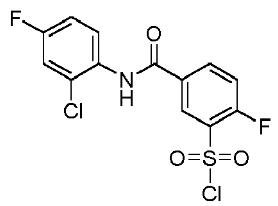

Similarly prepared according to procedure described for the preparation of **D7**, using as starting materials **D1** and 2-Chloro-4-fluoroaniline instead of 3,4,5-trifluoroaniline. Method 1; Rt=2.32min, m/z=366.03 $(M+H)^+$.

**Description 20: 4-fluoro-3-sulfamoyl-N-(3,4,5-trifluorophenyl)benzamide (D20)**

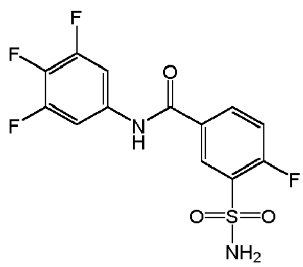

**D7** (3g, 8.16mmol) was dissolved in 1,4-Dioxane (20mL,0.23mol) and added in a single portion to ammonia

(10.42mL,163.18mmol) at 0°C. The reaction was stirred 15min then diluted with water and extracted with 2Me-THF. The organic layer was washed with 6N HCl and evaporated giving a brown solid (2.5g) that was triturated with DCM giving the title compound **D20** (2g, 5.74mmol) as off-white solid. Method 1; Rt: 1.85min. m/z: 349.21 $(M+H)^+$.

**Description 21: 2-chloro-4-fluoro-5-sulfamoyl-N-(3,4,5-trifluorophenyl)benzamide (D21)**

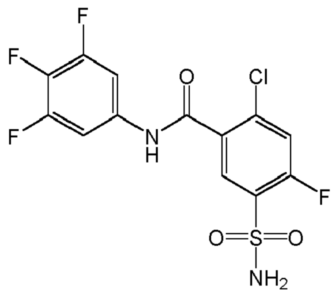

A 20mL vial was charged with 3,4,5-Trifluoroaniline (245.4mg, 1.67mmol) then a solution of **D2** (221.04mg, 0.76mmol) in Toluene (5mL) was added in a single portion. The vial was sealed and the reaction mixture stirred for 10min at room temperature, resulting in a white slurry. The reaction was diluted with toluene (3mL) and heated by microwave irradiation at 70°C for 20min. The reaction slurry was cooled to room temperature and poured into a flask containing acqu. $NH_4OH$ (aprox. 30mL) and stirred vigourously at room temperature overnight. The resulting white slurry, was diluted with DCM, treated with ice and acidified with 6N HCl until pH=1. The organic layer was diluted with EtOAc, washed with 6N HCl two times and brine, dried over $MgSO_4$ (dry), filtered and finally evaporated giving the title compound **D21** (250mg, 0.65mmol) as white solid. $^1H$ NMR (300 MHz, DMSO-$d_6$) $\delta$ ppm 7.13 - 7.30 (m, 1 H) 7.53 - 7.66 (m, 2 H) 7.89 - 8.02 (m, 3 H) 11.05 (s, 1 H). Method 9; Rt: 2.02min. m/z: 383.05 $(M+H)^+$.

**Description 22: 2-bromo-4-fluoro-5-sulfamoyl-*N*-(3,4,5-trifluorophenyl)benzamide (D22)**

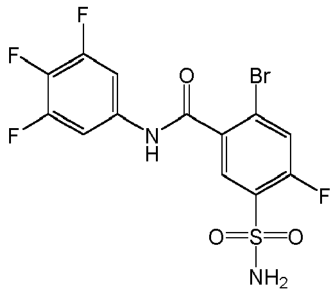

A 20mL vial was charged with **D3** (500mg, 1.49mmol), 3,4,5-Trifluoroaniline (437.85mg, 2.98mmol) and Toluene (4mL). The vial was sealed and stirred at room temperature giving after 10min a white suspension. The reaction was diluted with toluene (3mL) and the reaction mixture was heated by microwave irradiation at 100°C for 15min, diluted with toluene (5mL) and poured into 37% $NH_4OH$ (20mL). MeCN (2mL) was added and the resulting mixture stirred for 4hrs at room temperature.

The reaction mixture was diluted with EtOAc and extracted with EtOAc; the organic layer was dried over $MgSO_4$ (dry), filtered and evaporated giving the title compound **D22** (700mg, purity 75%). This intermediate was used in the next step without any purification. Method 1; Rt: 1.93min. m/z: 427.16 $(M+H)^+$.

**Description 23: N-(3-chloro-4-fluorophenyl)-4-fluoro-3-sulfamoylbenzamide (D23)**

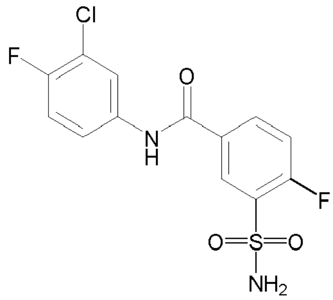

**D9** (200mg, 0,55mmol) was suspended in MeCN (2mL), cooled to 0°C with ice bath, then treated with a single portion of aqueous ammonia (2.18mL, 10.92mmol). The reaction was stirred 5min at 0°C giving a yellowish solution. Solvent was removed *in vacuo* and the residue was diluted with water and extracted with EtOAc. Solvent was removed *in vacuo* affording the title compound **D23** (200mg, 0,55mmol). Method 1; Rt: 1.88min. m/z: 346.98$(M+H)^+$.

**Description 24: 4-fluoro-3-(methylsulfamoyl)-N-(3,4,5-trifluorophenyl)benzamide (D24)**

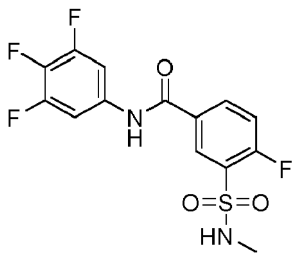

To a solution of **D7** (150 mg, 0.41 mmol) in MeCN (3.55 mL) cooled to 0°C, DIPEA (217.17 uL, 1.22 mmol) and methanamine (27.54 mg, 0.41 mmol) were added and the reaction mixture was stirred at room temperature for 1.5h. Water (5 mL) was added and the reaction mixture was stirred for 15 min. DCM (5 mL) was added and the stirring was continued for further 15 min. The solution was filtered on phase separator and the organic layer was concentrated under vacuo to give the crude title compound **D24** (151 mg) which was used without purification. Method 1; Rt=2.01min, m/z=362.79 $(M+H)^+$.

**Description 25: (R)-4-fluoro-N-(3,4,5-trifluorophenyl)-3-(N-(1,1,1-trifluoropropan-2-yl)sulfamoyl)benzamide (D25)**

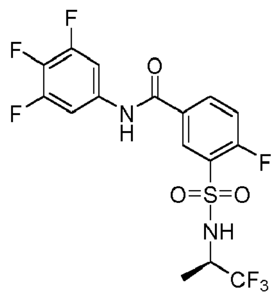

**D7** (100mg, 0.27mmol) was suspended in MeCN (2mL), DIPEA (0.24mL, 1.36mmol) and (2R)-1,1,1-trifluoro-2-propanamine hydrochloride (81.34mg, 0.54mmol) were added, and the resulting solution was stirred at RT for 3h. The reaction was diluted with EtOAc and washed twice with 3N HCl solution. Organic layer was dried over $Na_2SO_4$, filtered and concentrated under vacuo, to obtain the crude title compound **D25** (88mg) that was used in the next step without further purification. Method 1; Rt=2.32min, m/z=445.06 $(M+H)^+$.

**Description 26: (S)-4-fluoro-N-(3,4,5-trifluorophenyl)-3-(N-(1,1,1-trifluoropropan-2-yl)sulfamoyl)benzamide (D26)**

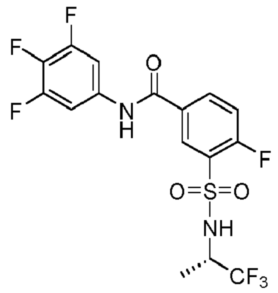

Similarly prepared according to procedure described for the preparation of compound **D25** using (2S)-1,1,1-Trifluoro-2-propanamine hydrochloride instead of (2R)-1,1,1-Trifluoro-2-propanamine hydrochloride. Method 1; Rt=2.32min, m/z=445.13 $(M+H)^+$.

**Description 27: 3-(N-cyclopropylsulfamoyl)-4-fluoro-N-(3,4,5-trifluorophenyl)benzamide (D27)**

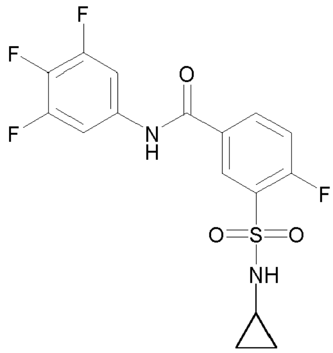

A suspension of cyclopropanamine hydrochloride (113.06uL, 1.63mmol) in MeCN (1mL) was treated at 0°C with DIPEA and then with **D7** (150mg, 0.41mmol). The resulting yellow solution was stirred at room temperature. Solvent was removed *in vacuo,* the residue was dissolved in 1/1 EtOAc/2-Methyl-THF and poured into a separating funnel. The organic layer was washed with 6N HCl, $NaHCO_3$ aq.sat. solution and brine, dried over $Na_2SO_4$, filtered and finally evaporated giving the title compound **D27** (125mg) as white solid. Method 1; Rt: 2.16min. m/z: 389.13$(M+H)^+$.

**Description 28: *trans*-4-fluoro-3-(N-(4-hydroxycyclohexyl)sulfamoyl)-N-(3,4,5-trifluorophenyl)benzamide (D28)**

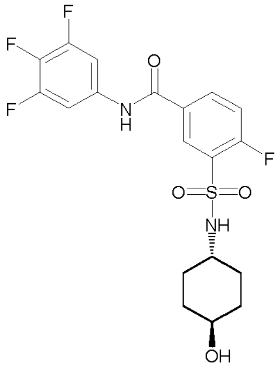

A suspension of *trans*-4-aminocyclohexan-1-ol hydrochloride (80mg, 0,53mmol) in MeCN (1mL) was treated at 0°C with triethylamine (197.92uL, 1.43mmol) and then with **D7** (150mg, 0.41mmol) The resulting solution become a white suspension and it was stirred at room temperature. Solvent was removed *in vacuo,* the residue was dissolved in 1/1 EtOAc/2-Methyl-THF and poured into a separating funnel. The organic layer was washed with 6N HCl, $NaHCO_3$ aq.sat.solution and brine, dried over $Na_2SO_4$ , filtered and finally evaporated giving the title compound **D28** (100mg) as white solid. Method 1; Rt: 1.90min. m/z: 447.16$(M+H)^+$.

**Description 29: *cis*-4-fluoro-3-(N-(4-hydroxycyclohexyl)sulfamoyl)-N-(3,4,5-trifluorophenyl)benzamide (D29)**

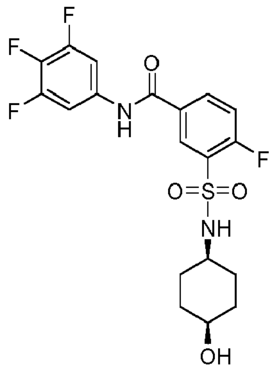

Similarly prepared according to procedure described for the preparation of **D25** using *cis*-4-Amino-cyclohexanol hydrochloride instead of (2R)-1,1,1-trifluoro-2-propanamine hydrochloride. Method 1; Rt=2.00min, m/z=447.22 $(M+H)^+$.

**Description 30: *trans*-4-fluoro-5-(N-((4-hydroxycyclohexyl)sulfamoyl)-2-methyl-N-(3,4,5-trifluorophenyl)benzamide (D30)**

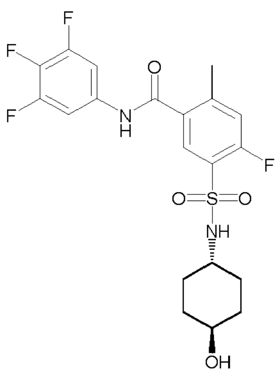

**D14** (100mg, 0.26mmol) was dissolved in MeCN (1.3mL) and cooled to 0°C. Triethylamine (0.13mL, 0.92mmol) and trans-4-Aminocyclohexanol hydrochloride (43.7mg, 0.29mmol) were added, and the reaction mixture was stirred at RT for 1h. EtOAc and few drops of MeOH were added, and the mixture was washed with 5% citric acid solution and sat. NaHCO3 solution. Organic layer was dried over $Na_2SO_4$, filtered and concentrated under vacuo, to obtain crude **D30** (122mg) as white solid. Method 1; Rt: 2.03min. m/z: 461.34 $(M+H)^+$.

**Description 31: tert-butyl 4-((2-fluoro-5-((3,4,5-trifluorophenyl)carbamoyl)phenyl)sulfonamido)piperidine-1-carboxylate (D31)**

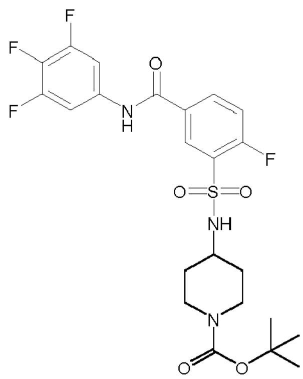

Tert-butyl 4-aminopiperidine-1-carboxylate (245.11mg, 1.22mmol) was suspended in MeCN (2mL), cooled to 0°C and treated with a single portion of **D7** (150mg, 0.41mmol). The resulting white suspension was stirred at room temperature for 1h. Solvent was removed *in vacuo,* the residue was dissolved in 1/1 EtOAc/2-Methyl-THF and poured into a separating funnel. The organic layer was washed with 6N HCl, $NaHCO_3$ sat. solution and brine, dried over $Na_2SO_4$, filtered and finally evaporated giving the title compound **D31** (140mg) as white solid. Method 1; Rt: 2.37min. m/z: 432.23$(M-Boc)^+$.

**Description 32: 4-fluoro-3-(N-(pyridin-4-yl)sulfamoyl)-N-(3,4,5-trifluorophenyl)benzamide (D32)**

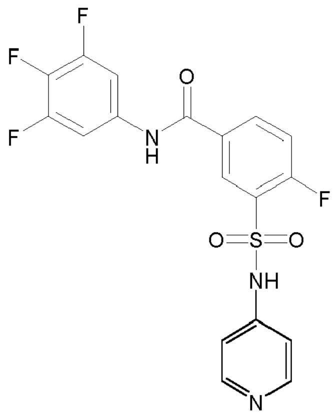

**D7** (50mg, 0.14mmol) was suspended in MeCN (1mL), treated with triethylamine (56.55uL, 0.41mmol) and pyridin-4-amine (40mg, 0.43mmol) resulting in a yellowish solution. The reaction was diluted with 2-Methyl-THF and EtOAc and acidified with 1N HCl to pH=1 (by paper), poured into a separating funnel and the acqueous layer separated. The aqueous layer was basified with $NaHCO_3$ and extracted with EtOAc; the combined organic extracts were evaporated, giving a residue containing the crude product (60mg). The crude product was suspended in DCM and filtered giving the title compound **D32** as yellowish solid. Method 1; Rt: 1.57min. m/z: 426.15 $(M+H)^+$.

**Description D33: 4-fluoro-3-(N-(oxetan-3-yl)sulfamoyl)-N-(3,4,5-trifluorophenyl)benzamide (D33)**

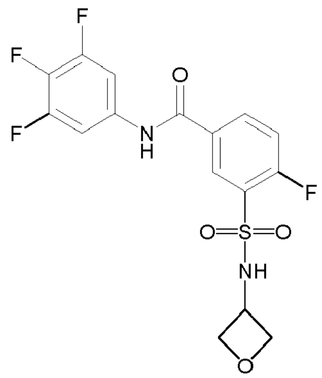

A solution of **D7** (300mg, 0.82mmol)in MeCN (2mL) was added to 3-oxetanamine (0.06mL, 0.82mmol) in a single portion at room temperature. The resulting white suspension was stirred at the same temperature for 1hr. Solvent was removed *in vacuo,* the residue dissolved in DCM, washed with 5% citric acid aqueous solution, dried over $Na_2SO_4$, filtered and finally evaporated giving the title compound **D33** (300mg,0.74mmol) as white solid. Method 1; Rt: 1.99min. m/z: 405.14$(M+H)^+$.

**Description 34: tert-butyl (S)-3-((2-fluoro-5-((3,4,5-trifluorophenyl)carbamoyl)phenyl)sulfonamido)pyrrolidine-1-carboxylate (D34)**

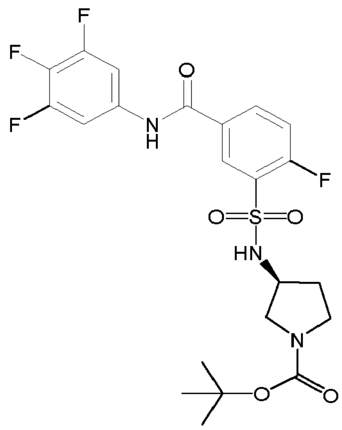

A solution of **D7** (200mg, 0,54mmol) in MeCN (2mL) was added to tert-butyl (S)-3-aminopyrrolidine-1-carboxylate (202.61mg, 1.09mmol in a single portion at room temperature. The resulting white suspension was stirred at room temperature for 1hr. Solvent was removed *in vacuo,* the residue dissolved in DCM, washed with 5% citric acid aqueous solution, dried over $Na_2SO_4$ , filtered and finally evaporated giving the title compound **D34** (280mg, 0.54mmol)as oil. Method 1; Rt: 2.35min. m/z: 518.22$(M+H)^+$.

**Description 35: 4-fluoro-3-(N-(3-hydroxycyclobutyl)sulfamoyl)-N-(3,4,5-trifluorophenyl)benzamide (D35)**

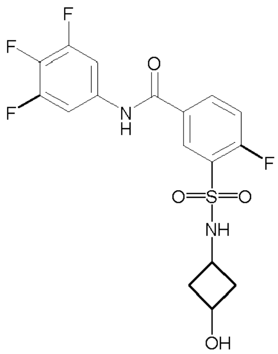

A solution of 3-aminocyclobutanol hydrochloride (134.44mg, 1.09mmol)in MeCN (2mL) was treated with a single portion of DIPEA (229.33uL, 1.63mmol) and then with **D7** (200mg, 0,54mmol). The resulting solution was stirred at room temperature for 1h. Solvent was removed *in vacuo,* the residue dissolved in DCM, washed with 5% citric acid aqueous solution, dried over $Na_2SO_4$ , filtered and finally evaporated giving the title compound **D35** (220mg, 0.526mmol) as oil. Method 1; Rt: 1.89min. m/z: 419.19$(M+H)^+$.

**Description 36: 4-fluoro-3-(N-((1R,3R)-3-hydroxycyclopentyl)sulfamoyl)-N-(3,4,5-trifluorophenyl)benzamide (D36)**

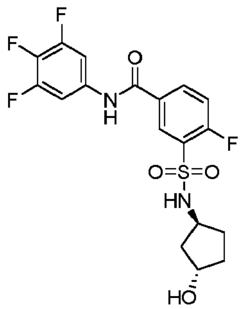

A suspension of (1S,3S)-3-Aminocyclopentan-1-ol hydrochloride 56.14mg,0.410mmol (cod. I-9981, Advanced ChemBlocks) in MeCN (1.5mL) was treated with N-ethyl-N-isopropylpropan-2-amine (198.95uL,1.14mmol) and stirred at room temperature for 15min. The reaction solution was cooled with ice bath and treated with a single portion of **D7** (100.mg,0.270mmol) and stirred for 5min at 0°C and at room temperature for 10min giving a yellow solution. Solvent was removed in vacuo, the residue was partitioned in DCM and citric acid (5% acq. solution). The organic layer was dried over $Na_2SO_4$ (anh.), filtered and evaporated, giving **D36** (105mg).

**Description 37: methyl 4-fluoro-3-methyl-5-sulfamoylbenzoate (D37)**

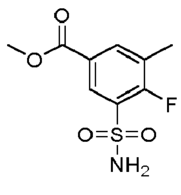

Similarly prepared according to procedure described for the preparation of the preparation of **D20** using as starting material methyl 3-(chlorosulfonyl)-4-fluoro-5-methylbenzoate (Enamine, cat.n° EN300-266824). Method 1; Rt=1.39min, m/z=248.14 $(M+H)^+$.

**Description 38: Synthesis of compound: 4-fluoro-3-methyl-5-sulfamoyl-*N*-(3,4,5-trifluorophenyl)benzamide (D38)**

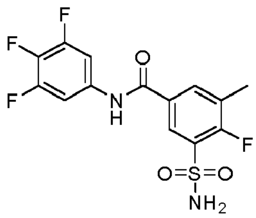

To a solution of methyl 4-fluoro-3-methyl-5-sulfamoylbenzoate **D37** (150mg, 0.610mmol) and 3,4,5-Trifluoroaniline (116mg 0.79mmol) in dry THF (3mL), lithium bis(trimethylsilyl)amide 1M in THF (3.52mL, 3.52mmol) was added dropwise. The reaction mixture was stirred at RT for 3h, then more 3,4,5-Trifluoroaniline (50mg, 0.340mmol) and lithium bis(trimethylsilyl)amide 1M in THF (1mL, 1mmol) were added, and the reaction mixture was stirred at RT overnight. The reaction was quenched with sat. $NH_4Cl$ solution, and EtOAc was added. Organic layer was washed with brine, then was dried over $Na_2SO_4$, filtered and concentrated under vacuo. The residue was triturated with PE, to obtain the title compound **D38** (218mg) as light-brown solid, that was used without further purification. Method 1; Rt=1.97min, m/z=363.12 $(M+H)^+$. $^1H$ NMR (300 MHz, DMSO-$d_6$) $\delta$ ppm 2.40 (d, *J*=2.11 Hz, 3 H) 7.63 - 8.01 (m, 4 H) 8.12 - 8.19 (m, 1 H) 8.19 - 8.25 (m, 1 H) 10.79 (brs, 1 H).

**Description 39: methyl 6-chloro-5-sulfamoylnicotinate (D39)**

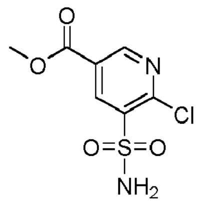

Methyl 6-chloro-5-(chlorosulfonyl)nicotinate (Enamine, EN300-41733; 525mg, 1.94mmol) was dissolved in 1,4-dioxane (2mL), 33% aqueous ammonia (2.29mL,19.44mmol) was added and the reaction mixture was stirred 20min at RT. The reaction was diluted with EtOAc and water, the phases were separated, the organic layer was dried over $Na_2SO_4$, filtered and concentrated under vacuo to obtain crude title compound **D39** (285mg), that was used without further purification. Method 1; Rt=1.20min, m/z=250.91 $(M+H)^+$.

**Description D40: 6-chloro-5-sulfamoyl-N-(3,4,5-trifluorophenyl)nicotinamide (D40)**

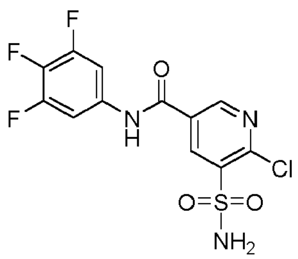

To a solution of crude compound **D39** (144mg, 0,570mmol) and 3,4,5-trifluoroaniline (109.9mg, 0.750mmol) in dry THF (2.5mL), lithium bis(trimethylsilyl)amide 1M in THF (3.33mL, 3.33mmol) was added dropwise. The reaction mixture was stirred at RT for 1h. The reaction was quenched with sat. $NH_4Cl$ solution, and EtOAc was added. The

organic layer was dried over $Na_2SO_4$, filtered and concentrated under vacuo. The resulting crude product was purified by preparative HPLC ($H_2O/CH_3CN$+1%TFA) to obtain, after lyophilization, the title compound **D40** (40mg) as light-yellow solid. Method 3; Rt: 3.17min, m/z: 366.10 $(M+H)^+$. $^1H$ NMR (300 MHz, DMSO-$d_6$) δ ppm 7.61 - 7.82 (m, 2 H) 8.01 (s, 2 H) 8.81 (d, *J*=2.29 Hz, 1 H) 9.13 (d, *J*=2.29 Hz, 1 H) 11.00 (s, 1 H).

**Example 1: 4-(methylamino)-3-sulfamoyl-*N*-(3,4,5-trifluorophenyl)benzamide (E1)**

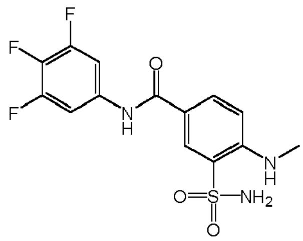

**D20** (58.mg, 0.170mmol) was dissolved in DMSO (1mL, 0.014mol) and treated with methanamine (77.59mg, 2.5mmol) and Triethylamine (346.27uL, 2.5mmol). The reaction solution was stirred overnight at room temperature. A second portion of methanamine (77.59mg, 2.5mmol) was added followed by triethylamine (346.27uL, 2.5mmol). After 5hrs at room temperature MeCN (200uL) were added and the reaction was stirred overnight at room temperature. The reaction was diluted with water and DCM, poured into a separating funnel and the organic layer washed twice with water and brine, dried over $MgSO_4$ (dry), filtered and finally evaporated yielding a yellowish solid (72mg). DCM (1mL) was added and the resulting white suspension was filtered yielding the title compound **E1** as white solid (33 mg). Method 3; Rt: 3.27min. MS(ES+) m/z: 360.15 $(M+H)^+$. $^1H$ NMR (300 MHz, DMSO-$d_6$) δ ppm 2.93 (d, *J*=4.86 Hz, 3 H) 6.30 - 6.47 (m, 1 H) 6.86 (d, *J*=8.89 Hz, 1 H) 7.43 (br s, 2 H) 7.64 - 7.86 (m, 2 H) 8.06(dd, *J*=8.76, 2.06 Hz, 1 H) 8.33 (d, J=2.20 Hz, 1 H) 10.41 (br s, 1 H).

**Example 2: 4-amino-3-(N-methylsulfamoyl)-N-(3,4,5-trifluorophenyl)benzamide (E2)**

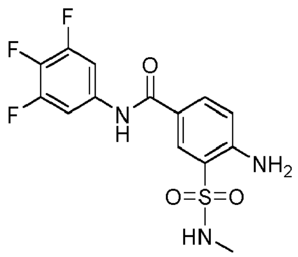

To a solution of crude **D24** (151 mg), in 1,4-dioxane (1.04 mL), aqueous ammonia (517 uL, 13.27 mmol) was added and the reaction mixture was stirred in a sealed vial at 90°C for 2h, and overnight at room temperature. The solvent was evaporated under reduced pressure. The crude product (40.06 mg) was purified by preparative HPLC ($H_2O/CH_3CN$+1%TFA) to afford, after lyophilization, the title compound **E2** (17.25 mg) as white powder. The remaining crude title compound was used without purification. Method 3; Rt: 3.23 min. m/z: 360.15 $(M+H)^+$. $^1H$ NMR (300 MHz, DMSO-$d_6$) δ ppm 2.44 (d, *J*=5.10 Hz, 3 H) 6.57 (br s, 2 H) 6.95 (d, *J*=8.70 Hz, 1 H) 7.50 - 7.59 (m, 1 H) 7.68 - 7.81 (m, 2 H) 0.00 (dd, *J*=9.80, 2.10 Hz, 1 H) 0.00 (d, *J*=2.10 Hz, 1 H) 10.36 (brs, 1 H).

**Example 3: 4-amino-3-sulfamoyl-*N*-(3,4,5-trifluorophenyl)benzamide (E3)**

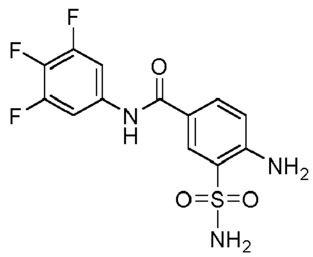

A pressure vessel was charged with **D7** (1.2g, 3.26mmol), 1,4-dioxane (6mL) and 33% aqueous ammonia (4mL, 34mmol). The pressure vessel was sealed and the reaction mixture was stirred 5min at RT, then heated at 95°C for 8h. The reaction was diluted with EtOAc and water, organic layer was dried over $Na_2SO_4$, filtered and concentrated under vacuo. The resulting crude product was purified by preparative HPLC ($H_2O/CH_3CN$+1%TFA) to obtain, after lyophilization, the title compound **E3** (840mg) as white solid. Method 3; Rt=3.01min, m/z=346.10 $(M+H)^+$. $^1H$ NMR (300 MHz, DMSO-$d_6$) δ ppm 6.49 (br s, 2 H) 6.88 (d, *J*=8.71 Hz, 1 H) 7.37 (s, 2 H) 7.62 - 7.81 (m, 2 H) 7.88 (dd, *J*=8.80, 2.20 Hz, 1 H) 8.26 (d, *J*=2.11 Hz, 1 H) 10.33 (s, 1 H).

**Example 4: 4-amino-N-(3,4-difluorophenyl)-3-sulfamoylbenzamide (E4)**

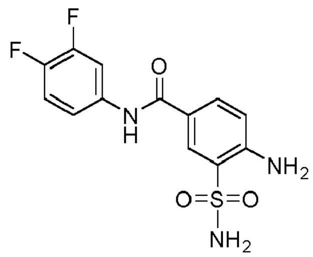

Similarly prepared according to the procedure described for the preparation of **E3,** starting from **D8.** Method 3; Rt=2.79min, m/z=328.20 $(M+H)^+$. $^1H$ NMR (300 MHz, DMSO-$d_6$) δ ppm 6.49 (br s, 2 H) 6.88 (d, *J*=8.71 Hz, 1 H) 7.37 (s, 2 H) 7.62 - 7.81 (m, 2 H) 7.88 (dd, *J*=8.80, 2.20 Hz, 1 H) 8.26 (d, *J*=2.11 Hz, 1 H) 10.33 (s, 1 H).

**Example 5: 4-amino-2-chloro-5-sulfamoyl-N-(3,4,5-trifluorophenyl)benzamide (E5)**

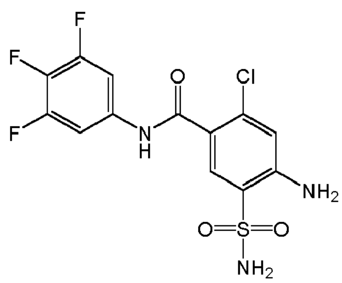

**D21** (250mg, 0.650mmol) was dissolved in 1,4-Dioxane (0,5mL), treated with ammonia (2.mL, 117.44mmol) and heated at 90°C in a closed vial for 12hrs. The yellowish reaction solution was poured, diluted with EtOAc (aprox. 20mL) and water. The organic layer was washed with brine and 0.2N HCl (2mL) then evaporated, giving a residue (220 mg). One amount (20mg) was purified by preparative HPLC ($H_2O/CH_3CN$+1%TFA) yielding the title compound **E5** (11mg) as white powder. Method 3; Rt: 3.21min. m/z: 380.15 $(M+H)^+$. $^1H$ NMR (300 MHz, DMSO-$d_6$) δ ppm 6.46 (br s, 2 H) 6.96 (s, 1 H) 7.46 (br s, 2 H) 7.53 - 7.70 (m, 2 H) 7.76 (br s, 1 H) 10.69 (br s, 1 H)

**Example 6: 4-amino-2-bromo-5-sulfamoyl-N-(3,4,5-trifluorophenyl)benzamide (E6)**

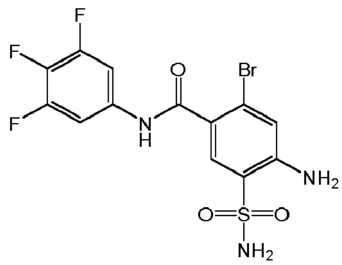

Similary prepared according to procedure described for **E5,** starting from **D22** 2-bromo-4-fluoro-5-sulfamoyl-N-(3,4,5-trifluorophenyl)benzamide and purified by preparative HPLC ($H_2O$,$CH_3CN$, 0.1% HCOOH). Method 3; Rt: 3.24min. m/z: 424.12 $(M+H)^+$. $^1H$ NMR (300 MHz, DMSO-$d_6$) δ ppm 6.42 (s, 2 H) 7.14 (s, 1 H) 7.47 (br s, 2 H) 7.60 (dd, *J*=10.22*,* 6.56 Hz, 2 H) 7.71 (s, 1 H) 10.73 (br s, 1 H).

**Example 7: 4-amino-N-(4-fluoro-3-(trifluoromethyl)phenyl)-3-sulfamoylbenzamide (E7)**

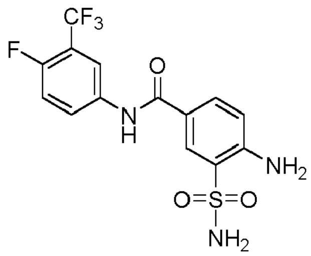

Similarly prepared according to the procedure described for the preparation of compound **E3,** starting from **D10.** Method 3; Rt=3.16min, m/z=378.12 $(M+H)^+$. $^1H$ NMR (300 MHz, DMSO-d6) δ ppm 6.47 (s, 2 H) 6.88 (d, J=8.62 Hz, 1 H) 7.36 (s, 2 H) 7.50 (t, J=9.90 Hz, 1 H) 7.91 (dd, J=8.67, 2.15 Hz, 1 H) 8.05 - 8.13 (m, 1 H) 8.22 (dd, J=6.69, 2.48 Hz, 1 H) 8.29 (d, J=2.11 Hz, 1 H) 10.34 (s, 1 H).

**Example 8: 4-amino-N-(3-cyano-4-fluorophenyl)-3-sulfamoylbenzamide (E8)**

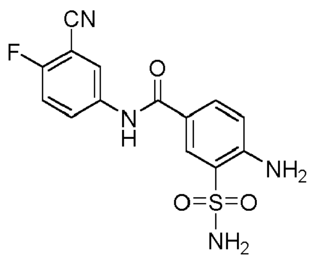

Similarly prepared according to procedure described for the preparation of **E3**, starting from **D11**. Method 3; Rt=2.65min, m/z=334.95, $(M+H)^+$. 1H NMR (300 MHz, DMSO-$d_6$) δ ppm 6.44 (br s, 2 H) 6.88 (d, *J*=8.71 Hz, 1 H) 7.37 (s, 2 H) 7.53 (t, *J*=9.17 Hz, 1 H) 7.90 (dd, *J*=8.62, 2.11 Hz, 1 H) 7.99 - 8.13 (m, 1 H) 8.25 (dd, *J*=5.78, 2.66 Hz, 1 H) 8.28 (d, *J*=2.11 Hz, 1 H) 10.36 (s, 1 H).

**Example 9: 4-amino-N-(3-(difluoromethyl)-4-fluorophenyl)-3-sulfamoylbenzamide (E9)**

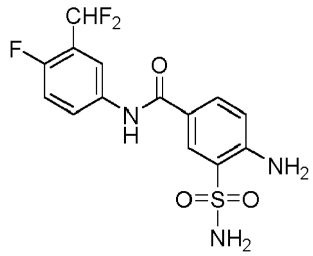

Similarly prepared according to procedure described for the preparation of **E3** starting from **D12** LF_042_097. Method 3; Rt=2.85, m/z=360.08 $(M+H)^+$. 1H NMR (300 MHz, DMSO-d6) δ ppm 6.45 (br s, 2 H) 6.87 (d, J=8.62 Hz, 1 H) 7.01 - 7.45 (m, 4 H) 7.86 - 7.98 (m, 2 H) 8.03 - 8.12 (m, 1 H) 8.28 (d, J=2.11 Hz, 1 H) 10.25 (s, 1 H).

**Example 10: 4-amino-N-(3-chloro-4-fluorophenyl)-3-sulfamoylbenzamide (E10)**

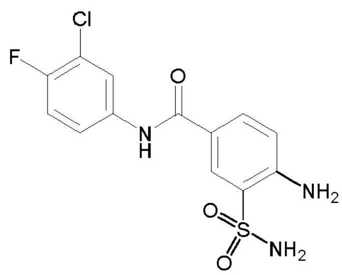

A mixture **of D23** (200.mg, 0,580mmol), 1,4-dioxane (2mL) and aqueous ammonia (2.3mL, 10.38mmol) were heated in a closed vial at 100°C for 10hrs. Solvent was removed, the residue suspended in DCM and filtered giving a off-white solid (110mg). One half of this crude product was purified by preparative HPLC ($H_2O/CH_3CN$+1%TFA) giving the title compound **E10** (8.4mg) as off-white solid. Method 3; Rt: 2.98min. m/z: 344.07 $(M+H)^+$. $^1H$ NMR (300 MHz, DMSO-$d_6$) δ ppm 6.45 (br s, 2 H) 6.87 (d, *J*=8.71 Hz, 1 H) 7.31 - 7.43 (m, 3 H) 7.64 - 7.77 (m, 1 H) 7.89 (dd, *J*=8.67, 2.16 Hz, 1 H) 8.05 (dd, *J*=6.92, 2.52 Hz, 1 H) 8.26 (d, *J*=2.11 Hz, 1 H) 10.20 (s, 1 H).

**Example 11: 4-amino-N-(6-chloropyridin-3-yl)-3-sulfamoylbenzamide (E11)**

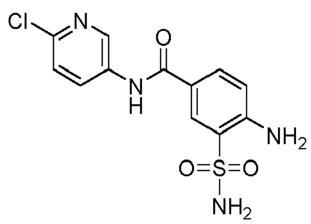

Similarly prepared according to procedure described for the preparation of **E3** starting from **D13.** Method 3; Rt=2.33min, m/z=327.05 $(M+H)^+$. 1H NMR (300 MHz, DMSO-d6) δ ppm 6.49 (br s, 2 H) 6.88 (d, J=8.71 Hz, 1 H) 7.37 (s, 2 H) 7.50 (d, J=8.71 Hz, 1 H) 7.91 (dd, J=8.62, 2.02 Hz, 1 H) 8.23 (dd, J=8.71, 2.75 Hz, 1 H) 8.29 (d, J=2.02 Hz, 1 H) 8.77 (d, J=2.57 Hz, 1 H) 10.35 (s, 1 H).

**Example 12: 4-amino-N-(4-fluoro-3-methylphenyl)-3-sulfamoylbenzamide (E12)**

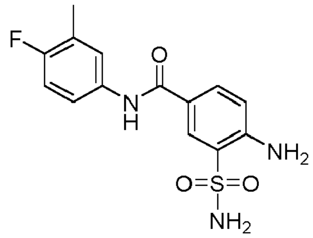

Similarly prepared according to procedure described for the preparation of **E3**, starting from **D15.** Method 3; Rt=2.83, m/z=324.14 $(M+H)^+$. $^1H$ NMR (300 MHz, DMSO-$d_6$) $\delta$ ppm 2.23 (br d, *J*=1.60 Hz, 3 H) 6.40 (s, 2 H) 6.86 (d, *J*=8.62 Hz, 1 H) 7.10 (t, *J*=9.26 Hz, 1 H) 7.34 (s, 2 H) 7.50 - 7.60 (m, 1 H) 7.60 - 7.71 (m, 1 H) 7.89 (dd, *J*=8.62, 2.11 Hz, 1 H) 8.25 (d, *J*=2.11 Hz, 1 H) 9.99 (s, 1 H).

**Example 13: 4-amino-N-(3,5-difluoro-4-methylphenyl)-3-sulfamoylbenzamide (E13)**

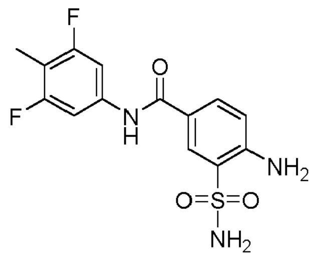

Similarly prepared according to procedure described for the preparation of **E3**, starting from **D16** LF_042_134. Method 3; Rt=3.17, m/z=342.25 $(M+H)^+$. $^1H$ NMR (300 MHz, DMSO-$d_6$) $\delta$ ppm 2.11 (s, 3 H) 6.47 (s, 2 H) 6.87 (d, *J*=8.71 Hz, 1 H) 7.36 (s, 2 H) 7.44 - 7.57 (m, 2 H) 7.88 (dd, *J*=8.67, 2.16 Hz, 1 H) 8.25 (d, *J*=2.11 Hz, 1 H) 10.25 (s, 1 H).

**Example 14: 4-amino-3-sulfamoyl-N-(2,3,4-trifluorophenyl)benzamide (E14)**

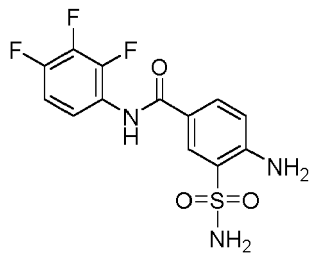

Similarly prepared according to procedure described for the preparation of **E3**, starting from **D17.** Method 3; Rt=2.65, m/z=346.17 $(M+H)^+$. $^1H$ NMR (300 MHz, DMSO-$d_6$) $\delta$ ppm 6.47 (s, 2 H) 6.87 (d, *J*=8.71 Hz, 1 H) 7.22 - 7.47 (m, 4 H) 7.88 (dd, *J*=8.71, 2.20 Hz, 1 H) 8.27 (d, *J*=2.11 Hz, 1 H) 10.11 (br s, 1 H).

**Example 15: 4-amino-3-sulfamoyl-N-(2,4,5-trifluorophenyl)benzamide (E15)**

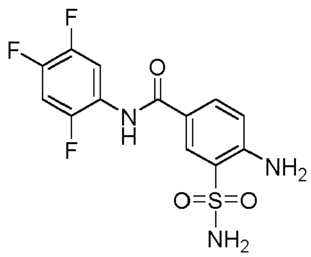

Similarly prepared according to procedure described for the preparation of **E3**, starting from **D18.** Method 3; Rt=2.68, m/z=346.17 $(M+H)^+$. $^1H$ NMR (300 MHz, DMSO-$d_6$) δ ppm 6.47 (s, 2 H) 6.87 (d, *J*=8.71 Hz, 1 H) 7.22 - 7.47 (m, 4 H) 7.88 (dd, *J*=8.71, 2.20 Hz, 1 H) 8.27 (d, *J*=2.11 Hz, 1 H) 10.11 (br s, 1 H)

**Example 16: 4-amino-N-(2-chloro-4-fluorophenyl)-3-sulfamoylbenzamide (E16)**

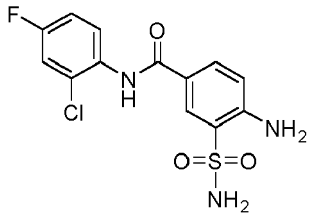

Similarly prepared according to procedure described for the preparation of **E3**, starting from **D19.** $^1H$ NMR (300 MHz, DMSO-$d_6$) δ ppm 6.44 (br s, 2 H) 6.87 (d, *J*=8.62 Hz, 1 H) 7.22 - 7.31 (m, 1 H) 7.35 (s, 2 H) 7.47 - 7.62 (m, 2 H) 7.89 (dd, *J*=8.80, 2.20 Hz, 1 H) 8.27 (d, *J*=2.20 Hz, 1 H) 9.85 (s, 1 H)

**Example 17: 4-amino-2-methyl-5-sulfamoyl-N-(3,4,5-trifluorophenyl)benzamide (E17)**

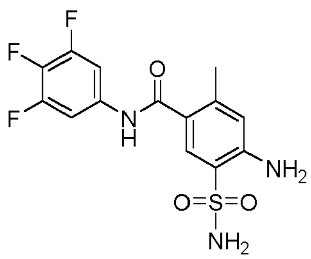

**D14** (150mg, 0.390mmol) was suspended in 1,4-Dioxane (1mL) Aqueous ammonia (0.96mL, 24.6mmol) was added and the reaction mixture was stirred at 100°C for 8h and overnight at RT. More Aqueous ammonia (0.3mL, 7.69mmol) was added and the reaction mixture was stirred at 100°C for another 8h. The reaction was diluted with EtOAc and water, organic layer was dried over $Na_2SO_4$, filtered and concentrated under vacuo. The resulting crude were purified by preparative HPLC ($H_{2O}/CH_3CN$+1% TFA) to obtain, after lyophilization, the title compound **E17** (110mg) as white solid. Method 3; Rt=3.16min, m/z=360.22 $(M+H)^+$. $^1H$ NMR (300 MHz, DMSO-$d_6$) δ ppm 2.32 (s, 3 H) 6.22 (br s, 2 H) 6.68 (s, 1 H) 7.26 (s, 2 H) 7.56 - 7.70 (m, 2 H) 7.74 (s, 1 H) 10.49 (s, 1 H).

**Example 18: (R)-4-amino-N-(3,4,5-trifluorophenyl)-3-(N-(1,1,1-trifluoropropan-2-yl)sulfamoyl)benzamide (E18)**

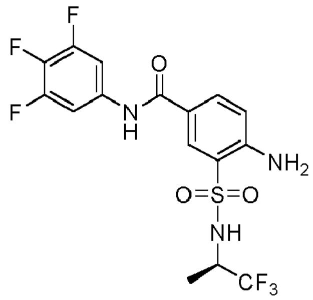

Similarly prepared according to procedure described for the preparation of **E3,** starting from **D25**. Method 3; Rt: 3.75min, m/z: 442.16 $(M+H)^+$. $^1H$ NMR (300 MHz, DMSO-$d_6$) δ ppm 1.06 (d, *J*=6.88 Hz, 3 H) 3.95 - 4.03 (m, 1 H) 6.56 (br s, 2 H) 6.92 (d, *J*=8.71 Hz, 1 H) 7.58 - 7.80 (m, 2 H) 7.91 (dd, *J*=8.71, 2.20 Hz, 1 H) 8.25 (d, *J*=2.11 Hz, 1 H) 8.55 (d, *J*=9.17 Hz, 1 H) 10.35 (s, 1 H).

**Example 19: (S)-4-amino-N-(3,4,5-trifluorophenyl)-3-(N-(1,1,1-trifluoropropan-2-yl)sulfamoyl)benzamide (E19)**

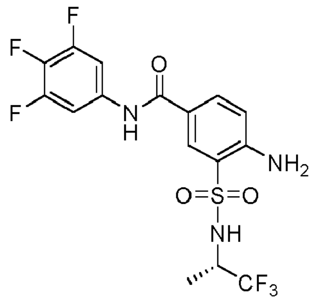

Similarly prepared according to procedure described for the preparation of **E3**, starting from **D26**. Method 3; Rt: 3.75min, m/z: 442.16 $(M+H)^+$. $^1H$ NMR (300 MHz, DMSO-$d_6$) δ ppm 1.06 (d, *J*=6.88 Hz, 3 H) 3.95 - 4.00 (m, 1 H) 6.57 (br s, 2 H) 6.92 (d, *J*=8.80 Hz, 1 H) 7.63 - 7.78 (m, 2 H) 7.91 (dd, *J*=8.80, 2.20 Hz, 1 H) 8.25 (d, *J*=2.20 Hz, 1 H) 8.55 (d, *J*=9.17 Hz, 1 H) 10.35 (s, 1 H).

**Example 20: 4-amino-3-(N-cyclopropylsulfamoyl)-N-(3,4,5-trifluorophenyl)benzamide (E20)**

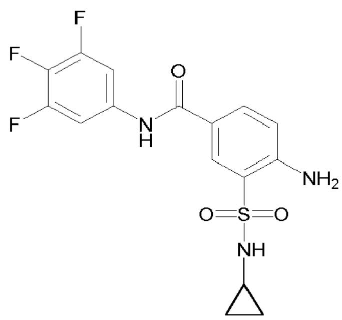

A solution of **D27** (125mg, 0.32mmol) in aqueous ammonia (0.38mL, 3.22mmol)and 1,4-dioxane (1.1mL, 0.013mol) was heated at 100°C for 8hrs in a closed vial. Solvent was removed *in vacuo,* the residue was treated with toluene and further evaporated by high vacuum pump, giving a residue (107mg, off-white solid). A sample of this crude material (24.5mg) was purified by preparative HPLC ($H_2O/CH_3CN$+1%TFA) to afford, after lyophilization the title compound **E20** (13.2mg) as white solid. Method 3; Rt: 3.55min. m/z: 386.23 $(M+H)^+$. $^1H$ NMR (300 MHz, DMSO-$d_6$) δ ppm 0.35 - 0,50 (m, 4 H) 2.11 (td, *J*=6.51, 3.12 Hz, 1 H) 6.54 (br s, 2 H) 6.91 (d, *J*=8.71 Hz, 1 H) 7.64 - 7.80 (m, 2 H) 7.88

(dd, *J*=8.70, 2.00 Hz, 1 H) 7.95 (dd, *J*=2.50 Hz, 1 H) 8.25 (d, *J*=2.11 Hz, 1 H) 10.35 (br s, 1 H).

**Example 21: frans-4-amino-3-(N-(4-hydroxycyclohexyl)sulfamoyl)-N-(3,4,5-trifluorophenyl)benzamide (E21)**

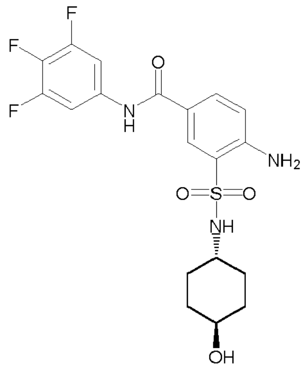

A solution of **D28** (100mg, 0.22mmol) in aqueous ammonia (801.08uL, 2.24mmol)and 1,4-dioxane (1mL) was heated at 100°C for 8hrs in a closed vial. Solvent was removed *in vacuo,* the residue was treated with toluene and further evaporated. Solvent traces were removed by high vacuum pump, giving a residue as off-white solid (108mg). A sample of this crude material (20mg) was purified by preparative HPLC ($H_2O/CH_3CN$+1%TFA), affording the title compound **E21** (9mg) as white solid. Method 3; Rt: 3.11. m/z: 444.25 $(M+H)^+$. $^1H$ NMR (300 MHz, DMSO-$d_6$) $\delta$ ppm 0.87 - 1.35 (m, 4 H) 1.50 - 1.86 (m, 4 H) 2.78 - 2.97 (m, 1 H) 3.20 - 3.34 (m, 2 H) 6.51 (br s, 2 H) 6.88 (d, *J*=8.71 Hz, 1 H) 7.54 - 7.82 (m, 3 H) 7.89 (dd, *J*=8.71, 2.11 Hz, 1 H) 8.23 (d, *J*=2.11 Hz, 1 H) 10.34 (s, 1 H). $^1H$ NMR (300 MHz, DMSO-$d_6$+TFA) $\delta$ ppm 0.87 - 1.37 (m, 4 H) 1.45 - 1.86 (m, 4 H) 2.79 - 3.01 (m, 1 H) 3.14 - 3.43 (m, 1 H) 6.88 (d, *J*=8.71 Hz, 1 H) 7.55 - 7.82 (m, 3 H) 0.00 (dd, *J*=8.50, 2.20 Hz, 1 H) 8.23 (d, *J*=2.11 Hz, 1 H) 10.33 (s, 1 H).

**Example 22: *cis*-4-amino-3-(N-(4-hydroxycyclohexyl)sulfamoyl)-N-(3,4,5-trifluorophenyl)benzamide (E22)**

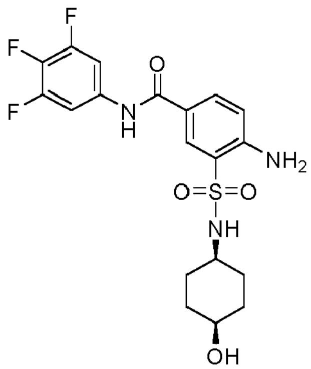

Similarly prepared according to the procedure described for the preparation of compound **E3,** starting **from D29.** Method 3; Rt=3.24min, m/z=444.25 $(M+H)^+$. $^1H$ NMR (300 MHz, DMSO-$d_6$) $\delta$ ppm 1.35 (m, *J*=3.30 Hz, 4 H) 1.43 - 1.62 (m, 4 H) 2.82 - 3.08 (m, 1 H) 3.49 - 3.58 (m, 2 H) 6.52 (br s, 2 H) 6.87 (d, *J*=8.62 Hz, 1 H) 7.53 - 7.79 (m, 3 H) 7.88 (dd, *J*=8.71, 2.29 Hz, 1 H) 8.23 (d, *J*=2.20 Hz, 1 H) 10.33 (s, 1 H).

**Example 23: *trans*-4-amino-5-(N-(4-hydroxycyclohexyl)sulfamoyl)-2-methyl-N-(3,4,5-trifluorophenyl)benzamide (E23)**

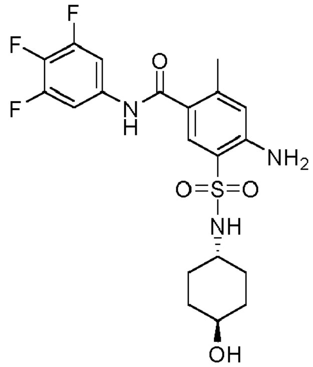

Similarly prepared according to the procedure described for the preparation of compound **E3,** starting from **D30** LF_042_146. Method 3; Rt=2.03min, m/z=461.34 $(M+H)^+$. $^1H$ NMR (300 MHz, DMSO-$d_6$) δ ppm 0.94 - 1.30 (m, 4 H) 1.54 - 1.83 (m, 4 H) 2.34 (s, 3 H) 2.77 - 2.96 (m, 1 H) 3.20 - 3.41 (m, 1 H) 6.24 (br s, 2 H) 6.68 (s, 1 H) 7.54 (d, *J*=7.52 Hz, 1 H) 7.58 - 7.70 (m, 2 H) 7.73 (s, 1 H) 10.46 (s, 1 H).

**Example 24: *cis*-4-amino-3-(N-3-hydroxycyclobutyl)sulfamoyl)-N-(3,4,5-trifluorophenyl)benzamide (E24)**

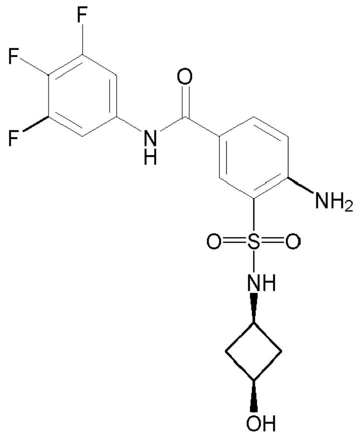

Cis-4-fluoro-3-(N-(3-hydroxycyclobutyl)sulfamoyl)-N-(3,4,5-trifluorophenyl)benzamide was prepared according to the procedure described for the synthesis of **D35**, using *cis-3*-aminocyclobutanol instead of 3-aminocyclobutanol. The intermediate compound was further reacted with aqueous ammonia in 1,4-dioxane according to the procedure described for the preparation of **E10**. Method 3; Rt: 3.12. m/z: 416.29 $(M+H)^+$. $^1H$ NMR (300 MHz, DMSO-$d_6$) δ ppm 1.43 - 1.74 (m, 2 H) 2.11 - 2.26 (m, 2 H) 2.94 - 3.17 (m, 1 H) 3.59 - 3.71 (m, 1 H) 6.55 (br s, 2 H) 6.88 (d, *J*=8.71 Hz, 1 H) 7.72 (dd, *J*=10.64, 6.60 Hz, 2 H) 7.86 - 7.95 (m, 2 H) 8.16 - 8.24 (m, 1 H) 10.33 (s, 1 H).

**Example 25: *trans*-4-amino-3-(N-3-hydroxycyclobutyl)sulfamoyl)-N-(3,4,5-trifluorophenyl)benzamide (E25)**

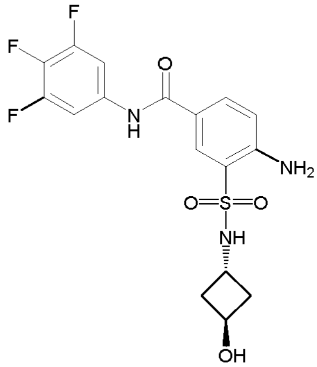

*Trans*-4-fluoro-3-(N-(3-hydroxycyclobutyl)sulfamoyl)-N-(3,4,5-trifluorophenyl)benzamide was prepared according to the procedure described for the synthesis of **D35,** using *trans* 3-aminocyclobutanol instead of 3-aminocyclobutanol. The intermediate compound was further reacted with aqueous ammonia in 1,4-dioxane according to the procedure described for the preparation of **E10**. Method 3; Rt: 3.04. m/z: 416.35 $(M+H)^+$. $^1H$ NMR (300 MHz, DMSO-$d_6$) $\delta$ ppm 1.88 (br dd, *J*=7.93, 4.08 Hz, 2 H) 1.92 - 2.04 (m, 2 H) 3.70 (br d, *J*=7.70 Hz, 2 H) 4.13 (br t, *J*=3.58 Hz, 1 H) 6.54 (br s, 2 H) 6.88 (d, *J*=8.71 Hz, 1 H) 7.61 - 7.81 (m, 2 H) 7.90 (dd, J=8.71, 2.20 Hz, 1 H) 7.97 (d, *J*=8.16 Hz, 1 H) 8.19 (d, *J*=2.20 Hz, 1 H) 10.34 (s, 1 H).

**Example 26: 4-amino-3-(N-((IR,3R)-3-hydroxycyclopentyl)sulfamoyl)-N-(3,4,5-trifluorophenyl)benzamide (E26)**

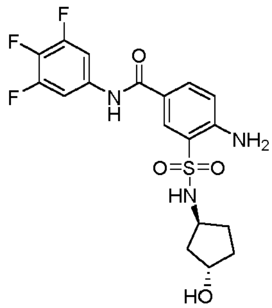

A solution of **D36** (105mg, 0.24mmol) in aqueous ammonia (0.5mL,4.6mmol) and 1,4-Dioxane (0.5mL) was heated for 8hrs at 100°C in a closed vial.. The reaction solution was diluted with DCM/EtOAc (about 7/3) and water, then the organic layer was evaporated giving a residue (80mg) that was purified by preparative HPLC ($H_2O/CH_3CN$+0.1%TFA).Method 3; Rt: 3.11. m/z: 430.27 $(M+H)^+$. 1H NMR (300 MHz, DMSO-$d_6$) $\delta$ ppm 1.12 - 1.36 (m, 2 H) 1.36 - 1.50 (m, 1 H) 1.50 - 1.64 (m, 1 H) 1.64 - 1.90 (m, 3 H) 3.49 - 3.66 (m, 3 H) 3.95 - 4.15 (m, 1 H) 6.53 (br s, 2 H) 6.89 (d, *J*=8.71 Hz, 1 H) 7.66 - 7.77 (m, 3 H) 7.90 (dd, *J*=8.71, 2.20 Hz, 1 H) 8.23 (d, *J*=2.20 Hz, 1 H) 10.35 (s, 1 H)

**Example 27: *tert*-butyl 4-((2-amino-5-((3,4,5-trifluorophenyl)carbamoyl)phenyl)sulfonamido)piperidine-1-carboxylate (E27)**

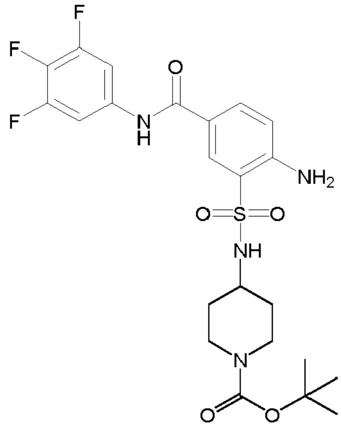

A solution of **D31** (147mg, 0.28mmol) in aqueous ammonia (0.99mL, 2.77mmol) and 1,4-dioxane (1.1mL) was heated at 100°C for 8hrs in a closed vial. Solvent was removed *in vacuo,* the residue was treated with toluene and further evaporated. Traces of solvent were removed by high vacuum pump, giving a residue as off-white solid (107mg). A sample of this crude material (20mg) was purified by preparative HPLC ($H_2O/CH_3CN$+1%TFA) affording the title compound **E27** (19.86mg) as white solid. Method 3; Rt: 3.93min. m/z: 529.09 $(M+H)^+$. $^1H$ NMR (300 MHz, DMSO-$d_6$) δ ppm 1.13 - 1.32 (m, 2 H) 1.36 (s, 9 H) 1.47 - 1.65 (m, 2 H) 2.75 - 2.95 (m, 2 H) 3.05 - 3.23 (m, 1 H) 3.67 (br d, *J*=13.57 Hz, 2 H) 6.53 (br s, 2 H) 6.89 (d, *J*=8.71 Hz, 1 H) 7.64 - 7.78 (m, 2 H) 7.82 (d, *J*=7.89 Hz, 1 H) 7.90 (dd, *J*=8.67, 2.15 Hz, 1 H) 8.24 (d, *J*=2.11 Hz, 1 H) 10.34 (br s, 1 H)

**Example 28: 4-amino-3-(N-(piperidin-4-yl)sulfamoyl)-N-(3,4,5-trifluorophenyl)benzamide (E28)**

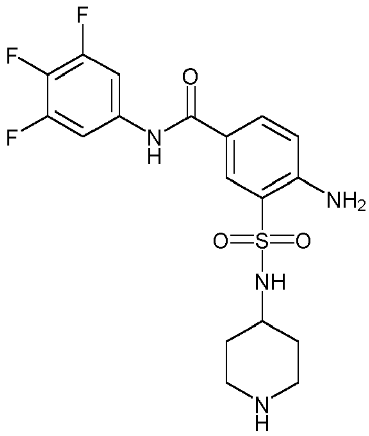

A solution of **E27** in DCM (1mL) was treated at room temperature with TFA (1mL). The yellow reaction solution was magnetically stirred at room temperature for 1h. Solvent was removed *in vacuo* and the residue purified by preparative HPLC ($H_2O/CH_3CN$+1‰TFA) affording the title compound **E28** (8.6mg) as TFA salt. $^1H$ NMR (300 MHz, DMSO-$d_6$) δ ppm 1.40 - 1.64 (m, 2 H) 1.64 - 1.87 (m, 2 H) 2.78 - 3.03 (m, 2 H) 3.08 - 3.28 (m, 3 H) 6.54 (br s, 2 H) 6.91 (d, *J*=8.71 Hz, 1 H) 7.72 (dd, *J*=10,59, 6.56 Hz, 2 H) 7.91 (dd, *J*=8.71, 2.11 Hz, 1 H) 8.03 (d, *J*=7.61 Hz, 1 H) 8.17 (br s, 1 H) 8.25 (d, *J*=2.11 Hz, 1 H) 8.41 (br s, 1 H) 10.36 (br s, 1 H). $^1H$ NMR (300 MHz, DMSO-$d_6$+TFA) δ ppm 1.43 - 1.64 (m, 2 H) 1.75 (br dd, *J*=13.75, 3.30 Hz, 2 H) 2.79 - 3.00 (m, 2 H) 3.08 - 3.22 (m, 2 H) 3.22 - 3.38 (m, 1 H) 6.91 (d, *J*=8.71 Hz, 1 H) 7.61 - 7.81 (m, 2 H) 7.91 (dd, *J*=8.71, 2.20 Hz, 1 H) 8.03 (d, *J*=7.52 Hz, 1 H) 8.13 - 8.34 (m, 2 H) 8.35 - 8.60 (m, 1 H) 10.35 (s, 1 H). Method 3; Rt: 2.48min. m/z: 429.26 $(M+H)^+$.

**Example 29: 4-amino-3-((4-hydroxypiperidin-1-yl)sulfonyl)-N-(3,4,5-trifluorophenyl)benzamide (E29)**

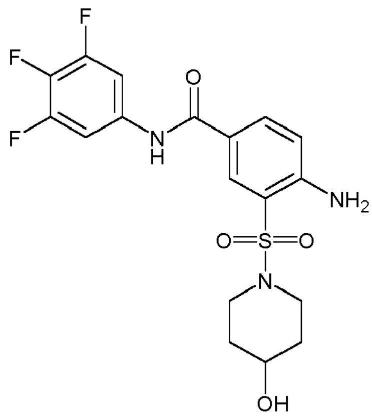

A mixture of 4-fluoro-3-((4-hydroxypiperidin-1-yl)sulfonyl)-N-(3,4,5-trifluorophenyl)benzamide (prepared according to WO2013/096744) (50mg, 0.11mmol), 1,4-dioxane (150uL) and aqueous ammonia (0.3mL, 2.29mmol) were heated at 100°C for 8hrs in a sealed tube. The reaction was diluted with EtOAc and water, organic layer was dried over $Na_2SO_4$, filtered and finally evaporated. The residue was suspended in DCM and filtered affording the title compound **E29** (15.5mg) as white solid. Method 3; Rt: 3.26min. m/z: 430.2 $(M+H)^+$. $^1H$ NMR (300 MHz, DMSO-$d_6$) $\delta$ ppm 1.30 - 1.52 (m, 2 H) 1.60 - 1.83 (m, 2 H) 2.77 - 2.92 (m, 2 H) 3.25 (br s, 2 H) 3.45 - 3.65 (m, 1 H) 4.67 (d, *J*=3.76 Hz, 1 H) 6.53 - 6.75 (m, 2 H) 6.93 (d, *J*=8.71 Hz, 1 H) 7.69 (dd, *J*=10,50, 6.46 Hz, 2 H) 7.81 - 7.98 (m, 1 H) 8.08 (d, *J*=2.02 Hz, 1 H) 10.31 (s, 1 H)

**Example 30: 3-((4-hydroxypiperidin-1-yl)sulfonyl)-4-(methylamino)-N-(3,4,5-trifluorophenyl)benzamide (E30)**

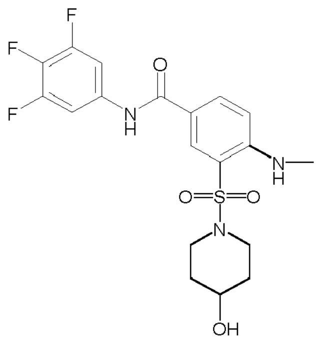

A mixture of 3-((4-hydroxypiperidin-1-yl)sulfonyl)-N-(3,4,5-trifluorophenyl)benzamide (prepared according to WO2013/096744) (50mg, 0.12mmol), methanamine (1.04mL, 2.08mmol) in a DMSO (700uL,0.010mol) and MeCN (140uL,0.003mol) mixture was treated with triethylamine (480.87uL, 3.47mmol) and stirred at room temperature overnight. The reaction solution was purified by preparative HPLC ($H_2O/CH_3CN$+1%TFA) affording the title compound **E30** (23mg). $^1H$ NMR (300 MHz, DMSO-$d_6$) $\delta$ ppm 1.28 - 1.51 (m, 2 H) 1.58 - 1.81 (m, 2 H) 2.75 - 2.97 (m, 5 H) 3.19 - 3.28 (m, 2 H) 3.46 - 3.65 (m, 1 H) 4.66 (d, *J*=3.90 Hz, 1 H) 6.72 - 6.81 (m, 1 H) 6.89 (d, *J*=8.90 Hz, 1 H) 7.63 - 7.77 (m, 2 H) 8.03 - 8.11 (m, 1 H) 8.15 (d, *J*=2.11 Hz, 1 H) 10.35 (s, 1 H).

**Example 31: 4-amino-3-(N-(pyridin-4-yl)sulfamoyl)-N-(3,4,5-trifluorophenyl)benzamide (E31)**

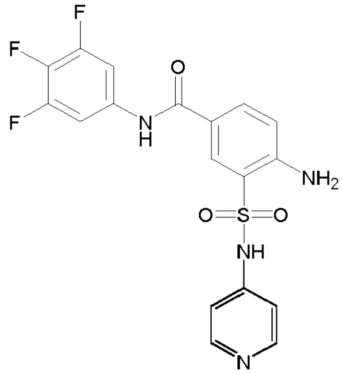

A 5mL vial was charged with **D32** (100mg, 0.24mmol) dioxane (1mL) and aqueous ammonia (2mL). The vial was sealed and heated for 8hrs at 100°C. Solvents were removed by evaporation and the residue partitioned between water and EtOAc. The organic extract were combined and evaporated, the residue was dissolved in 1/2 dioxane/aqueous ammonia (2mL) and heated in a closed vial for 8hrs at 100°C. Solvent was removed *in vacuo* and the residue partitioned betweeen water and EtOAc. The organic extract was evaporated and the residue was purified by flash chromatography on direct phase (EtOAc/MeOH). The fractions containing the product were combined and evaporated affording a residue (20mg) that was purified by preparative HPLC ($H_2O/CH_3CN$+1‰TFA) affording the title compound **E31** as TFA salt (1.24mg). Method 3; Rt: 2.63min. m/z: 423.1 $(M+H)^+$. $^1H$ NMR (300 MHz, DMSO-$d_6$) δ ppm 6.52 (br s, 1 H) 6.82 (d, *J*=8.62 Hz, 1 H) 7.03 (br d, *J*=6.88 Hz, 2 H) 7.66 - 7.79 (m, 2 H) 7.83 (dd, *J*=8.67, 2.16 Hz, 1 H) 8.03 (d, *J*=6.00 Hz, 2 H) 8.35 (d, *J*=2.11 Hz, 1 H) 10.35 (s, 1 H).

**Example 32: 4-amino-3-(N-(3-hydroxycyclobutyl)sulfamoyl)-N-(3,4,5-trifluorophenyl)benzamide (E32)**

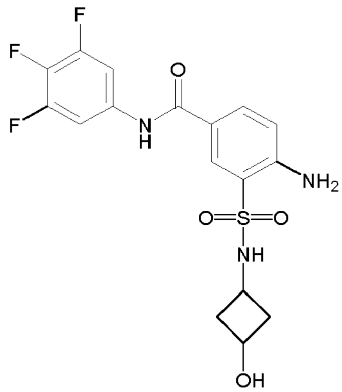

Similarly prepared according to procedure described for the preparation of **E10**, starting from **D35.** Method 1; Rt: 1.81min. m/z: 416.40$(M+H)^+$.

**Example 33: 4-amino-3-(N-(oxetan-3-yl)sulfamoyl)-N-(3,4,5-trifluorophenyl)benzamide (E33)**

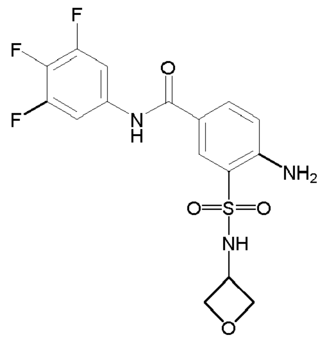

Similarly prepared according to procedure described for the preparation of **E10**, starting from **D33.** Method 3; Rt: 3.20. m/z: 402.30(M+H)$^+$. 1H NMR (300 MHz, DMSO-$d_6$) δ ppm 4.23 - 4.43 (m, 3 H) 4.44 - 4.54 (m, 2 H) 6.58 (br s, 2 H) 6.90 (d, *J*=8.71 Hz, 1 H) 7.60 - 7.80 (m, 2 H) 7.90 (dd, *J*=8.71, 2.20 Hz, 1 H) 8.18 (d, *J*=2.20 Hz, 1 H) 8.66 (br s, 1 H) 10.36 (br s, 1 H).

**Example 34: *tert-butyl* (S)-3-((2-amino-5-((3,4,5-trifluorophenyl)carbamoyl)phenyl)sulfonamido)pyrrolidine-1-carboxylate (E34)**

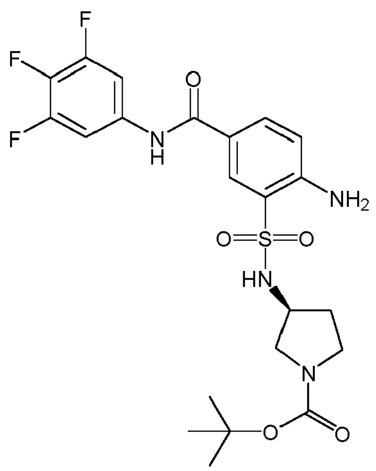

Prepared similary as described for the preparation of **E10** using as starting material **D34** and purified by preparative HPLC ($H_2O/CH_3CN$+ 0.1%HCOOH),. Method 3; Rt: 3.81min. m/z: 515.24(M+H)$^+$.$^1$H NMR (300 MHz, DMSO-$d_6$) δ ppm 1.35 (br d, *J*=5.69 Hz, 9 H) 1.55 - 1.77 (m, 1 H) 1.78 - 2.00 (m, 1 H) 2.88 - 3.05 (m, 1 H) 3.07 - 3.31 (m, 4 H) 3.64 (br s, 1 H) 6.56 (br s, 2 H) 6.91 (d, *J*=8.71 Hz, 1 H) 7.64 - 7.79 (m, 2 H) 7.92 (dd, *J*=8.71, 2.11 Hz, 1 H) 8.07 (br s, 1 H) 8.24 (d, *J*=2.02 Hz, 1 H) 10.37 (s, 1 H).

**Example E35: (S)-4-amino-3-(N-(pyrrolidin-3-yl)sulfamoyl)-N-(3,4,5-trifluorophenyl)benzamide (E35)**

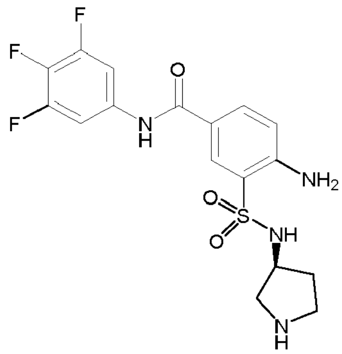

A solution of **E34** (30mg, 0.06mmol) in DCM (0,5mL) was treated with trifluoroacetic acid (0,5mL, 6.53mmol) at room temperature. Solvent was removed *in vacuo,* giving a residue that was purified by preparative HPLC ($H_2O/CH_3CN$ 0.1 %TFA) giving the title compound **E35** (10mg) as white solid. Method 3; Rt: 2.47min. m/z: 415.27 $(M+H)^+$. $^1H$ NMR (300 MHz, DMSO-$d_6$) δ ppm 1.61 - 1.85 (m, 1 H) 1.85 - 2.10 (m, 1 H) 2.94 (br s, 1 H) 3.17 (br s, 3 H) 3.57 - 3.88 (m, 1 H) 6.60 (br s, 2 H) 6.95 (d, *J*=8.71 Hz, 1 H) 7.63 - 7.80 (m, 2 H) 7.95 (dd, *J*=8.76, 2.16 Hz, 1 H) 8.16 (d, *J*=6.42 Hz, 1 H) 8.24 (d, *J*=2.11 Hz, 1 H) 8.47 - 8.68 (m, 1 H) 8.70 - 9.02 (m, 1 H) 10.38 (s, 1 H).

**Example 36: 4-amino-3-methyl-5-sulfamoyl-N-(3,4,5-trifluorophenyl)benzamide (E36)**

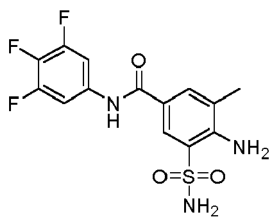

A pressure vessel was charged with **D38** (217mg, 0.600mmol), 1,4-dioxane (1.5mL) and 33% aqueous ammonia (0.75mL,6.36mmol). The pressure vessel was sealed and the reaction mixture was heated at 95°C for 7.5h, then stirred at RT overnight. More 33% aqueous ammonia (0,5mL, 4.24mmol) was added, and the reaction mixture was stirred at 100°C for another 8.5h. The reaction was diluted with EtOAc and water, organic layer was dried over $Na_2SO_4$, filtered and concentrated under vacuo. The residue was triturated with DCM, then was purified by preparative HPLC ($H_2O/CH_3CN$+1%TFA) to obtain, after lyophilisation the title compound **E36** (86mg) as off-white solid. Method 3; Rt=3.19min, m/z=360.15 $(M+H)^+$. $^1H$ NMR (300 MHz, DMSO-$d_6$) δ ppm 2.22 (s, 3 H) 6.21 (s, 2 H) 7.41 (s, 2 H) 7.65 - 7.79 (m, 2 H) 7.80 - 7.89 (m, 1 H) 8.19 (d, *J*=2.02 Hz, 1 H) 10.33 (s, 1 H).

**Example 37: 6-amino-5-sulfamoyl-N-(3,4,5-trifluorophenyl)nicotinamide (E37)**

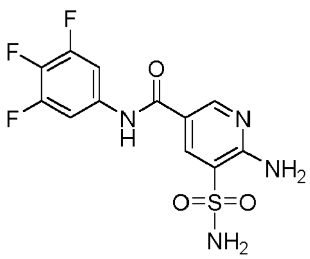

Similarly prepared according to the procedure described for the preparation of compound **E3,** starting from **D40.** Method 3: Rt=2.82min, m/z=347.04 $(M+H)^+$. $^1H$ NMR (300 MHz, DMSO-$d_6$) δ ppm 7.07 (br s, 2 H) 7.60 (s, 2 H) 7.65 - 7.81 (m, 2 H) 8.46 (d, *J*=2.20 Hz, 1 H) 8.80 (d, *J*=2.20 Hz, 1 H) 10.48 (s, 1 H).

The examples shown in Table 1 were prepared according to the synthetic methods described above. The general synthetic strategies, the appropriate intermediate materials and the relevant reaction steps (where appropriate), are indicated in Table 1 by referring to the appropriate Scheme.

**Table 1**

| Example | Compound Name | Structure | (ES+) m/z | Reaction Scheme, Intermediate |
|---|---|---|---|---|
| E38 | 4-amino-3-(N-(3-(hydroxymethyl) oxetan-3-yl)sulfamoyl)-N-(3,4,5-trifluorophenyl)benzamide | 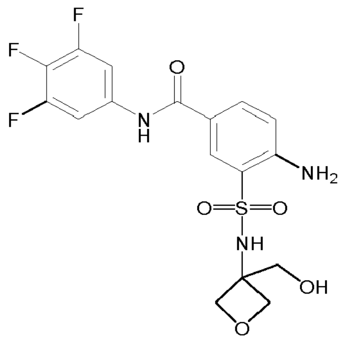 | 432 | Scheme 1 D7 → STEP F → STEP H |
| E39 | 4-amino-3-(N-((1-hydroxycyclohexyl) methyl)s ulfamoyl)-N-(3,4,5-trifluorophenyl)benzamide | 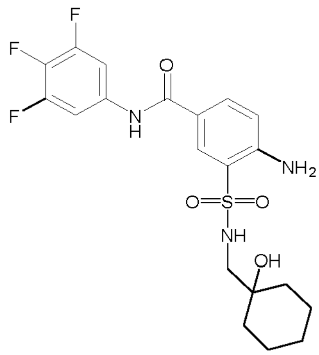 | 458 | Scheme 1 D7 → STEP F → STEP H |
| E40 | 4-amino-N-(4-fluoro-3-methylphenyl)-2-methyl-5-sulfamoylbenzamide | 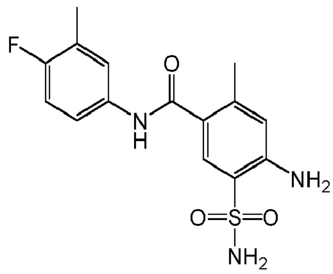 | 338 | Scheme 1 D6 → STEP B → STEP C |
| E41 | 4-amino-5-(N-((1r,4r)-4-hydroxycyclohexyl)sulfamoy 1)-2-methyl-N-(3,4,5-trifluorophenyl) benzamide | 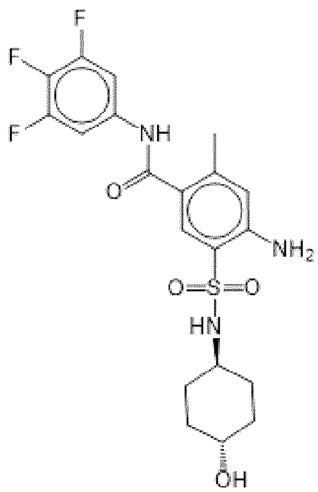 | 458 | Scheme 1 D14 → STEP F → STEP H |

(continued)

| Example | Compound Name | Structure | (ES+) m/z | Reaction Scheme, Intermediate |
|---|---|---|---|---|
| E42 | 4-amino-3-(N-(1-(pyridin-2-yl)ethyl) sulfamoyl)-N-(3,4,5-trifluorophenyl) benzamide | 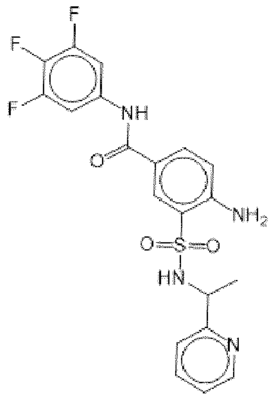 | 451 | Scheme 1 D7 → STEP F → STEP H |
| E43 | trans-4-amino-N-(3-chloro-4-fluorophenyl)-3-(N-(4-hydroxycyclohexyl)sulfamoy l) benzamide | 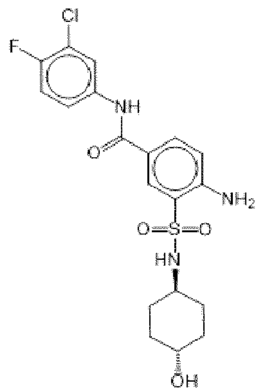 | 442 | Scheme 1 D9 → STEP F → STEP H |
| E44 | 4-amino-N-(3-(difluoromethyl)-4-fluorophenyl)-3-(N-((1r,4r)-4-hydroxycyclohexyl)sulfamoy l) benzamide | 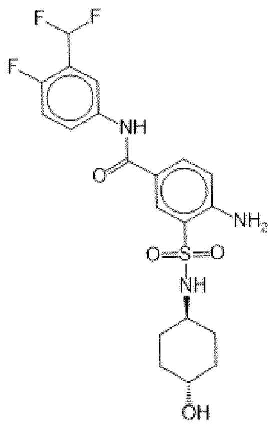 | 458 | Scheme 1 D12 → STEP F → STEP H |

(continued)

| Example | Compound Name | Structure | (ES+) m/z | Reaction Scheme, Intermediate |
|---|---|---|---|---|
| E46 | trans-4-amino-N-(3-(difluoromethyl)-4-fluorophenyl)-3-(N-(4-hydroxycyclohexyl)sulfamoy l) benzamide | 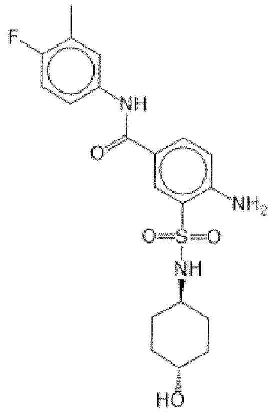 | 422 | D15 → STEP F → STEP H |
| E47 | 4-amino-3-(N-((1S,3R)-3-hydroxycyclopentyl)sulfamo yl)-N-(3,4,5-trifluorophenyl) benzamide | 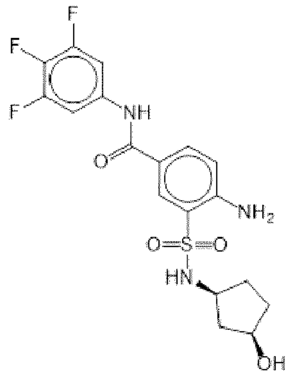 | 430 | Scheme 1 D7 → STEP F → STEP H |
| E48 | 4-amino-3-(N-(1,3-dihydroxypropan-2-yl)sulfamoyl)-N-(3,4,5-trifluorophenyl)benzamide | 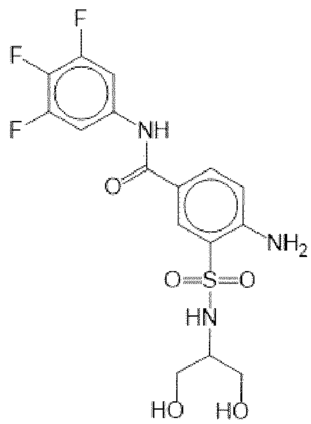 | 420 | Scheme 1 D7 → STEP F → STEP H |

(continued)

| Example | Compound Name | Structure | (ES+) m/z | Reaction Scheme, Intermediate |
|---|---|---|---|---|
| E49 | 4-amino-3-(N-((1R,3S)-3-hydroxycyclopentyl)sulfamoyl)-N-(3,4,5-trifluorophenyl) benzamide | 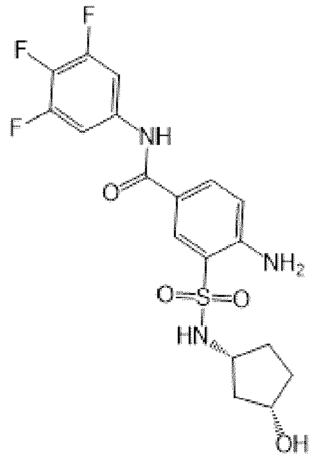 | 430 | Scheme 1 D7 → STEP F → STEP H |
| E50 | 4-amino-3-((4-hydroxy-4-(hydroxymethyl)piperidin-1-yl) sulfonyl)-N-(3,4,5-trifluorophenyl) benzamide | 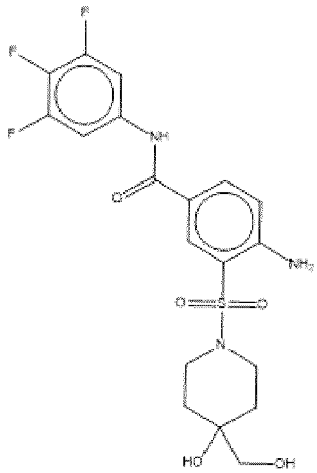 | 460 | Scheme 1 D7 → STEP F → STEP H |
| E51 | tert-butyl ((1R,2S)-2-((2-amino-5-((3,4,5-trifluorophenyl)carbamoyl)phenyl)sulfonamido)cyclopent yl) carbamate | 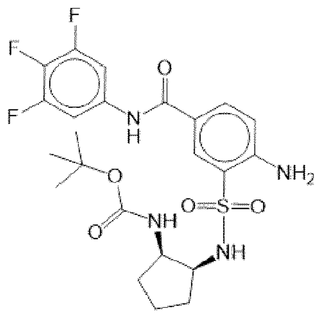 | 529 | Scheme 1 D7 → STEP F → STEP H |
| E52 | tert-butyl ((1S,2R)-2-((2-amino-5-((3,4,5-trifluorophenyl)carbamoyl)phenyl)sulfonamido)cyclopent yl) carbamate | 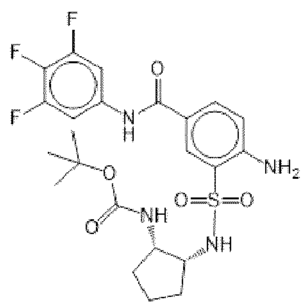 | 529 | Scheme 1 D7 → STEP F → STEP H |

(continued)

| Example | Compound Name | Structure | (ES+) m/z | Reaction Scheme, Intermediate |
|---|---|---|---|---|
| E53 | 4-amino-3-((3-hydroxypyrrolidin-1-yl) sulfonyl)-N-(3,4,5-trifluorophenyl) benzamide | 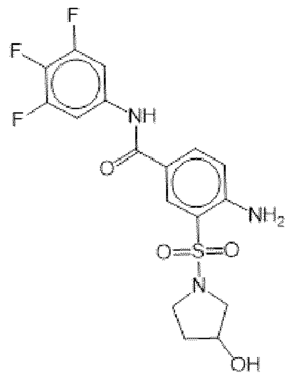 | 416 | Scheme 1 D7 → STEP F → STEP H |
| E54 | 4-amino-N-(3-chloro-4-fluorophenyl)-3-((4-hydroxypiperidin-1-yl)sulfonyl) benzamide | 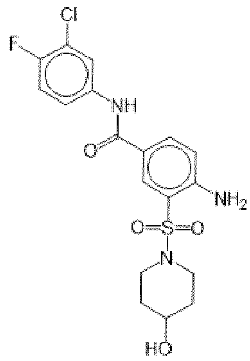 | 428 | Scheme 1 D9 → STEP F → STEP H |
| E55 | 4-amino-N-(3-chloro-4-fluorophenyl)-3-((3-hydroxyazetidin-1-yl)sulfonyl) benzamide | 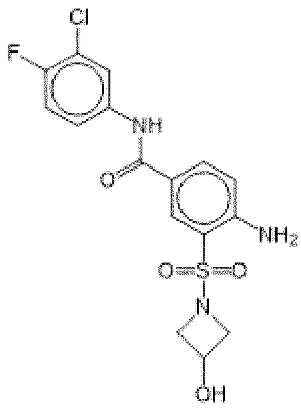 | 400 | Scheme 1 D9 → STEP F → STEP H |
| E56 | 4-amino-3-(N-(2,3-dihydroxypropyl) sulfamoyl)-N-(3,4,5-trifluorophenyl) benzamide | 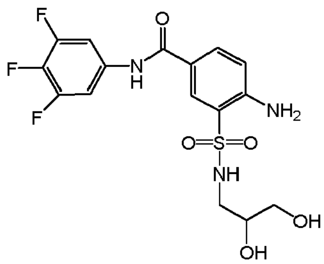 | 420 | Scheme 1 D7 → STEP F → STEP H |

(continued)

| Example | Compound Name | Structure | (ES+) m/z | Reaction Scheme, Intermediate |
|---|---|---|---|---|
| E57 | trans-4-amino-3-(N-(3-hydroxycyclopentyl)sulfamoyl)-N-(3,4,5-trifluorophenyl) benzamide | 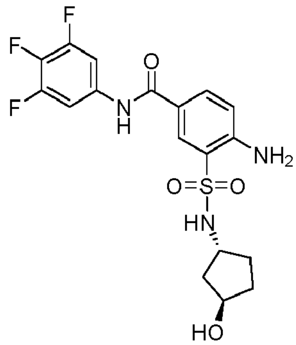 | 430 | Scheme 1 D7 → STEP F → STEP H |
| E58 | trans-6-amino-5-(N-(4-hydroxycyclohexyl)sulfamoyl)-N-(3,4,5-trifluorophenyl) nicotinamide | 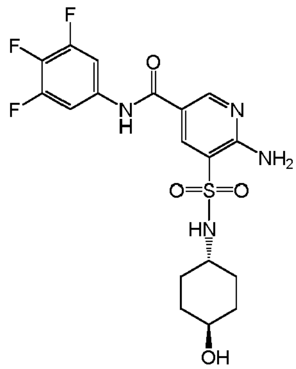 | 445 | Scheme 3 |
| E59 | 2-amino-5-sulfamoyl-N-(3,4,5-trifluorophenyl)benzamide | 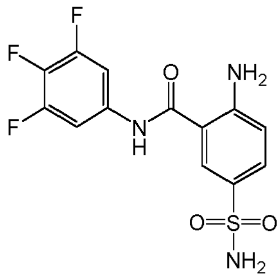 | 346 | Scheme 4 |
| E60 | 4-amino-N-(3-chloro-4-fluorophenyl)-2-methyl-5-sulfamoylbenzamide | 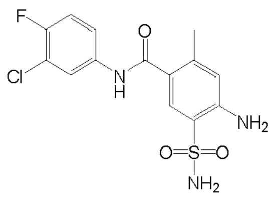 | 358 | Scheme 1 D6 → STEP B → STEP C |

(continued)

| Example | Compound Name | Structure | (ES+) m/z | Reaction Scheme, Intermediate |
|---|---|---|---|---|
| E61 | trans-2-amino-5-(N-(4-hydroxycyclohexyl)sulfamoyl)-N-(3,4,5-trifluorophenyl) benzamide | 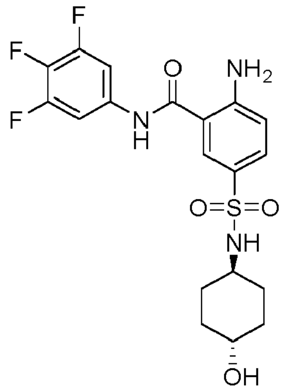 | 444 | Scheme 4 |
| E62 | 2-amino-5-((4-hydroxypiperidin-1-yl) sulfonyl) -N-(3,4,5-trifluorophenyl) benzamide | 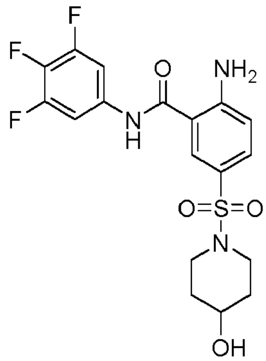 | 430 | Scheme 4 |
| E63 | (R)-4-amino-2-methyl-N-(3,4,5-trifluorophenyl)- 5-(N-(1,1,1-trifluoropropan-2-yl)sulfamoyl) benzamide | 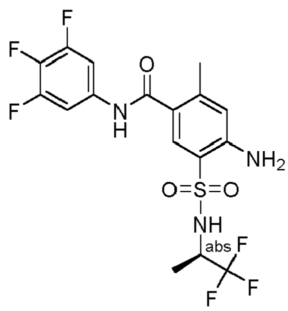 | 456 | Scheme 1 D14 → STEP F → STEP H |
| E64 | (S)-4-amino-2-methyl-N-(3,4,5-trifluorophenyl)- 5-(N-(1,1,1-trifluoropropan-2-yl)sulfamoyl) benzamide | 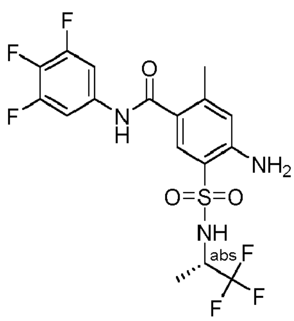 | 456 | Scheme 1 D14 → STEP F → STEP H |

(continued)

| Example | Compound Name | Structure | (ES+) m/z | Reaction Scheme, Intermediate |
|---|---|---|---|---|
| E65 | 4-amino-N-(3-chloro-4,5-difluorophenyl)-2-methyl-5-sulfamoylbenzamide | 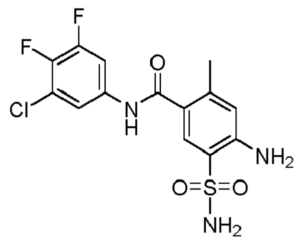 | 376 | Scheme 1 D6 → STEP B → STEP C |
| E66 | 4-amino-N-(6-chloropyridin-3-yl)-2-methyl-5-sulfamoylbenzamide | 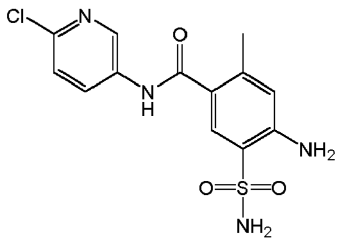 | 341 | Scheme 1 D6 → STEP B → STEP C |
| E67 | 4-amino-N-(4-fluoro-3-(trifluoromethyl)phenyl)-2-methyl-5-sulfamoylbenzamide | 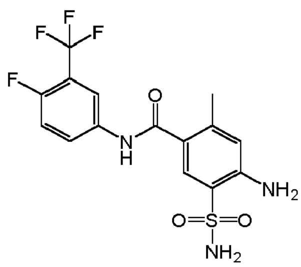 | 392 | Scheme 1 D6 → STEP B → STEP C |
| E68 | 4-amino-N-(3-(difluoromethyl)-4-fluorophenyl)-2-methyl- 5-sulfamoylbenzamide | 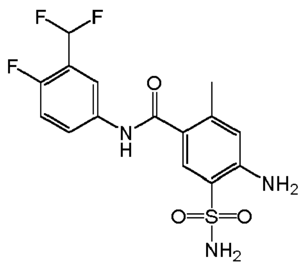 | 374 | Scheme 1 D6 → STEP B → STEP C |
| E69 | 4-amino-N-(3-cyano-4-fluorophenyl)-2-methyl-5-sulfamoylbenzamide | 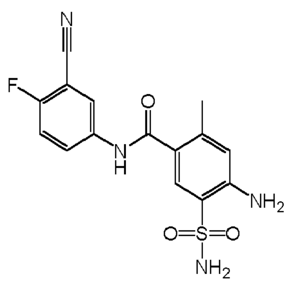 | 349 | Scheme 1 D6 → STEP B → STEP C |

***Biology***

*Assay*

**Cells and culture conditions**

HepAD38 cell line (Ladner et al., Antimicrob Agents Chemother, 1997, 41, 1715-20) was used for HBV inhibition

assays. HepAD38 is a subclone, derived from Hepatoblastoma cell line HepG2 (ATCC® Number: HB-8065™), that expresses HBV genome under the control of a tetracycline-responsive promoter in a TET-OFF system: addition of tetracycline suppresses HBV replication, while its removal switches on the process allowing HBV viral particles release in the cell supernatant. HepAD38 cell line is maintained in DMEM/F12, supplemented with 10% of fetal bovine serum, 1% of glutamine, 1% of penicillin/streptomycin, 0.4 mg/ml G418 and 0,3 ug/ml tetracycline. For the HBV inhibition assay, doxycycline-free medium is used in order to allow virion production.

**Anti-HBV activity *in vitro***

HBV inhibition activity in vitro was performed in 96 multiwell plates. During the initial (primary) screening compounds were first tested in triplicates at concentrations of 0.1μM, 0.5μM amd 1μM. For selected compounds, an 8-point dose-response curve can be obtained using 1:2 serial dilutions (starting from 2.5 μM, 1.25μM or 0.4 μM, depending on the degree of inhibition observed during the primary screening). From the dose-response curves, half maximal effective concentration ($EC_{50}$) can be calculated (see also below).

In more detail, compounds - typically dissolved in DMSO stock solutions - are diluted to 2x the final desired concentration in 100 μl of the above medium (without doxycycline) and plated in three replicates in the 96-well plates.

Simultaneously, HepAD38 cells - extensively pre-washed in tetracycline-free medium in order to induce HBV production - are suspended at $2*10^4$ cells in 100 μl of tetracycline-free medium and added to each well of the plate, to yield a final assay volume of 200 μl DMSO - used for stock solutions and compounds dilutions - which is always present in the assays at a final concentration of 0.5%.

Plates are then incubated 96 hours at 37°C and then subjected to cell viability assay in order to define compounds cytotoxicity and to extracellular HBV quantification in order to evaluate antiviral activity of compounds. Cytotoxicity is assessed by a commercial fluorescence assay that measures the metabolic activity of cells, directly related to cell viability (Cell Titer Blue, Promega). Anti-HBV activity was evaluated by quantification of extracellular HBV DNA with direct qPCR. In particular, supernatant was collected and centrifuged for cell debris clarification, viral DNA was extracted from virions by addition of lysis buffer (1 mM 1,4-dithiothreitol, 0.2% sodium dodecyl sulphate) and incubated at 95°C for 10 min. Samples were then diluted 1:40 and real time PCR amplification was performed with SYBR green assay (Power SYBR™ Green PCR Master Mix-Thermo Fisher Scientific) and specific HBV primer (HBV-DF:5'-ATTTGTTCAGTGGTTCGTAGGG-3' (SEQ ID No. 1), HBV-DR:5'-CGGTAAAAAGGGACTCAAGATG-3' (SEQ ID No. 2)).

All HBV inhibition or antiviral activity data are typically reported in percent (%) relative to a non-treated reference sample. Excel and Graphpad Prism programs are typically used for data elaboration and $EC_{50}$ calculation.

*Pharmacokinetics*

**Hepatocytes Stability studies**

Hepatic metabolism is often a major contributor to drug clearance from the body. Pooled cryopreserved human hepatocytes are thawed and re-suspended in Hepatocyte Culture Medium (HCM) complete. Compounds are diluted into the cell suspension (2.5 μl/2.5 mL, 1 million cells/mL) from 3 mM stock solution to give a 3 μM concentration (0.1% DMSO). 150 μl (x2) of this mixture are taken and transferred into a 96-deep-well plate containing an equal volume of quenching solution (100% acetonitrile plus 0.1 % formic acid) for time 0 incubation. Then 1 mL/well of the cell suspension-compound mixture (x2) are dispensed in 24-well plates. Incubations are performed at 37°C in a DUBNOFF water bath, under low shaking. Compounds are tested at 6 time points in duplicate: 0, 30, 60, 120, 180 and 240 min. At each time point, an aliquot of 150 μl is taken, transferred into a 96-deep-well plate and then the reaction is stopped by addition of one volume of 100% acetonitrile plus 0.1 % formic acid.

At 0, 120 and 240 min viability measurements by trypan blue exclusion test are performed. Samples are centrifuged at 1 100xg for 30 min at +4 °C and 250 μl of the supernatant are transferred to a new 96-deep-well plate for bio analytical.

Analytical Procedures:

The samples are centrifuged at 4500 g at 4°C for 5 minutes and split into two 96 deep-well plates. The study samples are further n-fold diluted or dried under nitrogen at 25°C and reconstituted according to the analytical method developed. Final plates are mixed for 10min, sonicated for 5 min and the samples are injected into LC-MS/MS or LC-HRMS system. Sample analyses were performed using an API 4000QTrap Mass Spectrometer interfaced via the Turbo Ion Spray (ESI) to an LC system consisting of an Acquity UPLC Sample Manager autosampler and Acquity UPLC Binary Solvent Manager Pump or a Thermo Scientific Orbitrap QExactive interfaced to an LC system consisting of a Dionex Ultimated 3000 UHPLC.

Results:

Stability is determined based on analysis of the disappearance of the compound as a function of incubation time, using area ratio (analyte peak area vs internal standard peak area). The elimination constant k is calculated by plotting mean disappearance values on a semi-logarithmic scale and fitting with a best fit linear regression. The half-life (t1/2) expressed in hours is derived using Equation 1: Equation 1: t1/2 = ln2/(-k). When the half-life could not be calculated, data are reported as: <0.5 or >4 hours. Intrinsic clearance given in μl/min/million cells is calculated using the Equation: Clint=KV/N .Where K=0.693/tl/2, V=incubation volume (ml) and N=number of hepatocytes/sample.

**Mouse PK studies**

C57BL/6 mice are used to evaluate plasma and liver exposure and pharmacokinetic parameters after intravenous and oral administration (from 2 to 200 mpk according to the compound tested and the administration route). 12 (+ 3 spare) healthy C57BL/6N male mice are obtained from Charles River S.p.A. Calco (Como), Italy. Animals are ordered weighing 21 to 27 grams and approximately 7 weeks old. Before and during testing, animals are housed in Individual Ventilated Cages (IVCs Tecniplast) with sawdust as bedding (three animals for cage). Cages are identified by a color code label recording the sample ID, animal number and details of treatment (route, dose and sex). Animals are identified by unique number on tail via permanent marker. Animal room controls are set to maintain temperature within the range from 20 to 24 °C and relative humidity within the range from 40 to 70 % and an average daily airflow of at least 10 fresh air changes per hour. Actual conditions are recorded. The room is lit by fluorescent tubes controlled to give an artificial cycle of 12 hours light and 12 hours dark each day. All animals are weighed immediately before testing. Animals are dosed IV in a fed state and PO in a fasted state.

Compounds (from 0.4 to 20 mg/mL according to the compound tested and the administration route) are dissolved in DMSO/PEG400/$H_2O$ (20/60/20) for IV administration and 0.5% Methocel E50 or 20% Hydroxypropyl-β-cyclodextrin (HP-β-CD) in citrate buffer pH=5 for PO administration. The appropriate dose volume of the test item, calculated for each animal according to body weight (administration volume: 5 mL/kg for IV or 5 mL/kg or 10 mL/kg for PO), is administered by injection into lateral tail vein using 2.5 mL syringe (BD Plasipak) for IV administration and administered orally by gavage using 5mL Syringe (BD Plastipak) for PO administration. Whole blood sample (about 0.200 mL) is collected via retro orbital sinus using Isoflurane as anesthetic. Blood is collected in Li-Heparin Sartsted$^R$ gel tubes appropriately labeled indicating animal number and time point. Tubes are put in wet ice and then centrifuged within 15 minutes from blood collection. Centrifugation is performed using a Heraeus Multifuge$^R$ 3S/3S-R set, at 2200 x g for 10 minutes, internal temperature is kept at 4°C. After the centrifugation, about 100μL of plasma samples are obtained and immediately transferred to 1.5 mL Eppendorf tubes and frozen at -20 °C (24/24 h alarmed).Whole blood samples are collected at different times point (0 - 0.25 - 0.5 - 1 - 2 - 4 - 6 - 8 - 24 hours) after dosing and kept frozen (-20°C) until assayed by LC/MS/MS. Livers are explanted at different time point (0 - 8 - 24 hours), washed with saline solution, transferred to Eppendorf tubes and frozen at -80°C until assayed by LC/MS/MS. Analytical procedures: Plasma samples are extracted using Liquid Handling Robot Hamilton StarPlus by protein precipitation with acetonitrile. Then the samples are centrifuged (3000 rpm x 15 min at 4 °C) and the supernatant transferred and dried under nitrogen. The samples are reconstituted in water/acetonitrile 90/10 or 50/50 and then injected directly into an UPLC column. Sample analyses are performed using an API 4000 or/and API 5000 or/and API 4000QTrap Mass Spectrometer interfaced via the Turbo Ion Spray (ESI) to an LC system consisting of an Acquity UPLC Sample Manager autosampler and Acquity UPLC Binary Solvent Manager Pump. The results are calculated using Analyst Software linear regression with 1/x*x weighting. The Assay Precision is calculated for the Quality Controls by Watson Lims database.

Cmax is the maximum compound concentration from oral dosing; Tmax is the time at which Cmax is reached; AUC (0-24) is the area under the concentration vs time curve from 0 to 24 hours; AUC extrap is the area under the curve (AUC) extrapolated to infinity, from dosing time based on the last observed concentration.

Pharmacokinetic Analysis: The plasma clearance (CLp) of the compounds is calculated (using Watson PK program) as the dose divided by the area under the plasma concentration-time curve from time zero to infinity ($AUC_{0-\infty}$). The apparent half-life is estimated from the slope of the terminal phase of the log plasma concentration-time data. The volume of distribution (Vdss) is determined using the following noncompartmental method:

$$Vdss = (Dose\ IV \times AUMC)/(AUC_{0-\infty})2$$

where AUMC is the total area under the first moment of the drug concentration time curve from time zero to infinity. Bioavailability is estimated as the $AUC_{0-\infty}$ ratio following oral and intravenous administration, normalized for differences in dose.

RESULTS

The exemplified compounds described herein were tested in the assays described above. All the compounds displayed no measurable cytotoxicity at the tested compound concentration.

Results for HBV inhibition are reported in the following Table 2.

Legend: A indicates HBV inhibition greater than 50% at the concentration indicated in the table or $EC_{50}$ less than 1μM; B indicates HBV inhibition less than 50% at the concentration indicated in the table or $EC_{50}$ greater than 1μM.

**Table 2**

| Example | Compound Name | HBV inhibition % (conc μM) | HBV inh $EC_{50}$ (μM) |
|---|---|---|---|
| E1 | 4-(methylamino)-3-sulfamoyl-*N*-(3,4,5-trifluorophenyl)benzamide | B (1) | - |
| E2 | 4-amino-3-(N-methylsulfamoyl)-N-(3,4,5-trifluorophenyl) benzamide | A (1) | - |
| E3 | 4-amino-3-sulfamoyl-*N*-(3,4,5-trifluorophenyl)benzamide | A (0.5) | - |
| E4 | 4-amino-*N*-(3,4-difluorophenyl)-3-sulfamoylbenzamide | A (0.5) | - |
| E5 | 4-amino-2-chloro-5-sulfamoyl-N-(3,4,5-trifluorophenyl)benzamide | - | A |
| E6 | 4-amino-2-bromo-5-sulfamoyl-N-(3,4,5-trifluorophenyl)benzamide | A (1) | - |
| E7 | 4-amino-N-(4-fluoro-3-(trifluoromethyl)phenyl)-3-sulfamoylbenzamide | A (0.5) | - |
| E8 | 4-amino-N-(3-cyano-4-fluorophenyl)-3-sulfamoylbenzamide | A (0.5) | - |
| E9 | 4-amino-N-(3-(difluoromethyl)-4-fluorophenyl)-3-sulfamoylbenzamide | A (0.5) | - |
| E10 | 4-amino-N-(3-chloro-4-fluorophenyl)-3-sulfamoylbenzamide | A (0.5) | - |
| E11 | 4-amino-N-(6-chloropyridin-3-yl)-3-sulfamoylbenzamide | B (0.5) | - |
| E12 | 4-amino-N-(4-fluoro-3-methylphenyl)-3-sulfamoylbenzamide | A (0.5) | - |
| E13 | 4-amino-N-(3,5-difluoro-4-methylphenyl)-3-sulfamoylbenzamide | B (0.5) | - |
| E14 | 4-amino-3-sulfamoyl-N-(2,3,4-trifluorophenyl)benzamide | B (0.5) | - |
| E15 | 4-amino-3-sulfamoyl-N-(2,4,5-trifluorophenyl)benzamide | B (0.5) | - |
| E16 | 4-amino-N-(2-chloro-4-fluorophenyl)-3-sulfamoylbenzamide | B (0.5) | - |
| E17 | 4-amino-2-methyl-5-sulfamoyl-N-(3,4,5-trifluorophenyl)benzamide | - | A |
| E18 | (R)-4-amino-N-(3,4,5-trifluorophenyl)-3-(N-(1,1,1-trifluoropropan-2-yl) sulfamoyl)benzamide | A (0.5) | - |
| E19 | (S)-4-ammo-N-(3,4,5-trifluorophenyl)-3-(N-(1,1,1-trifluoropropan-2-yl) sulfamoyl)benzamide | A (0.5) | - |
| E20 | 4-amino-3-(N-cyclopropylsulfamoyl)-N-(3,4,5-trifluorophenyl) benzamide | A (0.5) | - |
| E21 | *trans*-4-amino-3-(N-(4-hydroxycyclohexyl)sulfamoyl)-N-(3,4,5-trifluorophenyl)benzamide | - | A |
| E22 | *cis*-4-amino-3-(N-(4-hydroxycyclohexyl)sulfamoyl)-N-(3,4,5-trifluorophenyl)benzamide | A (0.5) | - |
| E23 | *trans*-4-amino-5-(N-(4-hydroxycyclohexyl)sulfamoyl)-2-methyl-N-(3,4,5-trifluorophenyl)benzamide | A (0.5) | - |
| E24 | *cis*-4-amino-3-(N-3-hydroxycyclobutyl)sulfamoyl)-N-(3,4,5-trifluorophenyl)benzamide | A (0.5) | - |
| E25 | *trans*-4-amino-3-(N-3-hydroxycyclobutyl)sulfamoyl)-N-(3,4,5-trifluorophenyl)benzamide | A (0.5) | - |

(continued)

| Example | Compound Name | HBV inhibition % (conc μM) | HBV inh $EC_{50}$ (μM) |
|---|---|---|---|
| E26 | 4-amino-3-(N-((1R,3R)-3-hydroxycyclopentyl)sulfamoyl)-N-(3,4,5-trifluorophenyl)benzamide | A (0.5) | - |
| E27 | *tert*-butyl 4-((2-amino-5-((3,4,5-trifluorophenyl)carbamoyl)phenyl) sulfonamido)piperid ine-1-carboxylate | B (0.5) | - |
| E28 | 4-amino-3-(N-(piperidin-4-yl)sulfamoyl)-N-(3,4,5-trifluorophenyl) benzamide | B (0.5) | - |
| E29 | 4-amino-3-((4-hydroxypiperidin-1-yl)sulfonyl)-N-(3,4,5-trifluorophenyl) benzamide | A (0.5) | - |
| E30 | 3-((4-hydroxypiperidm-1-yl)sulfonyl)-4-(methylamino)-N-(3,4,5-trifluorophenyl)benzamide | B (0.5) | - |
| E31 | 4-amino-3-(N-(pyridin-4-yl)sulfamoyl)-N-(3,4,5-trifluorophenyl) benzamide | B (0.5) | - |
| E33 | 4-ammo-3-(N-(oxetan-3-yl)sulfamoyl)-N-(3,4,5-trifluorophenyl) benzamide | A (0.5) | - |
| E34 | *tert*-butyl (S)-3-((2-amino-5-((3,4,5-trifluorophenyl)carbamoyl)phenyl) sulfonamido)pyrroli dine-1-carboxylate | A (0.5) | - |
| E35 | (S)-4-amino-3-(N-(pyrrolidin-3-yl)sulfamoyl)-N-(3,4,5-trifluorophenyl) benzamide | B (0.5) | - |
| E36 | 4-amino-3-methyl-5-sulfamoyl-N-(3 ,4,5-trifluorophenyl)benzamide | A (0.5) | - |
| E37 | 6-amino-5-sulfamoyl-N-(3,4 ,5-trifluorophenyl)nicotinamide | B (0.5) | - |
| E38 | 4-amino-3-(N-(3-(hydroxymethyl)oxetan-3-yl)sulfamoyl)-N-(3,4,5-trifluorophenyl)benzamide | A (0.5) | - |
| E39 | 4-amino-3-(N-((1-hydroxycyclohexyl)methyl)sulfamoyl)-N-(3,4,5-trifluorophenyl)benzamide | A (0.5) | - |
| E40 | 4-amino-N-(4-fluoro-3-methylphenyl)-2-methyl-5-sulfamoylbenzamide | A | |
| E41 | 4-amino-5-(N-((1 R,4R)-4-hydroxycyclohexyl)sulfamoyl)-2-methyl-N-(3,4,5-trifluorophenyl)benzamide | A (0.5) | - |
| E42 | 4-ammo-3-(N-(1-(pyridm-2-yl)ethyl)sulfamoyl)-N-(3,4,5-trifluorophenyl)benzamide | B (0.5) | - |
| E43 | trans-4-amino-N-(3-chloro-4-fluorophenyl)-3-(N-(4-hydroxycyclohexyl) sulfamoyl)benzamide | A (0.5) | - |
| E44 | 4-amino-N-(3-(difluoromethyl)-4-fluorophenyl)-3-(N-((1 R,4R)-4-hydroxycyclohexyl)sulfamoyl)benzamide | A (0.5) | - |
| E46 | trans-4-amino-N-(3-(difluoromethyl)-4-fluorophenyl)-3 -(N-(4-hydroxycyclohexyl)sulfamoyl)benzamide | A (0.5) | - |
| E47 | 4-amino-3-(N-((1S,3R)-3-hydroxycyclopentyl)sulfamoyl)-N-(3,4,5-trifluorophenyl)benzamide | A (0.5) | - |
| E48 | 4-amino-3-(N-(1,3-dihydroxypropan-2-yl)sulfamoyl)-N-(3,4,5-trifluorophenyl)benzamide | B (0.5) | - |
| E49 | 4-amino-3-(N-((1R,3S)-3-hydroxycyclopentyl)sulfamoyl)-N-(3,4,5-trifluorophenyl)benzamide | A (0.5) | - |

(continued)

| Example | Compound Name | HBV inhibition % (conc μM) | HBV inh $EC_{50}$ (μM) |
|---|---|---|---|
| E50 | 4-amino-3-((4-hydroxy-4-(hydroxymethyl)piperidin-1-yl) sulfonyl)-N-(3,4,5-trifluorophenyl)benzamide | A (0.5) | - |
| E51 | tert-butyl((1R,2S)-2-((2-amino-5-((3,4,5-trifluorophenyl)carbamoyl) phenyl)sulfonamido)cyclop entyl)carbamate | A (1) | - |
| E52 | tert-butyl ((1S,2R)-2-((2-amino-5-((3,4,5-trifluorophenyl)carbamoyl) phenyl)sulfonamido)cyclop entyl)carbamate | A (1) | - |
| E53 | 4-amino-3-((3-hydroxypyrrolidin-1-yl)sulfonyl)-N-(3,4,5-trifluorophenyl) benzamide | A (0.5) | - |
| E54 | 4-amino-N-(3-chloro-4-fluorophenyl)-3-((4-hydroxypiperidin-1-yl) sulfonyl)benzamide | A (0.5) | - |
| E55 | 4-amino-N-(3-chloro-4-fluorophenyl)-3-((3-hydroxyazetidin-1-yl) sulfonyl)benzamide | A (0.5) | - |
| E56 | 4-amino-3-(N-(2,3-dihydroxypropyl)sulfamoyl)-N-(3,4,5-trifluorophenyl)benzamide | A (0.5) | - |
| E57 | trans-4-amino-3-(N-(3-hydroxycyclopentyl)sulfamoyl)-N-(3,4,5-trifluorophenyl)benzamide | A (0.5) | - |
| E58 | trans-6-amino-5-(N-(4-hydroxycyclohexyl)sulfamoyl)-N-(3,4,5-trifluorophenyl)nicotinamide | B (0.1) | - |
| E59 | 2-amino-5-sulfamoyl-N-(3,4,5-trifluorophenyl)benzamide | B (0.5) | - |
| E60 | 4-amino-N-(3-chloro-4-fluorophenyl)-2-methyl-5-sulfamoylbenzamide | - | A |
| E61 | trans-2-amino-5-(N-(4-hydroxycyclohexyl)sulfamoyl)-N-(3,4,5-trifluorophenyl)benzamide | - | A |
| E62 | 2-amino-5-((4-hydroxypiperidin-1-yl)sulfonyl) -N-(3,4,5-trifluorophenyl) benzamide | - | A |
| E63 | (R)-4-amino-2-methyl-N-(3,4,5-trifluorophenyl)-5-(N-(1,1,1-trifluoropropan-2-yl)sulfamoyl)benzamide | A (0.1) | - |
| E64 | (S)-4-amino-2-methyl-N-(3,4,5-trifluorophenyl)-5-(N-(1,1,1-trifluoropropan-2-yl)sulfamoyl)benzamide | A (0.5) | - |
| E65 | 4-amino-N-(3-chloro-4,5-difluorophenyl)-2-methyl-5-sulfamoylbenzamide | A (0.1) | - |
| E66 | 4-amino-N-(6-chloropyridin- 3 -yl)-2-methyl-5-sulfamoylbenzamide | B (0.5) | - |
| E67 | 4-amino-N-(4-fluoro-3-(trifluoromethyl)phenyl)-2-methyl- 5-sulfamoylbenzamide | A (0.5) | - |
| E68 | 4-amino-N-(3-(difluoromethyl)-4-fluorophenyl)-2-methyl-5-sulfamoylbenzamide | A (0.1) | - |
| E69 | 4-amino-N-(3-cyano-4-fluorophenyl)-2-methyl-5-sulfamoylbenzamide | A (0.5) | - |

***In vivo properties***

Compounds of the invention were evaluated in *in vitro* and *in vivo* pharmacokinetic studies. Compound 4-amino-3-sulfamoyl-*N*-(3,4,5-trifluorophenyl)benzamide (**E3**) is stable in mouse and human hepatocytes (data not shown). When dosed *in vivo* in mice, the compound showed low *in vivo* clearance (10 mL/min/Kg).

Plasma PK parameters and plasma and liver concentrations after PO administration in mice at 100mpk in 0.5%

methocell were evaluated for compound **E3** and are summarized in Table 3, Table 4 and Table 5 below.

**Table 3. Plasma PK parameters for Compound E3**

| Parameter | Route | Original Dose | Cmax | Tmax | AUC(0-24h) | AUC Extrap |
|---|---|---|---|---|---|---|
| *Units* | | *mg/kg* | *μM* | *Hours* | *μM*Hours* | *μM*Hours* |
| Subject 01 | PO | 100 | 13.0 | 2.00 | 160 | 161.7 |
| Subject 02 | PO | 100 | 17.2 | 1.00 | 171 | 172.2 |
| Subject 03 | PO | 100 | 17.0 | 2.00 | 186 | 186.4 |
| *Mean* | | | 16 | 1.7 | 172 | 173 |

**Table 4. Plasma concentrations (uM) of compound E3 after PO administration at 100mpk in 0.5% methocell**

| *Subject* | *Hours* | | | | | | | |
|---|---|---|---|---|---|---|---|---|
| | *0.25* | *0.5* | *1* | *2* | *4* | *6* | *8* | *24* |
| **01** | 6.15 | 7.77 | 10.2 | 13.0 | 10.9 | 11.9 | 9.01 | 0.268 |
| **02** | 5.05 | 7.23 | 17.2 | 10.7 | 11.2 | 10.5 | 10.4 | 0.184 |
| **03** | 3.95 | 10.6 | 9.61 | 17.0 | 10.6 | 9.06 | 12.1 | 0.0502 |
| Mean | 5 | 9 | 12 | 14 | 11 | 10 | 10 | 0.2 |

**Table 5. Liver concentrations (uM) of compound E3 after PO administration at 100mpk in 0.5% methocell**

| Subject | *Hours* | |
|---|---|---|
| | *8* | *24* |
| 01 | 125.87 | 3.0342 |
| 02 | 119.96 | 1.8500 |
| 03 | 142.60 | 0.62717 |
| Mean | 129 | 2 |

As reported in the Tables 4 and 5, **E3** liver levels (8h) after PO administration are 13 fold higher than plasma. Data represent the ratio between the liver and plasma concentration at the same time point (8h).

The high liver-to-plasma concentration of **E3** is an important factor to be considered given that the liver is the principal tissue affected by hepatitis B disease. HBV inhibitors with hepatoselective distribution profiles represent an important strategy in developing safe drug candidates (Tu M. et al., Current Topics in Medicinal Chemistry, 2013, 13, 857-866).

## Claims

**1.** A compound of general formula (I):

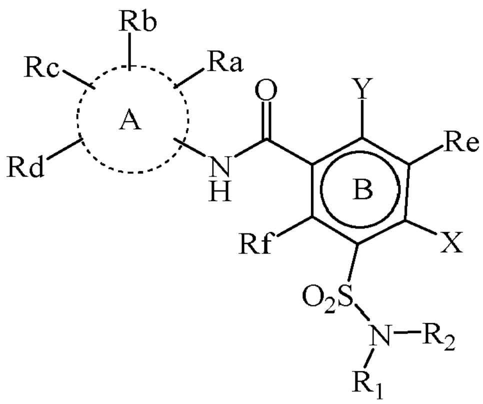

(I)

wherein:

A is a 6-membered aromatic or heteroaromatic ring;
B is a 6-membered aryl optionally containing one or more N atoms;
X is H or $NR_3R_4$;
Y is selected from the group consisting of hydrogen, halogen, $C_{1-6}$alkyl, $NH_2$, NH($C_{1-6}$alkyl), $N(CH_3)_2$, $NHC(O)CH_3$, OH, saturated or partially unsaturated $C_{3-7}$cycloalkyl, 5- or 6-membered heteroaryl and CN or is absent;
with the proviso that, when X is H, Y is selected form the group consisting of $NH_2$, NH($C_{1-6}$alkyl), $N(CH_3)_2$, $NHC(O)CH_3$;
$R_1$ and $R_2$ are each independently selected from H, linear or branched $C_{1-6}$alkyl, saturated or partially unsaturated $C_{3-7}$cycloalkyl, $C_{3-7}$heterocycloalkyl and heteroaryl, each of said linear or branched $C_{1-6}$alkyl, saturated or partially unsaturated $C_{3-7}$cycloalkyl, $C_{3-7}$heterocycloalkyl or heteroaryl group being optionally substituted with one or more substituents selected from OH, halogen, $NH_2$, NH(C=O)O$C_{1-6}$alkyl, NH($C_{1-6}$alkyl), $C_{1-6}$alkyl, $C_{3-7}$cycloalkyl, $C_{3-7}$heterocycloalkyl, $C_{1-6}$hydroxyalkyl, 5- or 6-membered heteroaryl, C(=O)$C_{1-6}$alkyl, C(=O)O$C_{1-6}$alkyl, O$C_{1-6}$alkyl, $O(CH_2)_nC_{3-10}$cycloalkyl and $O(CH_2)_nC_{3-10}$heterocycloalkyl;
or $R_1$ and $R_2$ taken together form with the N atom to which they are attached a saturated or partially unsaturated 3-10 membered heterocyclic ring optionally containing another heteroatom selected from N, O and S, said saturated or partially unsaturated 3-10 membered heterocyclic ring being optionally substituted with one or more substituents selected from OH, halogen, $C_{1-6}$alkyl, $C_{1-6}$haloalkyl and $(CH2)_nR_5$;
each occurrence of n is independently 0, 1, 2, 3 or 4;
$R_3$ and $R_4$ are each independently H, or linear or branched $C_{1-3}$alkyl optionally substituted with one or more groups selected from halogen, $NH_2$, NH$C_{1-6}$alkyl, N($C_{1-6}$alkyl)$_2$, NH(C=O)$C_{1-6}$alkyl, NH(C=O)O$C_{1-6}$alkyl, O$C_{1-6}$alkyl, $O(CH_2)_nC_{3-10}$cycloalkyl and $O(CH_2)_nC_{3-10}$heterocycloalkyl, with the proviso that $NR_3R_4$ does not form a saturated, partially saturated or unsaturated heterocyclic ring;
$R_5$ is selected from the group consisting of OH, $NH_2$, $NH(CH_3)$, $N(CH_3)_2$, $NHC(O)CH_3$, CN, halo$C_{1-3}$alkyl, $C_{1-3}$alkoxy, halo$C_{1-3}$alkoxy, heterocyclic ring, aryl and heteroaryl;
Ra is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rb is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rc is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rd is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Re is selected from the group consisting of hydrogen, halogen, $C_{1-3}$alkyl; or is absent;
Rf is hydrogen, halogen, $C_{1-3}$alkyl; or is absent;
provided that the compound is not 2-amino-N-(4-chloro-2-methylphenyl)-5-sulfamoylbenzamide or N-(2-methoxyphenyl)-2-(methylamino)-5-(piperidin-1-ylsulfonyl)benzamide;
and pharmaceutically acceptable salts, tautomers, isomers, stereoisomers thereof.

**2.** The compound according to claim 1 having formula (Ia):

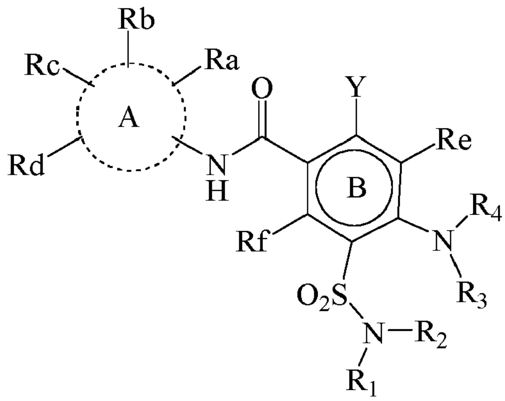

(Ia)

wherein:

A is a 6-membered aromatic or heteroaromatic ring;
B is a 6-membered aryl optionally containing one or more N atoms;
Y is selected from the group consisting of hydrogen, halogen, $C_{1-3}$alkyl, $NH_2$, NH($C_{1-6}$alkyl), $N(CH_3)_2$, $NHC(O)CH_3$, OH, saturated or partially unsaturated $C_{3-7}$cycloalkyl, 5- or 6-membered heteroaryl and CN or is absent;
$R_1$ and $R_2$ are each independently selected from H, linear or branched $C_{1-6}$alkyl, saturated or partially unsaturated $C_{3-7}$cycloalkyl, $C_{3-7}$heterocycloalkyl and heteroaryl, each of said linear or branched $C_{1-6}$alkyl, saturated or partially unsaturated $C_{3-7}$cycloalkyl, $C_{3-7}$heterocycloalkyl or heteroaryl group being optionally substituted with one or more substituents selected from OH, halogen, $NH_2$, NH(C=O)O$C_{1-6}$alkyl, NH($C_{1-6}$alkyl), $C_{1-6}$alkyl, $C_{3-7}$cycloalkyl, $C_{3-7}$heterocycloalkyl, $C_{1-6}$hydroxyalkyl, 5- or 6-membered heteroaryl, C(=O)$C_{1-6}$alkyl, C(=O)O$C_{1-6}$alkyl, O$C_{1-6}$alkyl, $O(CH_2)_nC_{3-10}$cycloalkyl and $O(CH_2)_nC_{3-10}$heterocycloalkyl;
or $R_1$ and $R_2$ taken together form with the N atom to which they are attached a saturated or partially unsaturated 3-10 membered heterocyclic ring optionally containing another heteroatom selected from N, O and S, said saturated or partially unsaturated 3-10 membered heterocyclic ring being optionally substituted with one or more substituents selected from OH, halogen, $C_{1-6}$alkyl, $C_{1-6}$haloalkyl, $(CH_2)_nR_5$;
each occurrence of n is independently 0, 1, 2, 3 or 4;
$R_3$ and $R_4$ are each independently H or linear or branched $C_{1-3}$alkyl optionally substituted with one or more groups selected from halogen, $NH_2$, NH$C_{1-6}$alkyl, N($C_{1-6}$alkyl)$_2$, NH(C=O)$C_{1-6}$alkyl, NH(C=O)O$C_{1-6}$alkyl, O$C_{1-6}$alkyl, $O(CH_2)_nC_{3-10}$cycloalkyl and $O(CH_2)_nC_{3-10}$heterocycloalkyl, with the proviso that $NR_3R_4$does not form a saturated, partially saturated or unsaturated heterocyclic ring;
$R_5$ is selected from the group consisting of OH, $NH_2$, $NH(CH_3)$, $N(CH_3)_2$, $NHC(O)CH_3$, CN, halo$C_{1-3}$alkyl, $C_{1-3}$alkoxy, halo$C_{1-3}$alkoxy, heterocyclic ring, aryl and heteroaryl;
Ra is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rb is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rc is selected from the group consisting of hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Rd is selected from the group consisting hydrogen, halogen, $C_{1-3}$alkyl, halo$C_{1-3}$alkyl, $C_{1-3}$alkoxy, halo$C_{1-3}$alkoxy, $NH_2$, $NH(CH_3)$, $N(CH_3)_2$, $NHC(O)CH_3$, OH and CN; or is absent;
Re is selected from the group consisting of hydrogen, halogen and $C_{1-3}$alkyl; or is absent;
Rf is hydrogen, halogen and $C_{1-3}$alkyl; or is absent;
and pharmaceutically acceptable salts, tautomers, isomers, stereoisomers thereof.

**3.** The compound according to claim 2, wherein:

A is a 6-membered aromatic or heteroaromatic ring;
B is a 6-membered aryl optionally containing one or more N atoms;
Y is selected from the group consisting of hydrogen, halogen, $C_{1-3}$alkyl, $NH_2$, NH($C_{1-6}$alkyl), $N(CH_3)_2$, $NHC(O)CH_3$, OH and CN or is absent;
$R_1$ is H, linear or branched $C_{1-6}$alkyl, cyclopropyl, cyclobutyl, cyclopentyl, cyclohexyl, piperidinyl, pirrolidinyl, oxetanyl, tetrahydrofuranyl, pyridinyl, said $C_{1-6}$alkyl, cyclopropyl, cyclobutyl, cyclopentyl, cyclohexyl, piperidinyl, pirrolidinyl, oxetanyl, tetrahydrofuranyl or
pyridinyl being optionally substituted with one or more substituents selected from OH, halogen, $NH_2$, NH(C=O)O$C_{1-6}$alkyl, NH($C_{1-6}$alkyl), $C_{1-6}$hydroxyalkyl, 5- or 6-membered heteroaryl, C(=O)$C_{1-6}$alkyl, C(=O)O$C_{1-6}$alkyl, O$C_{1-6}$alkyl;
$R_2$ is H or methyl;
or $R_1$ and $R_2$ taken together form with the N atom to which they are attached a heterocyclic ring selected from piperidine, pirrolidine, morpholine, thiomorpholine and piperazine, said ring being optionally substituted with one or more substituents selected from halogen, $C_{1-3}$alkyl, OH and $CH_2R_5$;
$R_3$ and $R_4$ are each independently H or $C_{1-3}$alkyl; in particular hydrogen or methyl;
$R_5$ is selected from the group consisting of OH, $NH_2$, $NH(CH_3)$, $N(CH_3)_2$, $NHC(O)CH_3$, CN, halo$C_{1-3}$alkyl, $C_{1-3}$alkoxy, halo$C_{1-3}$alkoxy, heterocyclic ring, aryl and heteroaryl;
Ra is selected from the group consisting of hydrogen, halogen, $C_{1-3}$alkyl, halo$C_{1-3}$alkyl and CN; or is absent;
Rb is selected from the group consisting of hydrogen, halogen, $C_{1-3}$alkyl, halo$C_{1-3}$alkyl and CN; or is absent;
Rc is selected from the group consisting of hydrogen, halogen, $C_{1-3}$alkyl, halo$C_{1-3}$alkyl and CN; or is absent;
Rd is selected from the group consisting of hydrogen, halogen, $C_{1-3}$alkyl, halo$C_{1-3}$alkyl and CN; or is absent;
Re is selected from the group consisting of hydrogen, halogen, $C_{1-3}$alkyl, or is absent;

Rf is hydrogen or is absent;
and pharmaceutically acceptable salts, tautomers, isomers, stereoisomers thereof.

**4.** The compound according to anyone of claims 2 or 3, wherein:

A is a 6-membered aromatic or heteroaromatic ring;
B is a 6-membered aryl optionally containing one or more N atoms;
Y is selected from the group consisting of hydrogen, halogen, $C_{1-3}$alkyl, or is absent;
$R_1$ is hydrogen, methyl, or is selected from the group consisting of:

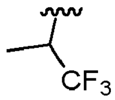, 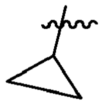, 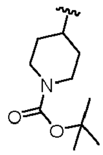,

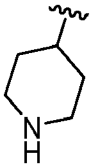, 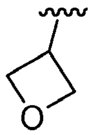, 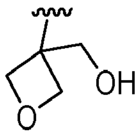, 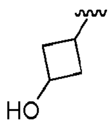, 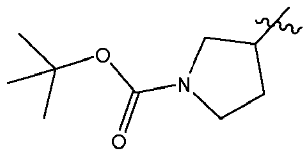, 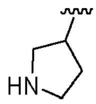, 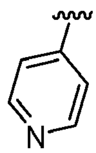, 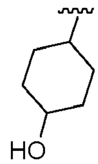,

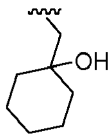, 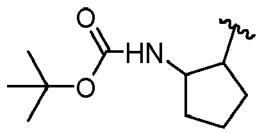, 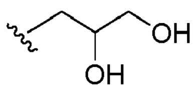, 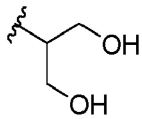, 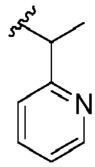

and

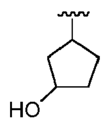;

$R_2$ is H or methyl;
or $R_1$ and $R_2$ taken together form with the N atom to which they are attached a heterocyclic ring selected from the group consisting of:

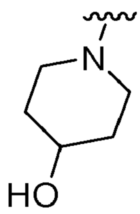, 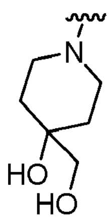, 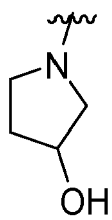

and

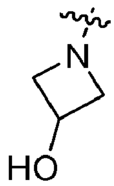
;

$R_3$ and $R_4$ are each independently H or $C_{1-3}$alkyl; in particular hydrogen or methyl;
Ra is selected from the group consisting of hydrogen, halogen, $C_{1-3}$alkyl, halo$C_{1-3}$alkyl and CN; or is absent;
Rb is selected from the group consisting of hydrogen, halogen, $C_{1-3}$alkyl, halo$C_{1-3}$alkyl and CN; or is absent;
Rc is selected from the group consisting of hydrogen, halogen, $C_{1-3}$alkyl, halo$C_{1-3}$alkyl and CN; or is absent;
Rd is selected from the group consisting of hydrogen, halogen, $C_{1-3}$alkyl, halo$C_{1-3}$alkyl and CN; or is absent;
Re is selected from the group consisting of hydrogen, halogen, $C_{1-3}$alkyl, or is absent;
Rf is hydrogen; or is absent;
and pharmaceutically acceptable salts, tautomers, isomers, stereoisomers thereof.

**5.** The compound of anyone of previous claims, wherein:

A is phenyl or pyridyl;
B is phenyl or pyridyl;
wherein preferably A is phenyl and B is phenyl
and pharmaceutically acceptable salts, tautomers, isomers, stereoisomers thereof.

**6.** The compound according to anyone of previous claims wherein at least one of Ra, Rb, Rc and Rd is F and the other(s) is/are hydrogen and pharmaceutically acceptable salts, tautomers, isomers, stereoisomers thereof.

**7.** The compound according to claim 1 being selected from the following list:

- 4-amino-3-(N-methylsulfamoyl)-N-(3,4,5-trifluorophenyl)benzamide;
- 4-amino-3-sulfamoyl-*N*-(3,4,5-trifluorophenyl)benzamide;
- 4-amino-N-(3,4-difluorophenyl)-3-sulfamoylbenzamide;
- 4-amino-2-chloro-5-sulfamoyl-N-(3,4,5-trifluorophenyl)benzamide;
- 4-amino-2-bromo-5-sulfamoyl-N-(3,4,5-trifluorophenyl)benzamide;
- 4-amino-N-(4-fluoro-3-(trifluoromethyl)phenyl)-3-sulfamoylbenzamide;
- 4-amino-N-(3-cyano-4-fluorophenyl)-3-sulfamoylbenzamide;
- 4-amino-N-(3-(difluoromethyl)-4-fluorophenyl)-3-sulfamoylbenzamide;
- 4-amino-N-(3-chloro-4-fluorophenyl)-3-sulfamoylbenzamide;
- 4-amino-N-(4-fluoro-3-methylphenyl)-3-sulfamoylbenzamide;
- 4-amino-2-methyl-5-sulfamoyl-N-(3,4,5-trifluorophenyl)benzamide;
- (R)-4-amino-N-(3,4,5-trifluorophenyl)-3-(N-(1,1,1-trifluoropropan-2-yl)sulfamoyl)benzamide;
- (S)-4-amino-N-(3,4,5-trifluorophenyl)-3-(N-(1,1,1-trifluoropropan-2-yl)sulfamoyl)benzamide;
- 4-amino-3-(N-cyclopropylsulfamoyl)-N-(3,4,5-trifluorophenyl)benzamide;
- *trans*-4-amino-3-(N-(4-hydroxycyclohexyl)sulfamoyl)-N-(3,4,5-trifluorophenyl)benzamide;
- *cis*-4-amino-3-(N-(4-hydroxycyclohexyl)sulfamoyl)-N-(3,4,5-trifluorophenyl)benzamide;
- *trans*-4-amino-5-(N-(4-hydroxycyclohexyl)sulfamoyl)-2-methyl-N-(3,4,5-trifluorophenyl) benzamide;
- *cis*-4-amino-3-(N-3-hydroxycyclobutyl)sulfamoyl)-N-(3,4,5-trifluorophenyl)benzamide;
- *trans*-4-amino-3-(N-3-hydroxycyclobutyl)sulfamoyl)-N-(3,4,5-trifluorophenyl)benzamide;
- 4-amino-3-(N-((1R,3R)-3-hydroxycyclopentyl)sulfamoyl)-N-(3,4,5-trifluorophenyl)benzamide;
- 4-amino-3-((4-hydroxypiperidin-1-yl)sulfonyl)-N-(3,4,5-trifluorophenyl)benzamide;
- 4-amino-3-(N-(oxetan-3-yl)sulfamoyl)-N-(3,4,5-trifluorophenyl)benzamide;
- *tert*-butyl(S)-3-((2-amino-5-((3,4,5-trifluorophenyl)carbamoyl)phenyl)sulfonamido) pyrrolidine-1-carboxylate;
- 4-amino-3-methyl-5-sulfamoyl-*N*-(3,4,5-trifluorophenyl)benzamide;
- 4-amino-3-(N-(3-(hydroxymethyl)oxetan-3-yl)sulfamoyl)-N-(3,4,5-trifluorophenyl)benzamide;
- 4-amino-3-(N-((1-hydroxycyclohexyl)methyl)sulfamoyl)-N-(3,4,5-trifluorophenyl)benzamide;
- 4-amino-N-(4-fluoro-3-methylphenyl)-2-methyl-5-sulfamoylbenzamide;
- 4-amino-5-(N-((1R,4R)-4-hydroxycyclohexyl)sulfamoyl)-2-methyl-N-(3,4,5-trifluorophenyl) benzamide;
- trans-4-amino-N-(3-chloro-4-fluorophenyl)-3-(N-(4-hydroxycyclohexyl)sulfamoyl)benzamide;

- 4-amino-N-(3-(difluoromethyl)-4-fluorophenyl)-3-(N-((1r1R,4r4R)-4-hydroxycyclohexyl) sulfamoyl)benzamide;
- trans-4-amino-N-(3-(difluoromethyl)-4-fluorophenyl)-3-(N-(4-hydroxycyclohexyl)sulfamoyl) benzamide;
- 4-amino-3-(N-((1S,3R)-3-hydroxycyclopentyl)sulfamoyl)-N-(3,4,5-trifluorophenyl)benzamide;
- 4-amino-3-(N-((1R,3S)-3-hydroxycyclopentyl)sulfamoyl)-N-(3,4,5-trifluorophenyl)benzamide;
- 4-amino-3-((4-hydroxy-4-(hydroxymethyl)piperidin-1-yl)sulfonyl)-N-(3,4,5-trifluorophenyl)benzamide;
- tert-butyl((1R,2S)-2-((2-amino-5-((3,4,5-trifluorophenyl)carbamoyl)phenyl)sulfonamido) cyclopentyl)carbamate;
- tert-butyl((1S,2R)-2-((2-amino-5-((3,4,5-trifluorophenyl)carbamoyl)phenyl)sulfonamido) cyclopentyl)carbamate;
- 4-amino-3-((3-hydroxypyrrolidin-1-yl)sulfonyl)-N-(3,4,5-trifluorophenyl)benzamide;
- 4-amino-N-(3-chloro-4-fluorophenyl)-3-((4-hydroxypiperidin-1-yl)sulfonyl)benzamide;
- 4-amino-N-(3-chloro-4-fluorophenyl)-3-((3-hydroxyazetidin-1-yl)sulfonyl)benzamide;
- 4-amino-3-(N-(2,3-dihydroxypropyl)sulfamoyl)-N-(3,4,5-trifluorophenyl)benzamide;
- trans-4-amino-3-(N-(3-hydroxycyclopentyl)sulfamoyl)-N-(3,4,5-trifluorophenyl)benzamide;
- trans-2-amino-5-(N-(4-hydroxycyclohexyl)sulfamoyl)-N-(3,4,5-trifluorophenyl)benzamide;
- 2-amino-5-((4-hydroxypiperidin-1-yl)sulfonyl)-N-(3,4,5-trifluorophenyl)benzamide;
- (R)-4-amino-2-methyl-N-(3,4,5-trifluorophenyl)-5-(N-(1,1,1-trifluoropropan-2-yl)sulfamoyl) benzamide;
- (S)-4-amino-2-methyl-N-(3,4,5-trifluorophenyl)-5-(N-(1,1,1-trifluoropropan-2-yl)sulfamoyl) benzamide;
- 4-amino-N-(3-chloro-4,5-difluorophenyl)-2-methyl-5-sulfamoylbenzamide;
- 4-amino-N-(4-fluoro-3-(trifluoromethyl)phenyl)-2-methyl-5-sulfamoylbenzamide;
- 4-amino-N-(3-(difluoromethyl)-4-fluorophenyl)-2-methyl-5-sulfamoylbenzamide;
- 4-amino-N-(3-cyano-4-fluorophenyl)-2-methyl-5-sulfamoylbenzamide;
- 4-amino-N-(3-chloro-4-fluorophenyl)-2-methyl-5-sulfamoylbenzamide; and pharmaceutically acceptable salts, tautomers, isomers, stereoisomers thereof.

**8.** A compound as defined in any one of previous claims for medical use.

**9.** A compound as defined in claim 8 for use in the treatment and/or prevention of a HBV infection.

**10.** A compound of general formula (I):

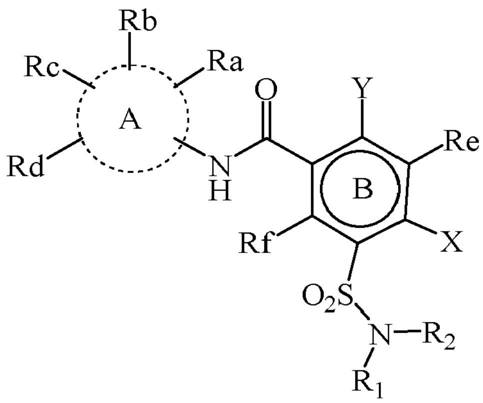

(I)

wherein:

A is a 6-membered aromatic or heteroaromatic ring;
B is a 6-membered aryl optionally containing one or more N atoms;
X is H or $NR_3R_4$;
Y is selected from the group consisting of hydrogen, halogen, $C_{1-6}$alkyl, $NH_2$, NH($C_{1-6}$alkyl), $N(CH_3)_2$, $NHC(O)CH_3$, OH, saturated or partially unsaturated $C_{3-7}$cycloalkyl, 5- or 6-membered heteroaryl and CN or is absent;
with the proviso that, when X is H, Y is selected form the group consisting of $NH_2$, NH($C_{1-6}$alkyl), $N(CH_3)_2$, $NHC(O)CH_3$;
$R_1$ and $R_2$ are each independently selected from H, linear or branched $C_{1-6}$alkyl, saturated or partially unsaturated

$C_{3-7}$cycloalkyl, $C_{3-7}$heterocycloalkyl and heteroaryl, each of said linear or branched $C_{1-6}$alkyl, saturated or partially unsaturated $C_{3-7}$cycloalkyl, $C_{3-7}$heterocycloalkyl or heteroaryl group being optionally substituted with one or more substituents selected from OH, halogen, $NH_2$, NH(C=O)O$C_{1-6}$alkyl, NH($C_{1-6}$alkyl), $C_{1-6}$alkyl, $C_{3-7}$cycloalkyl, $C_{3-7}$heterocycloalkyl, $C_{1-6}$hydroxyalkyl, 5- or 6-membered heteroaryl, C(=O)$C_{1-6}$alkyl, C(=O)O$C_{1-6}$alkyl, O$C_{1-6}$alkyl, O$(CH_2)_n$$C_{3-10}$cycloalkyl and O$(CH_2)_n$$C_{3-10}$heterocycloalkyl;

or $R_1$ and $R_2$ taken together form with the N atom to which they are attached a saturated or partially unsaturated 3-10 membered heterocyclic ring optionally containing another heteroatom selected from N, O and S, said saturated or partially unsaturated 3-10 membered heterocyclic ring being optionally substituted with one or more substituents selected from OH, halogen, $C_{1-6}$alkyl, $C_{1-6}$haloalkyl and $(CH_2)_nR_5$;

each occurrence of n is independently 0, 1, 2, 3 or 4;

$R_3$ and $R_4$ are each independently H, or linear or branched $C_{1-3}$alkyl optionally substituted with one or more groups selected from halogen, $NH_2$, NH$C_{1-6}$alkyl, N($C_{1-6}$alkyl$)_2$, NH(C=O)$C_{1-6}$alkyl, NH(C=O)O$C_{1-6}$alkyl, O$C_{1-6}$alkyl, O$(CH_2)_n$$C_{3-10}$cycloalkyl and O$(CH_2)_n$$C_{3-10}$heterocycloalkyl, with the proviso that $NR_3R_4$ does not form a saturated, partially saturated or unsaturated heterocyclic ring;

$R_5$ is selected from the group consisting of OH, $NH_2$, NH($CH_3$), N$(CH_3)_2$, NHC(O)$CH_3$, CN, halo$C_{1-3}$alkyl, $C_{1-3}$alkoxy, halo$C_{1-3}$alkoxy, heterocyclic ring, aryl and heteroaryl;

Ra is selected from the group consisting of hydrogen, halogen, $C_{1-3}$alkyl, halo$C_{1-3}$alkyl, $C_{1-3}$alkoxy, halo$C_{1-3}$alkoxy, $NH_2$, NH($CH_3$), N$(CH_3)_2$, NHC(O)$CH_3$, OH and CN; or is absent;

Rb is selected from the group consisting of hydrogen, halogen, $C_{1-3}$alkyl, halo$C_{1-3}$alkyl, $C_{1-3}$alkoxy, halo$C_{1-3}$alkoxy, $NH_2$, NH($CH_3$), N$(CH_3)_2$, NHC(O)$CH_3$, OH and CN; or is absent;

Rc is selected from the group consisting of hydrogen, halogen, $C_{1-3}$alkyl, halo$C_{1-3}$alkyl, $C_{1-3}$alkoxy, halo$C_{1-3}$alkoxy, $NH_2$, NH($CH_3$), N$(CH_3)_2$, NHC(O)$CH_3$, OH and CN; or is absent;

Rd is selected from the group consisting of hydrogen, halogen, $C_{1-3}$alkyl, halo$C_{1-3}$alkyl, $C_{1-3}$alkoxy, halo$C_{1-3}$alkoxy, $NH_2$, NH($CH_3$), N$(CH_3)_2$, NHC(O)$CH_3$, OH and CN; or is absent;

Re is selected from the group consisting of hydrogen, halogen, $C_{1-3}$alkyl; or is absent;

Rf is hydrogen, halogen, $C_{1-3}$alkyl; or is absent;

and pharmaceutically acceptable salts, tautomers, isomers, stereoisomers thereof, for use in the treatment and/or prevention of a HBV infection.

**11.** The compound according to anyone of claim 9 or 10 for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

**12.** The compound according to any one of claims 8-11, for use in combination with at least one further therapeutic agent.

**13.** The compound for use according to claim 12, wherein the at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof; wherein said therapeutic vaccine is preferably selected from: HBsAG-HBIG, HB-Vac, ABX203, NASVAC, GS-4774, GX-110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell; wherein said RNA interference therapeutic is preferably selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404; wherein said immunomodulator is preferably a TLR agonist, preferably a TLR7, TLR8 or TLR9 agonist, preferably being selected from: RG7795 (RO-6864018), GS-9620, SM360320 (9-benzyl-8-hydroxy-2-(2-methoxyethoxy)adenine), AZD 8848 (methyl[3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598; wherein said RIG-I modulator is preferably SB-9200; wherein said IL agonist or other immune acting protein is preferably INO-9112 or recombinant IL12; wherein said immune checkpoint modulator/inhibitor is preferably BMS-936558 (Opdivo (nivolumab)) or pembrolizumab; wherein said HBV entry inhibitor is preferably Myrcludex B, IVIG-Tonrol or GC-1102; wherein said cccDNA modulator is

preferably selected from: a direct cccDNA inhibitor, an inhibitor of cccDNA formation or maintenance, a cccDNA epigenetic modifier and an inhibitor of cccDNA transcription; wherein said capsid assembly inhibitor/modulator, core or X protein targeting agent, direct cccDNA inhibitor, inhibitor of cccDNA formation or maintenance, or cccDNA epigenetic modifier is preferably selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389; wherein said interferon or modified interferon is preferably selected from: interferon alpha (IFN-$\alpha$), pegylated interferon alpha (PEG-IFN-$\alpha$), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-$\lambda$), peginterferon lambda-1, interferon omega, interferon tau, interferon gamma (IFN-$\gamma$), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter; wherein said HBV antiviral of distinct or unknown mechanism is selected from: AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259; wherein said cyclophilin inhibitor is preferably selected from: OCB-030 (NVP-018), SCY-635, SCY-575 and CPI-431-32; wherein said HBV polymerase inhibitor is preferably selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir, preferably said tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157; wherein said dinucleotide is preferably SB9200; wherein said SMAC inhibitor is preferably Birinapant; wherein said HDV targeting agent is preferably Lonafamib; wherein said HBV RNA destabilizer or other small-molecule inhibitor of HBV protein expression is preferably RG7834 or AB-452.

**14.** A pharmaceutical composition comprising the compound as defined in anyone of claims 1-7 or 10, alone or in combination with at least one further therapeutic agent, and at least one pharmaceutically acceptable excipient, wherein the at least one further therapeutic agent is preferably selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof; wherein said therapeutic vaccine is preferably selected from: HBsAG-HBIG, HB-Vac, ABX203, NASVAC, GS-4774, GX-110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell; wherein said RNA interference therapeutic is preferably selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404; wherein said immunomodulator is preferably a TLR agonist, preferably a TLR7, TLR8 or TLR9 agonist, preferably being selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598; wherein said RIG-I modulator is preferably SB-9200; wherein said IL agonist or other immune acting protein is preferably INO-9112 or recombinant IL12; wherein said immune checkpoint modulator/inhibitor is preferably BMS-936558 (Opdivo (nivolumab)) or pembrolizumab; wherein said HBV entry inhibitor is preferably Myrcludex B, IVIG-Tonrol or GC-1102; wherein said cccDNA modulator is preferably selected from: a direct cccDNA inhibitor, an inhibitor of cccDNA formation or maintenance, a cccDNA epigenetic modifier and an inhibitor of cccDNA transcription; wherein said capsid assembly inhibitor/modulator, core or X protein targeting agent, direct cccDNA inhibitor, inhibitor of cccDNA formation or maintenance, or cccDNA epigenetic modifier is preferably selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389; wherein said interferon or modified interferon is preferably selected from: interferon alpha (IFN-$\alpha$), pegylated interferon alpha (PEG-IFN-$\alpha$), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter; wherein said HBV antiviral of distinct or unknown mechanism is selected from: AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259; wherein said cyclophilin inhibitor is preferably selected from: OCB-030 (NVP-018), SCY-635, SCY-575 and CPI-431-32; wherein said HBV polymerase inhibitor is preferably selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir, preferably said tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157; wherein said dinucleotide is preferably SB9200; wherein said SMAC inhibitor is preferably Birinapant; wherein said HDV targeting agent is preferably Lonafamib; wherein said HBV RNA destabilizer or other small-molecule inhibitor of HBV protein expression is preferably RG7834 or AB-452.

**15.** The pharmaceutical composition according to claim 14 for use in the treatment and/or prevention of HBV infections, preferably for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

Europäisches Patentamt
European Patent Office
Office européen des brevets

# EUROPEAN SEARCH REPORT

Application Number
EP 18 18 4456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JACKMAN G B ET AL: "Studies inthe field of diuretic agents. VIII. Some miscellaneous derivatives", JOURNAL OF PHARMACY AND PHARMACO, JOHN WILEY & SONS LTD, LONDON; GB, vol. 15, 1 March 1963 (1963-03-01), pages 202-211, XP008088353, ISSN: 0022-3573 * page 202; compound III * ----- | 1-3,5 | INV. C07D213/74 A61K31/337 A61K31/4025 A61K31/44 C07D213/76 C07C311/44 C07D207/14 C07D211/58 C07D211/96 C07D305/08 A61P31/20 |
| X | DATABASE REGISTRY [Online] CHEMICAL ABSTRACTS SERVICE, COLUMBUS, OHIO, US; 10 April 2005 (2005-04-10), XP002784472, Database accession no. 84818-66-5 * the whole document * ----- | 1-3,5 | |
| X | DATABASE REGISTRY [Online] CHEMICAL ABSTRACTS SERVICE, COLUMBUS, OHIO, US; 17 July 2006 (2006-07-17), XP002784473, Database accession no. 893765-13-4 * the whole document * ----- | 1-3,5 | TECHNICAL FIELDS SEARCHED (IPC) C07D A61K C07C |
| A,D | WO 2013/006394 A1 (INST HEPATITIS & VIRUS RES [US]; GUO JU-TAO [US]; XU XIAODONG [US]; BL) 10 January 2013 (2013-01-10) * abstract * * claim 1 * ----- | 1-15 | |
| A,D | WO 2014/165128 A2 (NOVIRA THERAPEUTICS INC [US]) 9 October 2014 (2014-10-09) * abstract * * claim 1 * ----- | 1-15 | |
| | -/-- | | |

The present search report has been drawn up for all claims

1

| Place of search | Date of completion of the search | Examiner |
|---|---|---|
| Munich | 6 September 2018 | Bissmire, Stewart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 18 4456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2015/120178 A1 (NOVIRA THERAPEUTICS INC [US]) 13 August 2015 (2015-08-13)<br>* abstract *<br>* claim 1 *<br>----- | 1-15 | |
| A,D | US 2016/185748 A1 (CLARK RYAN C [US] ET AL) 30 June 2016 (2016-06-30)<br>* abstract *<br>* claim 1 *<br>----- | 1-15 | |

TECHNICAL FIELDS SEARCHED (IPC)

The present search report has been drawn up for all claims

1

| Place of search | Date of completion of the search | Examiner |
|---|---|---|
| Munich | 6 September 2018 | Bissmire, Stewart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 18 18 4456

This annex lists the patent family members relating to the patent documents cited in the above-mentioned European search report. The members are as contained in the European Patent Office EDP file on
The European Patent Office is in no way liable for these particulars which are merely given for the purpose of information.

06-09-2018

| Patent document cited in search report | Publication date | Patent family member(s) | Publication date |
|---|---|---|---|
| WO 2013006394 A1 | 10-01-2013 | CN 103889953 A | 25-06-2014 |
| | | CN 106166157 A | 30-11-2016 |
| | | EP 2726459 A1 | 07-05-2014 |
| | | EP 3085368 A1 | 26-10-2016 |
| | | JP 5977347 B2 | 24-08-2016 |
| | | JP 2014523901 A | 18-09-2014 |
| | | JP 2017014225 A | 19-01-2017 |
| | | US 2014206666 A1 | 24-07-2014 |
| | | US 2016101074 A1 | 14-04-2016 |
| | | US 2017320818 A1 | 09-11-2017 |
| | | WO 2013006394 A1 | 10-01-2013 |
| WO 2014165128 A2 | 09-10-2014 | US 2014275167 A1 | 18-09-2014 |
| | | US 2015174115 A1 | 25-06-2015 |
| | | US 2016158214 A1 | 09-06-2016 |
| | | WO 2014165128 A2 | 09-10-2014 |
| WO 2015120178 A1 | 13-08-2015 | AU 2015214096 A1 | 01-09-2016 |
| | | CA 2936947 A1 | 13-08-2015 |
| | | CN 106232136 A | 14-12-2016 |
| | | EP 3102225 A1 | 14-12-2016 |
| | | JP 2017505314 A | 16-02-2017 |
| | | KR 20160128305 A | 07-11-2016 |
| | | US 2015216938 A1 | 06-08-2015 |
| | | US 2017182021 A1 | 29-06-2017 |
| | | WO 2015120178 A1 | 13-08-2015 |
| US 2016185748 A1 | 30-06-2016 | US 2016185748 A1 | 30-06-2016 |
| | | US 2018051000 A1 | 22-02-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013006394 A
- WO 2013096744 A
- WO 2014106019 A
- WO 2014165128 A
- WO 2015109130 A
- US 2015274652 A
- WO 2015120178 A
- WO 2016089990 A
- US 2016185748 A
- US 2016151375 A
- WO 2017001655 A1


### Non-patent literature cited in the description


- **GUIDOTTI LG ; CHISARI FV.** *Annu Rev Pathol.,* 2006, vol. 1, 23-61
- **SEEGER C.** *Mason WS.Virology.,* May 2015, vol. 479-480, 672-86
- **DURANTEL D ; ZOULIM F.** *J Hepatol.,* April 2016, vol. 64 (1), S117-S131
- **BURNS GS ; THOMPSON AJ.** *Cold Spring Harb Perspect Med.,* 30 October 2014, vol. 4 (12
- **HUGHES, S.A. et al.** *Lancet,* 2011, vol. 378, 73-85
- **ZOULIM ; DURANTEL D.** *Cold Spring Harb Perspect Med.,* 01 April 2015, vol. 5 (4
- *Gastroenterology,* 2018, vol. 154, 652-662
- **TU M. et al.** *Current Topics in Medicinal Chemistry,* 2013, vol. 13, 857-866
- Design of Prodrugs. Elsevier, 1985
- **E.L. ELIEL ; S.H. WILEN.** Stereochemistry of Carbon Compounds. John Wiley & Sons, 1994, 1119-1190
- **BERG et al.** Pharmaceutical Salts. *J. Pharm. Sci.,* 1977, vol. 66, 1-19
- **LADNER et al.** *Antimicrob Agents Chemother,* 1997, vol. 41, 1715-20